# EUROPEAN PATENT APPLICATION

(11) **EP 4 692 061 A1**
(43) Date of publication of application: **11.02.2026**
(21) Application number: 24781309.0
(22) Date of filing: 29.03.2024
(51) Int. Cl.: C07D 211/88, A61K 31/45, A61K 31/454, A61K 31/553, A61P 37/00, A61P 29/00, A61P 25/00, A61P 35/00, A61P 35/02, C07D 401/12

(54) **LOW-MOLECULAR-WEIGHT COMPOUND HAVING CEREBLON-BINDING ACTIVITY, AND USE THEREOF**

(30) Priority: 29.03.2023 KR 20230041340
(71) Applicant: Aevis Bio, Inc., Daejeon 34141 (KR); Korea Research Institute of Chemical Technology, Daejeon 34114 (KR)
(72) Inventor: KIM, Dong Seok, Daejeon 34033 (KR); HONG, Eun Ji, Seongnam-si, Gyeonggi-do 13137 (KR); KIM, Ju Wan, Yongin-si, Gyeonggi-do 17066 (KR); LIM, Yun Young, Incheon 21514 (KR); KWAK, Jae Sung, Gimcheon-si, Gyeongsangbuk-do 39660 (KR); LEE, Hyuk, Seoul 08000 (KR); SHIM, Su Yong, Daejeon 35350 (KR); PARK, A Reum, Daejeon 34114 (KR)
(74) Representative: Maiwald GmbH
(86) International application number: PCT/KR2024/004071
(87) International publication number: WO 2024/205319

(57) **Abstract**

The novel low-molecular-weight compound according to the present invention has a superior ability to bind to cereblon and thus can act as a novel immunomodulator for treating tumors or cancers.

## Description

### TECHNICAL FIELD

The present invention relates to a novel low-molecular-weight compound having cereblon-binding activity and the use thereof for treating tumors or cancers.

### BACKGROUND

The ubiquitin-proteasome pathway (UPP) is an essential pathway that regulates major regulatory proteins and degrades misfolded or abnormal proteins. The UPP plays a central role in various cellular processes, and defects or imbalances in this pathway can lead to the onset of diverse diseases. The covalent attachment of ubiquitin to specific protein substrates is achieved through the action of E3 ubiquitin ligases. These ligases comprise more than 500 different proteins and are classified into several classes according to the structural elements that define their E3 functional activity.

Cereblon (CRBN) interacts with damaged DNA binding protein 1 (DDB1) and, together with Cullin 4, forms a DDB1-CUL4A-Roc1 ubiquitin ligase complex, in which CRBN functions as a substrate receptor that recognizes specific proteins, leading to their ubiquitination and subsequent degradation by the proteasome.

Cereblon (CRBN) is known as a molecular target of immunomodulatory drugs (IMiDs) such as thalidomide, lenalidomide, and pomalidomide (Lopez-Girona et al.; Cereblon is a direct protein target for immunomodulatory and antiproliferative activities of lenalidomide and pomalidomide. Leukemia 2012; 26:2326-2335). Specifically, thalidomide, a drug approved in the late 1990s for the treatment of multiple myeloma, binds to cereblon and modulates the substrate specificity of the CRL4^{CRBN} ubiquitin ligase complex. This mechanism underlies the pleiotropic effects of thalidomide on immune cells and cancer cells. However, as adverse effects of thalidomide were reported, various studies have been conducted to develop analogs with higher efficacy and fewer side effects for use as anticancer agents. As a result, cereblon modulators such as lenalidomide, pomalidomide, CC-220, CC-122, CC-885, and TD-106 have been developed, and their clinical applicability has been demonstrated in hematologic malignancies such as multiple myeloma, myelodysplastic syndromes, lymphomas, and leukemia (Le Roy A et al.: Immunomodulatory Drugs Exert Anti-Leukemia Effects in Acute Myeloid Leukemia by Direct and Immunostimulatory Activities. Front Immunol. 2018; 9: 977).

The antitumor activity of cereblon modulators is mediated by the following mechanisms:
(1) inhibition of cancer cell proliferation and induction of apoptosis;
(2) interruption of nutritional support from the tumor stroma; and
(3) proliferation of T cells, production of cytokines, and activation of NK (natural killer) cells through stimulation of immune cells.

Accordingly, the inventors of the present invention have made efforts to develop a novel low-molecular-weight compound having substrate specificity for cereblon and capable of effectively inducing tumor cell death. As a result, the present invention has been completed by developing a novel low-molecular-weight compound that acts as a cereblon ligand useful for the treatment of tumors or cancers.

### DISCLOSURE

### TECHNICAL PROBLEM

The present invention is directed to providing novel compounds having binding activity to cereblon and uses thereof.

### TECHNICAL SOLUTION

To achieve the above object, the present invention provides a compound represented by the following Chemical Formula (I), or an optical isomer thereof, a racemic mixture thereof, a hydrate thereof, a solvate thereof, or a pharmaceutically acceptable salt thereof:

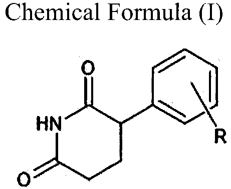

In Chemical Formula (I),
R is independently hydrogen (H), halogen (X), hydroxyl (OH), amino (NH₂), alkyl, alkylamine, aminocarbonylalkyl, aminocarbonylaryl, aminosulfonylalkyl, aminocarbonylalcohol, aminosulfonylaryl, aminosulfonylalkyl, aminoalkylaryl, aminocarbonylaminoaryl, alkylaminocarbonylalkoxy, aminosulfonylaminocarbonylaminoaryl, aminoarylalkylcarboxylalkyl, aminoarylalkylcarbonylaminoaryl, aminoarylalkylaminocarboxylalkyl, aminoarylalkylaminocarbonylaminoaryl, aminosulfonylarylaminocarbonylarylalkoxy, aminoarylalkylcarbonylaminoarylalkoxy, aminoarylalkylcarbonylaminoalkylcarboxylalkyl, aminoarylalkylcarbonylaminoalkylcarboxylic acid, aminoarylaminocarbonylalkylcarbonylaminoaryl, aminoarylaminocarbonylaryl, or aminosulfonylarylaminocarbonylaryl; and
(ii) R is a heterocycloalkyl group including a CONH moiety, wherein the heterocycloalkyl forms a bicyclic fused ring with the benzene ring, the hydrogen (H) bound to the nitrogen (N) of the CONH group is unsubstituted or substituted, and the carbon (C) of the heterocycloalkyl is unsubstituted or substituted with oxygen (O),
and any benzene ring present in Chemical Formula (I) is an unsubstituted benzene ring or a benzene ring having one or more hydrogen atoms substituted.

In the present invention, Chemical Formula (I) may be characterized by being represented by the following Chemical Formula (II):

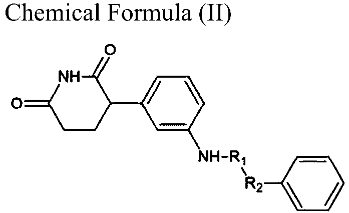

In Chemical Formula (II),
R₁ is absent, CH₂, CO, or SO₂;
R₂ is absent or C₆H₄NHCO; and
any benzene ring present in Chemical Formula (II) is an unsubstituted benzene ring or a benzene ring having one or more hydrogen atoms substituted.

In the present invention, the compound represented by Chemical Formula (II) may be characterized by being represented by any one of the Chemical Formulae selected from the group consisting of Chemical Formulae (II-1) to (II-5):

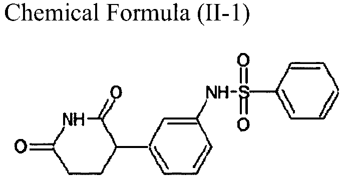

any benzene ring present in Chemical Formula (II-1) is an unsubstituted benzene ring or a benzene ring having one or more hydrogen atoms substituted; 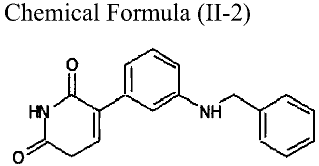
any benzene ring present in Chemical Formula (II-2) is an unsubstituted benzene ring or a benzene ring having one or more hydrogen atoms substituted; 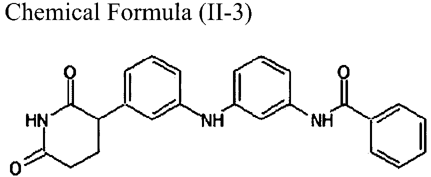
any benzene ring present in Chemical Formula (II-3) is an unsubstituted benzene ring or a benzene ring having one or more hydrogen atoms substituted; 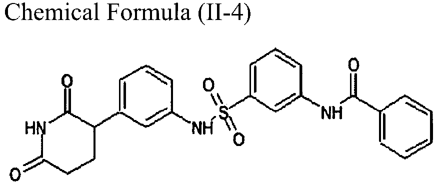
any benzene ring present in Chemical Formula (II-4) is an unsubstituted benzene ring or a benzene ring having one or more hydrogen atoms substituted; 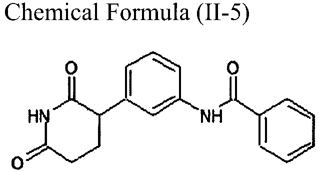
and any benzene ring present in Chemical Formula (II-5) is an unsubstituted benzene ring or a benzene ring having one or more hydrogen atoms substituted.

In the present invention, any benzene ring present in Chemical Formula (I) may be characterized by having one or more hydrogen atoms each independently substituted with alkyl, cycloalkyl, aryl, heterocyclyl, cycloalkylalkyl, aralkyl, heterocyclylalkyl, heterocycloalkylalkyl, halogen, hydroxyalkyl, alkoxy, alkoxyalkyl, amino, amido, urea, sulfonyl, hydroxy, aldehyde, carboxy, haloalkyl, alkoxy, cycloalkyloxy, aryloxy, heterocyclyloxy, heterocycloalkyloxy, cycloalkylalkyloxy, aralkyloxy, heterocyclylalkyloxy, heterocycloalkylalkyloxy; oxo (=O); amino, alkylamino, cycloalkylamino, arylamino, heterocyclylamino, heterocycloalkylamino, cycloalkylalkylamino, aralkylamino, heterocyclylalkylamino, heterocycloalkylalkylamino; imino; imido; amidino; guanidino; enamino; acylamino; sulfonylamino; urea, nitrourea; oxime; hydroxylamino; alkoxyamino; aralkoxyamino; hydrazino; hydrazido; hydrazono; azido; nitro; thio (-SH), alkylthio; =S; sulfinyl; sulfonyl; aminosulfonyl; phosphonate; phosphinyl; acyl; formyl; carboxy; ester; carbamate; amido; cyano; isocyanato; isothiocyanato; cyanato; thiocyanato; hydroxyl; alkoxy; alkoxyalkyl; amino; alkylamino; carboxy; nitro; cyano; thiol; thioether; imine; imide; amidine; guanidine; enamine; aminocarbonyl; acylamino; phosphonate; phosphine; thiocarbonyl; sulfinyl; sulfone; sulfonamide; ketone; aldehyde; ester; urea; urethane; oxime; hydroxylamine; alkoxyamine; aralkoxyamine; N-oxide; hydrazine; hydrazide; hydrazone; azide; isocyanate; isothiocyanate; cyanate; thiocyanate; B(OH)₂.

In the present invention, any benzene ring present in any one of Chemical Formulae (II-1) to (II-5) may be characterized by having one or more hydrogen atoms each independently substituted with any one selected from the group consisting of -F, -Cl, -Br, -I, (C₁-C₁₀) alkyl group, amino group (-NH₂), nitro group (-NO₂), -CF₃, -CF₂H, -CFH₂, -CCl₂, -CCl₂H, -CClH₂, - (CH₂)ₙCl, (C₁-C₁₀) alkoxy group, hydroxyl group (-OH), (C₁-C₁₀) alkoxyaryl group, and C₆-C₃₀ aryl group.

In the present invention, any benzene ring present in Chemical Formula (II-4) may be characterized by having one or more hydrogen atoms each independently substituted with halogen, C₁₋₁₀ alkyl, or C₁₋₁₀ haloalkyl.

In the present invention, the compound may be characterized by being represented by the following Chemical Formula (III):

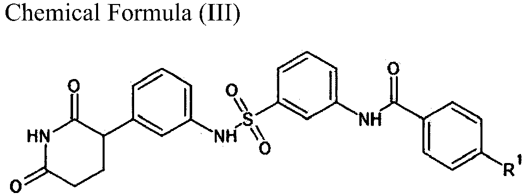

In Chemical Formula (III),
R¹ is a halogen (X), C₁₋₁₀ alkyl, or C₁₋₁₀ haloalkyl.

In the present invention, the compound, an optical isomer thereof, a racemic mixture thereof, a hydrate thereof, a solvate thereof, or a pharmaceutically acceptable salt thereof may be characterized by having binding affinity for cereblon.

The present invention also provides any one of the compounds shown in Table 1, an optical isomer thereof, a racemic mixture thereof, a hydrate thereof, a solvate thereof, or a pharmaceutically acceptable salt thereof.

The present invention is also directed to providing a pharmaceutical composition for preventing or treating a benign tumor or a cancer, comprising the compound, an optical isomer thereof, a racemic mixture thereof, a hydrate thereof, a solvate thereof, or a pharmaceutically acceptable salt thereof as an active ingredient.

In the present invention, the cancer may be a hematologic cancer or a solid cancer.

In the present invention, the hematologic cancer may be selected from the group consisting of acute leukemia, chronic leukemia, multiple myeloma, Hodgkin's lymphoma, and non-Hodgkin's lymphoma.

In the present invention, the solid cancer may be selected from the group consisting of melanoma, myosarcoma, multiple myeloma, head and neck cancer, nasopharyngeal cancer, esophageal cancer, gastroesophageal junction cancer, esophageal adenocarcinoma, gastric cancer, bladder cancer, colorectal cancer, colon cancer, rectal cancer, small intestinal cancer, anal cancer, liver cancer, gallbladder cancer, cholangiocarcinoma, biliary tract cancer, pancreatic cancer, thyroid cancer, parathyroid cancer, lung cancer, breast cancer, ovarian cancer, fallopian tube cancer, uterine cancer, vaginal cancer, vulvar cancer, penile cancer, renal cancer, adrenal cancer, urothelial carcinoma, prostate cancer, testicular tumor, bone and soft tissue sarcoma, skin cancer, glioma, brain tumor, spinal tumor, Kaposi's sarcoma, squamous cell carcinoma, pleural mesothelioma, and primary peritoneal cancer.

The present invention also provides a composition for inhibiting cereblon activity, comprising the compound, an optical isomer thereof, a racemic mixture thereof, a hydrate thereof, or a solvate thereof.

The present invention provides a method for preventing or treating a cereblon activity-related disease, such as a benign tumor or cancer, comprising administering to a patient in need thereof a compound, an optical isomer thereof, a racemic mixture thereof, a hydrate thereof, a solvate thereof, or a pharmaceutically acceptable salt thereof.

The present invention also provides the compound, an optical isomer thereof, a racemic mixture thereof, a hydrate thereof, a solvate thereof, or a pharmaceutically acceptable salt thereof for used in prevention or treatment of a cereblon activity-related disease, such as a benign tumor or cancer.

The present invention further provides the use of the compound, an optical isomer thereof, a racemic mixture thereof, a hydrate thereof, a solvate thereof, or a pharmaceutically acceptable salt thereof in the manufacture of a medicament for preventing or treating a cereblon activity-related disease, such as a benign tumor or cancer.

### ADVANTAGEOUS EFFECT

The compound according to the present invention has a superior ability to bind to cereblon and thus can act as a novel immunomodulator for treating tumors or cancers.

### BEST MODES FOR CARRYING OUT THE INVENTION

Unless otherwise defined, all technical and scientific terms used in this specification have the same meaning as commonly understood by a person skilled in the art to which the present invention pertains. In general, the nomenclature used in the present specification and the experimental methods described below are well known and commonly used in the art.

In the present invention, compounds having excellent binding affinity for cereblon were selected, and their tumor cell apoptosis effects were verified.

Accordingly, in one aspect, the present invention relates to a compound represented by the following Chemical Formula (I), or an optical isomer thereof, a racemic mixture thereof, a hydrate thereof, a solvate thereof, or a pharmaceutically acceptable salt thereof:

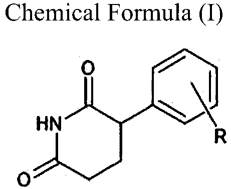

In Chemical Formula (I), R may independently be hydrogen (H), halogen (X), hydroxyl (OH), amino (NH₂), alkyl, alkylamine, aminocarbonylalkyl, aminocarbonylaryl, aminosulfonylalkyl, aminocarbonylalcohol, aminosulfonylaryl, aminosulfonylalkyl, aminoalkylaryl, aminocarbonylaminoaryl, alkylaminocarbonylalkoxy, aminosulfonylaminocarbonylaminoaryl, aminoarylalkylcarboxylalkyl, aminoarylalkylcarbonylaminoaryl, aminoarylalkylaminocarboxylalkyl, aminoarylalkylaminocarbonylaminoaryl, aminosulfonylarylaminocarbonylarylalkoxy, aminoarylalkylcarbonylaminoarylalkoxy, aminoarylalkylcarbonylaminoalkylcarboxylalkyl, aminoarylalkylcarbonylaminoalkylcarboxylic acid, aminoarylaminocarbonylalkylcarbonylaminoaryl, aminoarylaminocarbonylaryl, or aminosulfonylarylaminocarbonylaryl, but is not limited thereto.

In one embodiment, R may independently be hydrogen (H), halogen (X), hydroxyl (OH), NHCOC₁₋₆ alkyl, NHCOaryl, amino (NH₂), C₄₋₈ cycloalkyl, NH-C₄₋₈ heterocycloalkyl, NHCOC₃₋₇ heterocycloalkyl, NHCOC₂OC₂ heterocycloalkyl, NHSO₂C₃₋₇ heterocycloalkyl, NHSO₂C₆H₅, NHSO₂C₁₀H₆, or NHCOC₂H₄OH, but is not limited thereto.

In some embodiments, in Chemical Formula (I), R may independently be aminosulfonylphenyl (NHSO₂C₆H₅), aminoalkylphenyl (NHCH₂C₆H₅), aminophenylaminocarbonylphenyl (NHC₆H₄NHCOC₆H₅), aminosulfonylphenylaminocarbonylphenyl (NHSO₂C₆H₄NHCOC₆H₅), or aminocarbonylphenyl (NHCOC₆H₅), but is not limited thereto.

In another embodiment, in Chemical Formula (I), R may be a heterocycloalkyl comprising C(O)NH and may form a bicyclic fused ring structure with a benzene ring.

For example, in Chemical Formula (I), the benzene ring may be an aralkyl fused with a heterocycloalkyl.

In yet another embodiment, in Chemical Formula (I), R may be a heterocycloalkyl comprising C(O)NH, and the heterocycloalkyl may form a bicyclic fused ring with a benzene ring and may be a compound in which one carbon atom of the heterocycloalkyl is substituted with oxygen. For example, the compound of Chemical Formula (I) may have a structure comprising the following bicyclic fused ring:

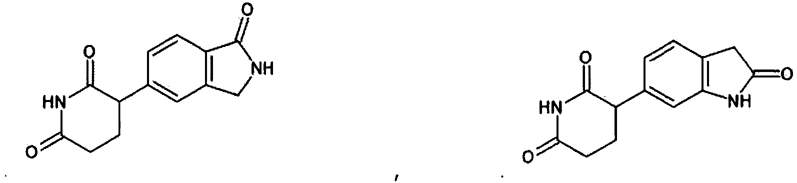

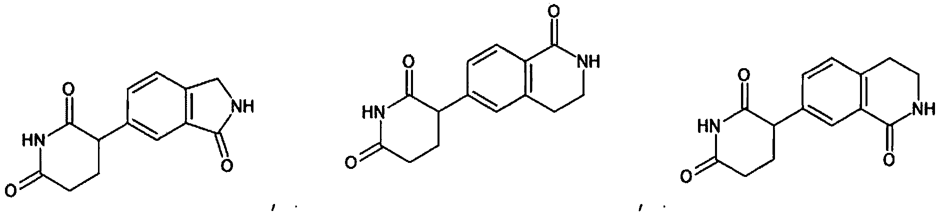

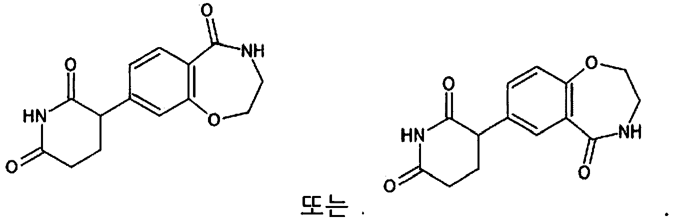

In the present invention, the compound of Chemical Formula (I) may be AEV40032, AEV40040, AEV40041, AEV40062, AEV40078, or AEV40079.

In the bicyclic fused ring, the heterocycloalkyl comprising CONH may have a hydrogen atom of the CONH group that is unsubstituted or substituted.

In the bicyclic fused ring, the heterocycloalkyl comprising CONH may have the hydrogen atom of the CONH group substituted with an alkyl group ( 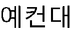 , CH₃), an alkoxy group, an aryl group ( 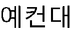 , C₆H₅), an alkylalkoxy group ( 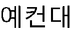 , C₂H₄OCH₃), an alkylaryl group ( 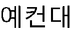 , CH₂C₆H₅, C₂H₄C₆H₅), an arylamine group (C₆H₄NH₂), SO₂C₆H₄Cl, SO₂CH₃, an alkylarylalkoxy group (e.g., CH₃C₆H₄OCH₃), C₆H₄(C₃₋₇ cycloalkyl)CH₃, C₆H₄NHCOC₅H₉O, C₆H₄NHCO(C₆H₄)C₁₋₇ alkyl, C₆H₄NHCO(C₃₋₇ cycloalkyl)CONHC₆H₄X, or C₆H₄NHCOC₆H₃X₂.

In the present invention, the compound of Chemical Formula (I) may be AEV40036, AEV40042, AEV40049, AEV40050, AEV40059, AEV40077, AEV40085, AEV40086, AEV40206, AEV40212, AEV40213, AEV40214, AEV40215, AEV40216, or AEV40217.

In another embodiment, in Chemical Formula (I), R may be a heterocyclopentyl in which carbons of a cyclopentyl ring are substituted with nitrogen (N) and oxygen (O), wherein the heterocyclopentyl forms a bicyclic fused ring with a benzene ring, the nitrogen (N) and carbon (C) in the heterocyclopentyl form a double bond, and a hydrogen (H) atom bonded to a carbon (C) positioned between the nitrogen (N) and oxygen (O) may be unsubstituted or substituted with a heterocyclohexyl in which one carbon is substituted with nitrogen (N) or with a heterocyclohexyl in which one carbon is substituted with nitrogen (N) and another carbon is substituted with oxygen (O).

For example, the compound of Chemical Formula (I) may have a structure comprising the following bicyclic fused ring:

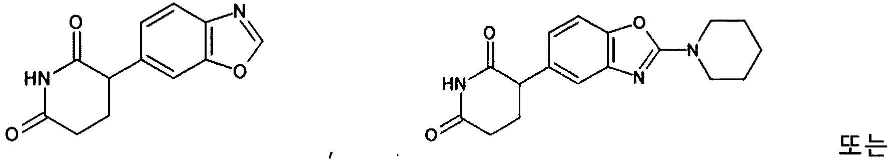

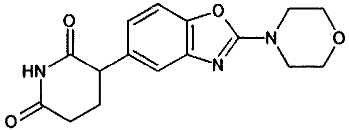

In the present invention, the compound of Chemical Formula (I) may be AEV40203, AEV40207, or AEV40208.

In one embodiment, R of Chemical Formula (I) may be any one selected from the following group:

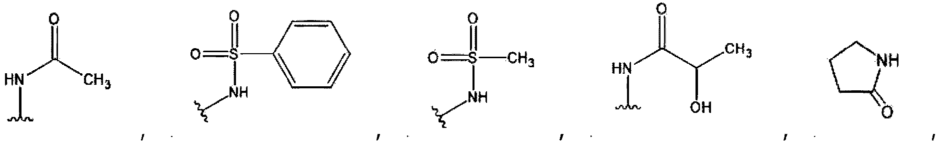

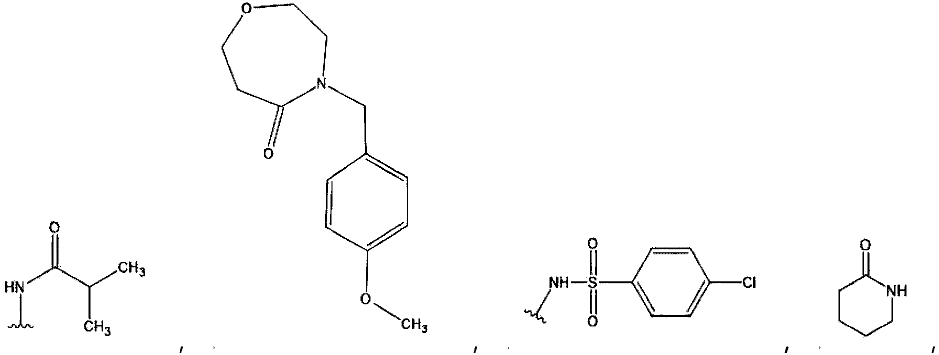

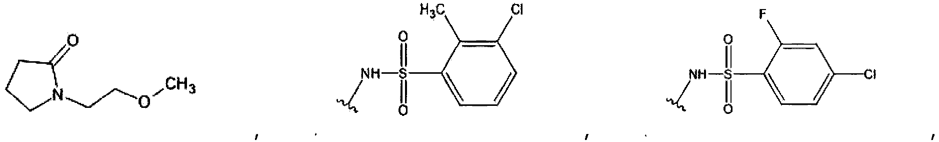

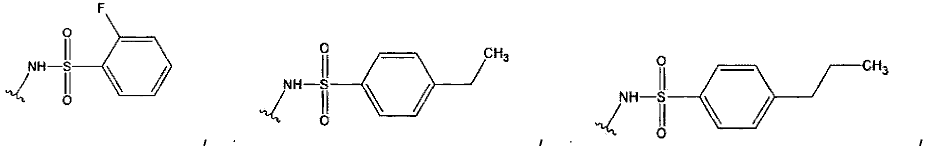

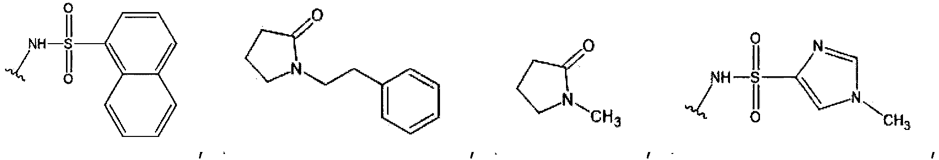

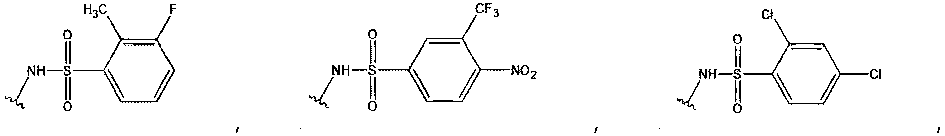

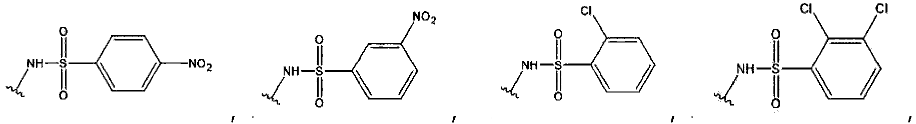

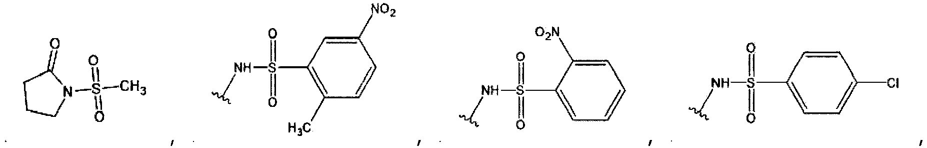

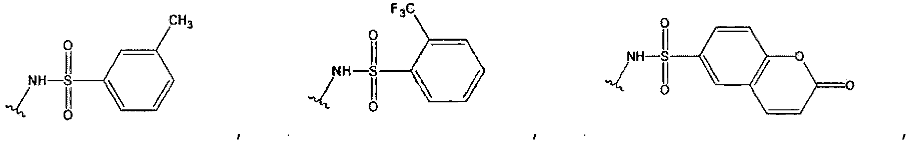

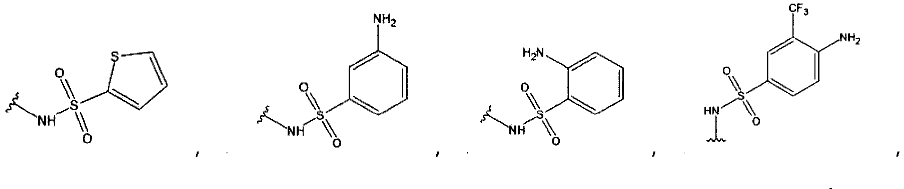

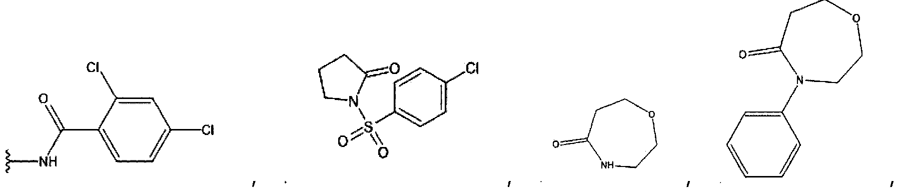

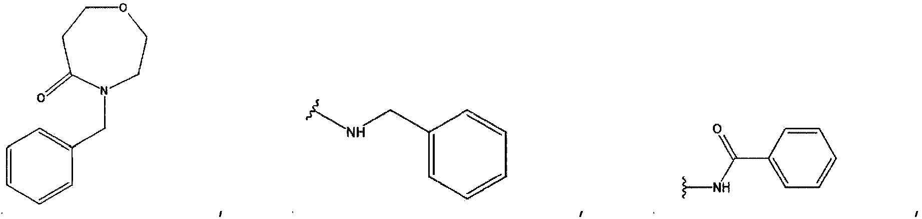

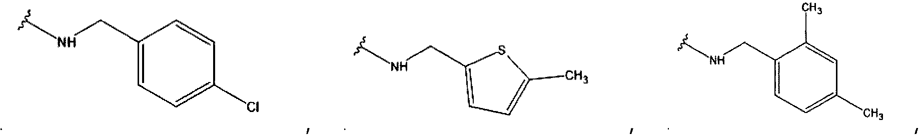

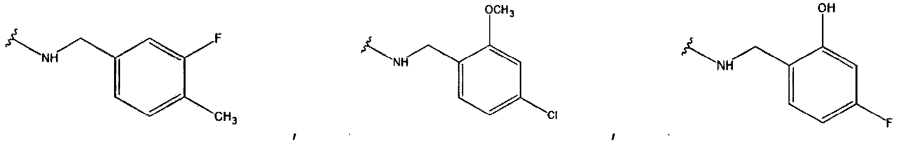

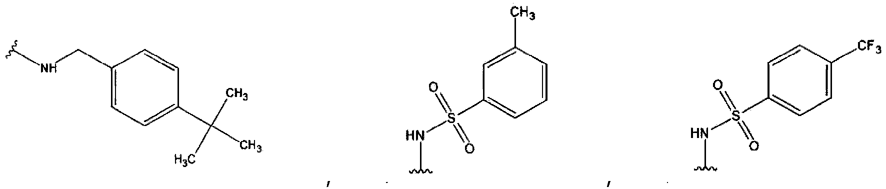

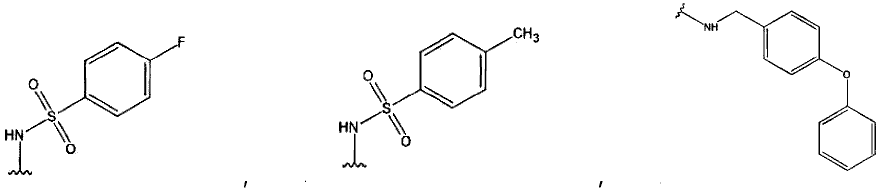

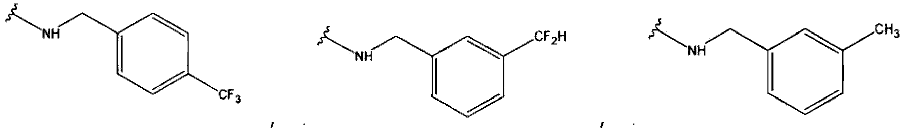

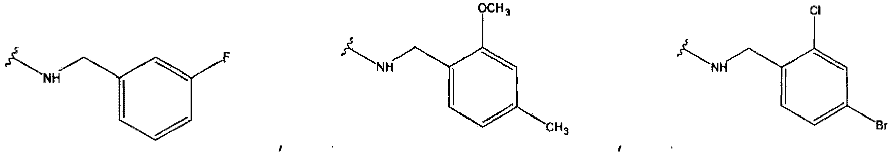

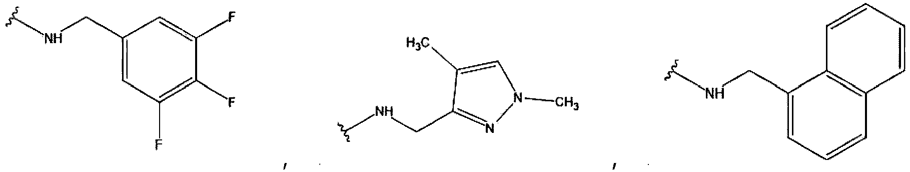

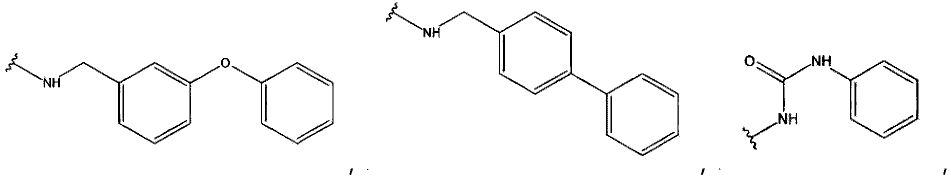

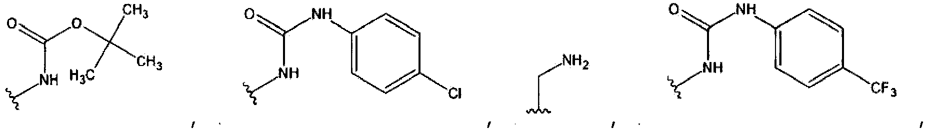

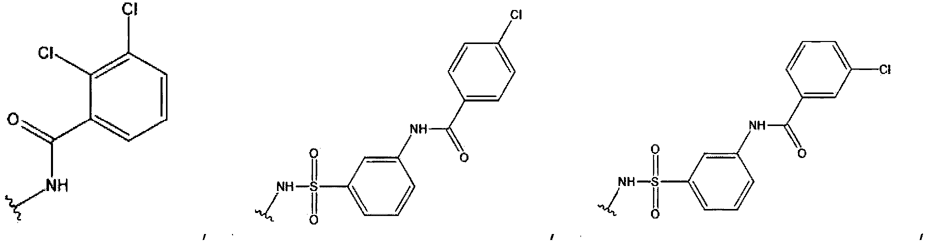

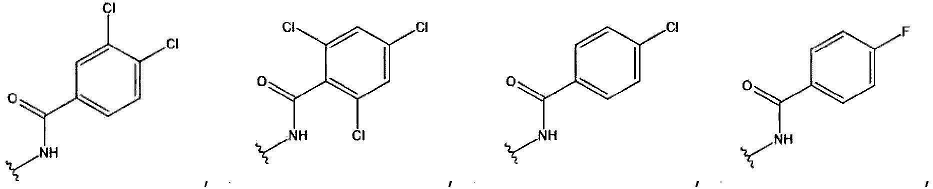

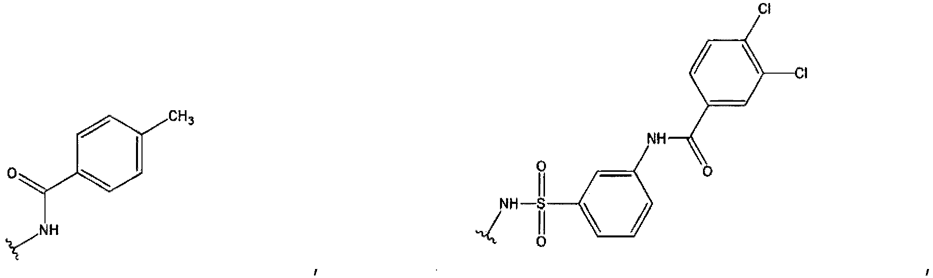

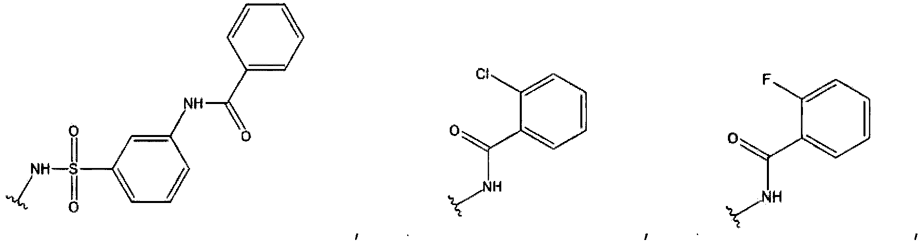

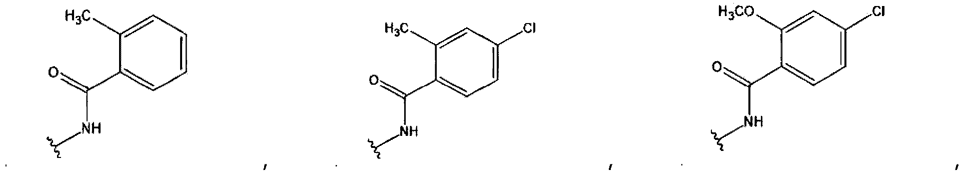

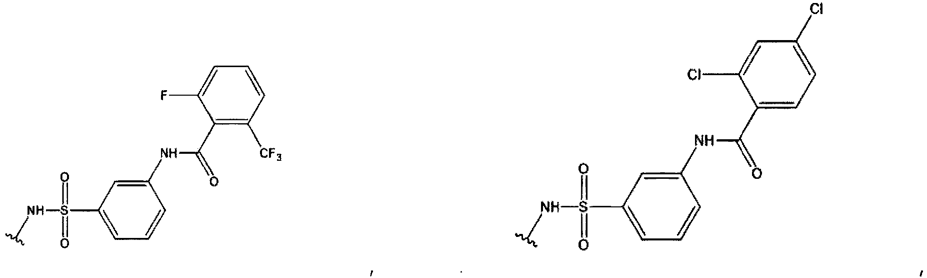

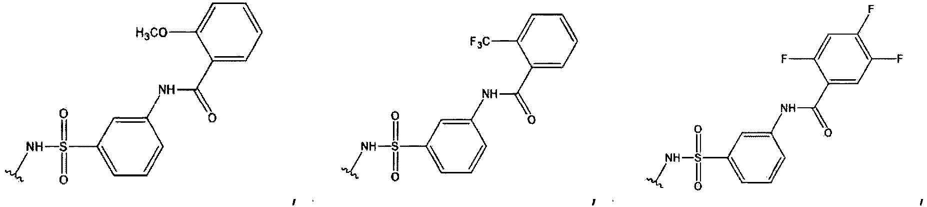

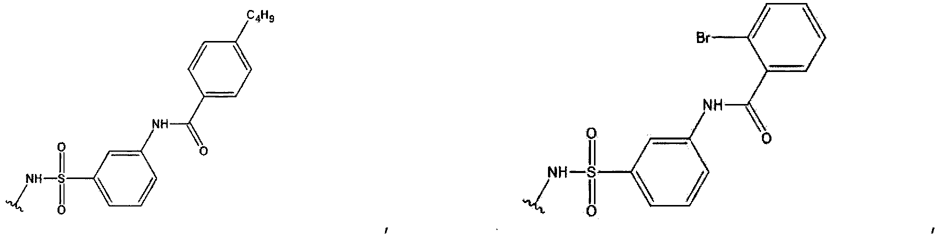

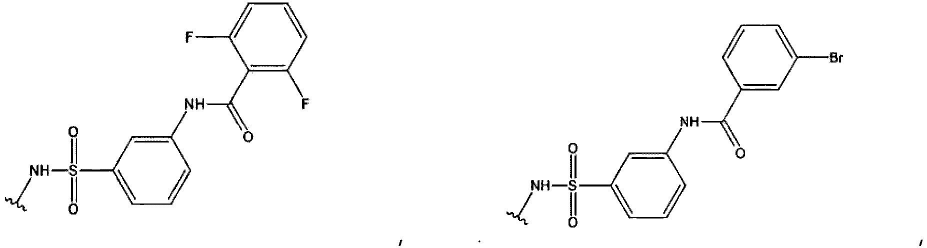

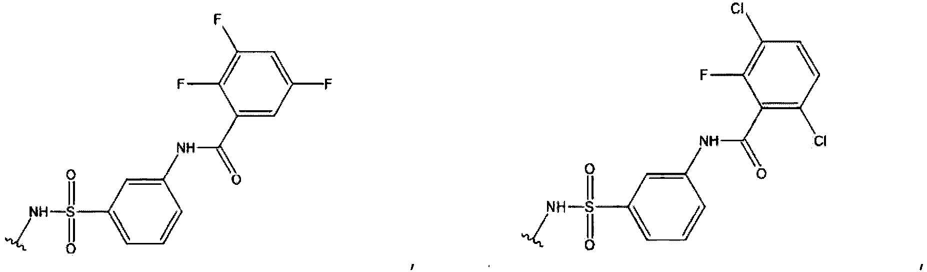

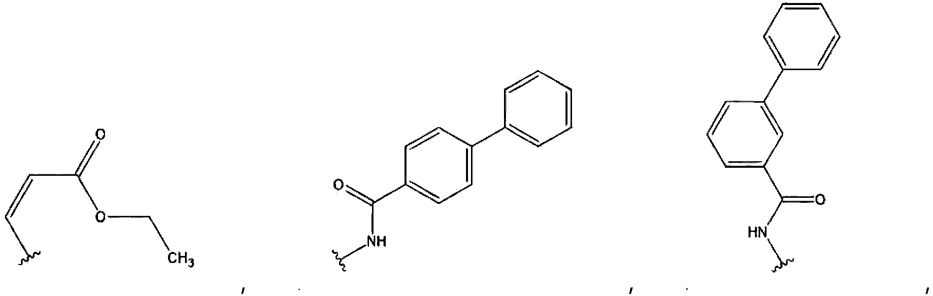

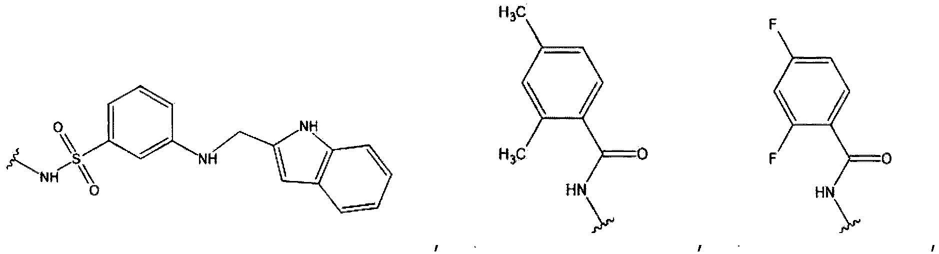

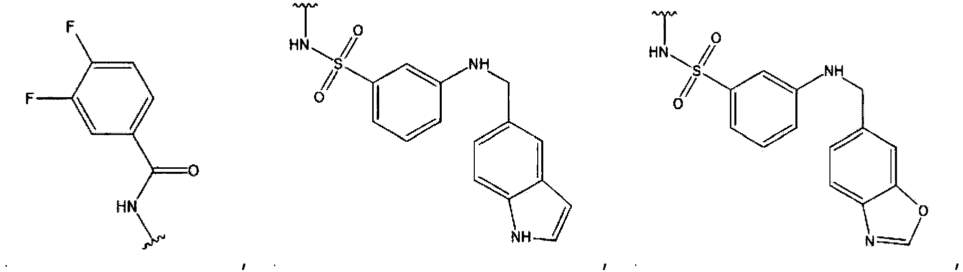

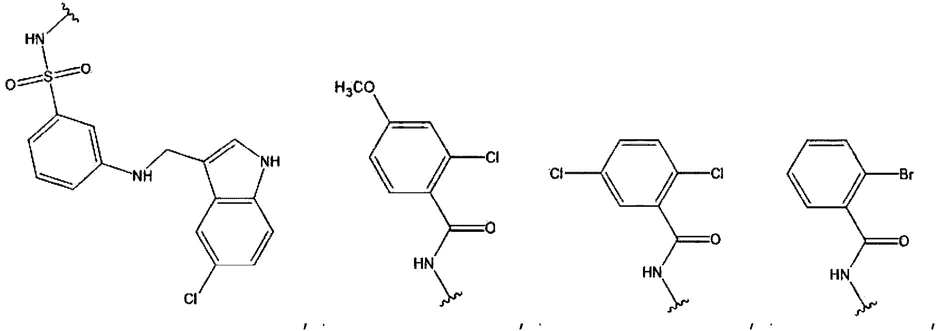

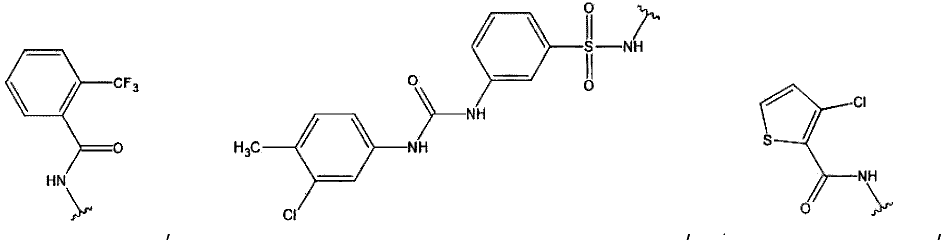

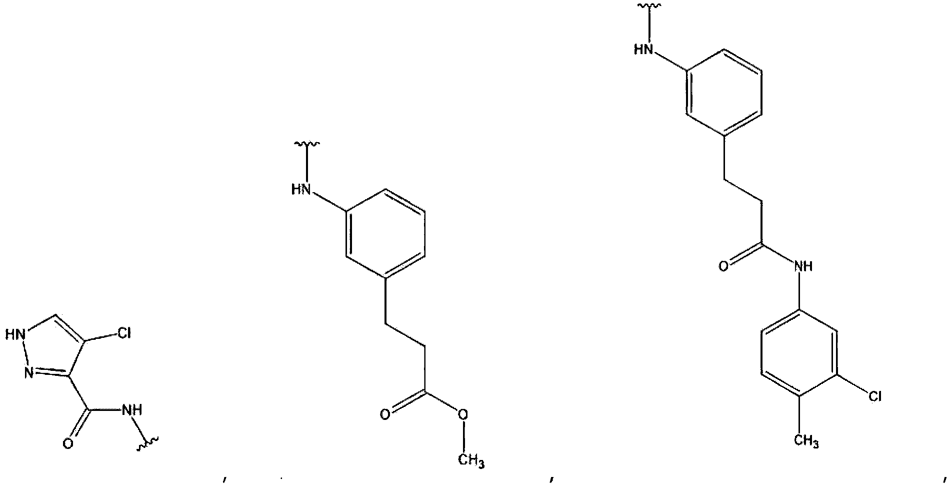

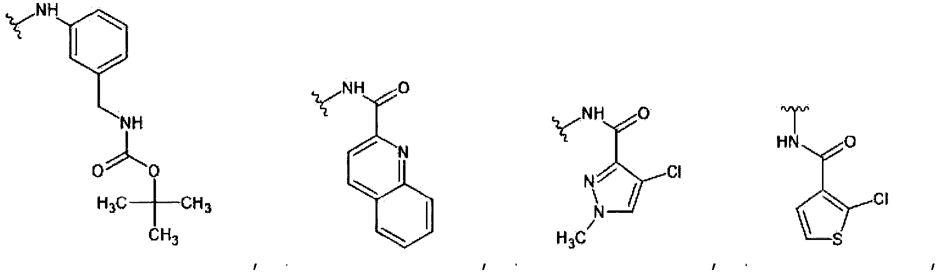

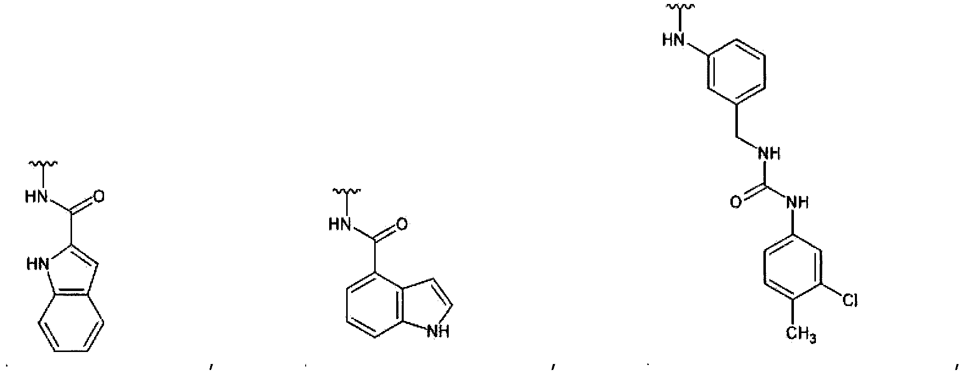

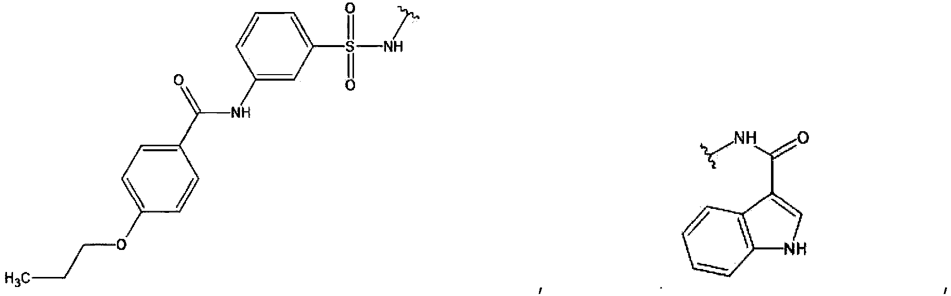

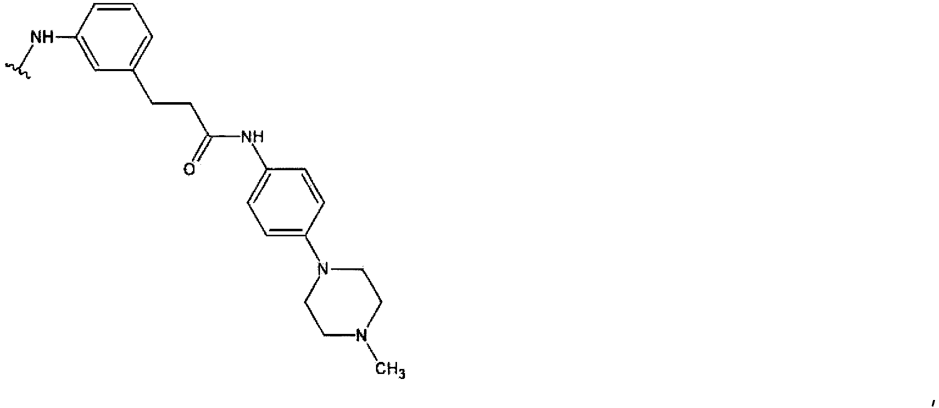

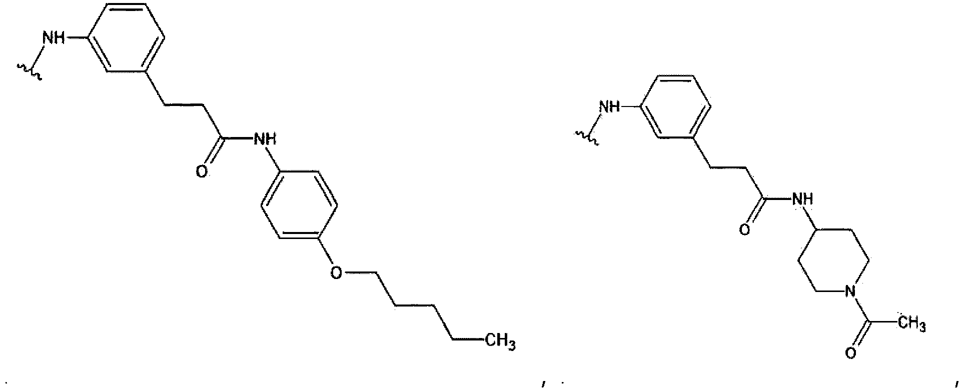

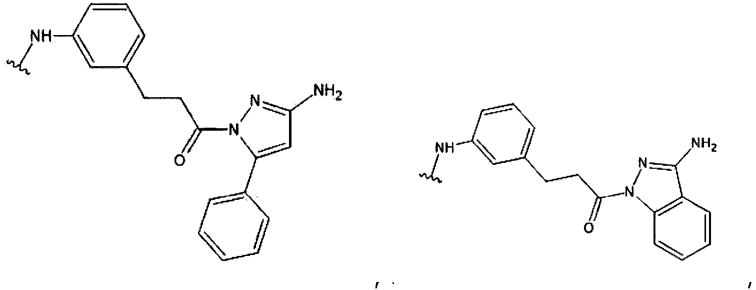

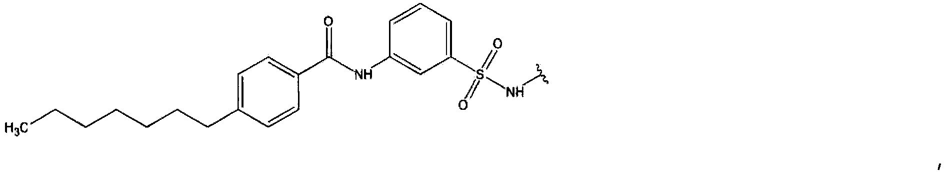

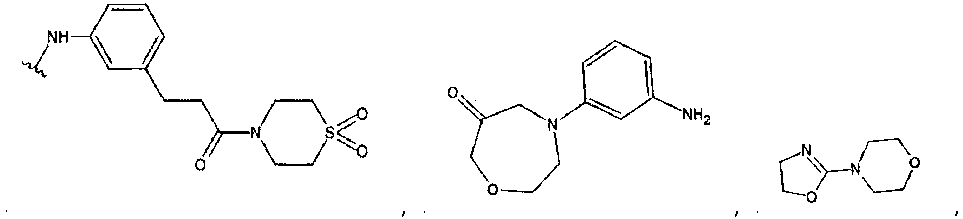

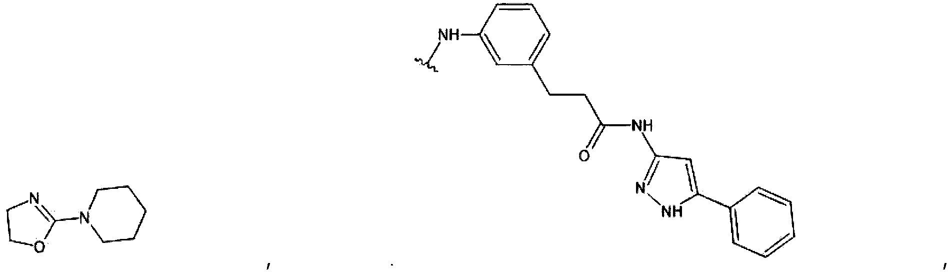

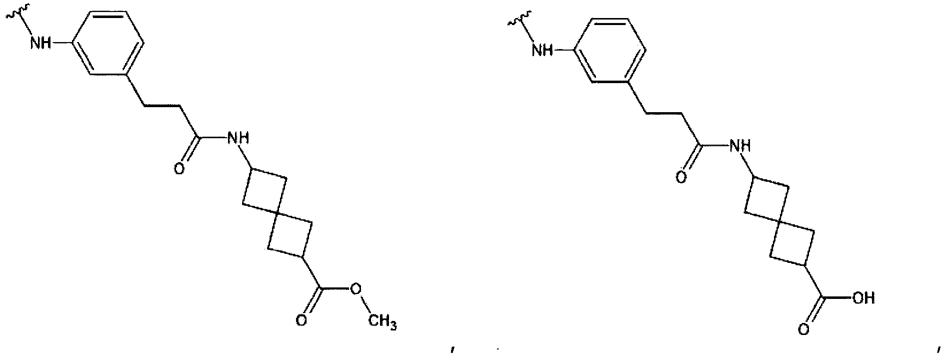

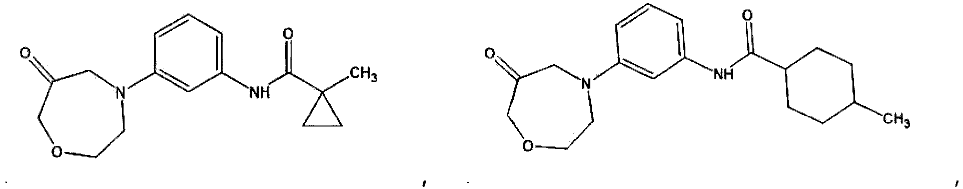

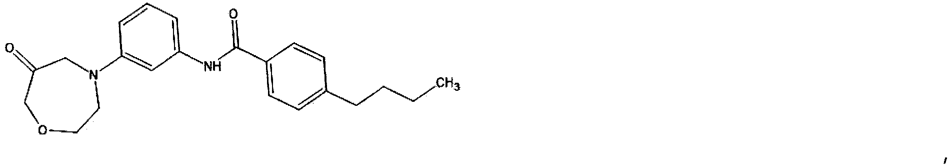

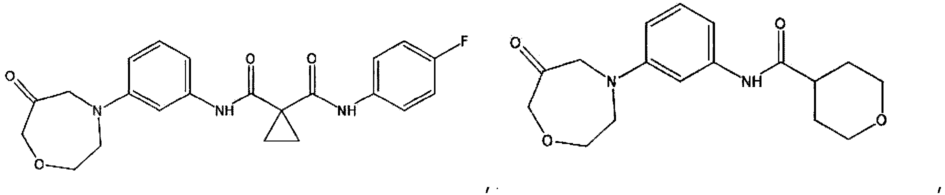

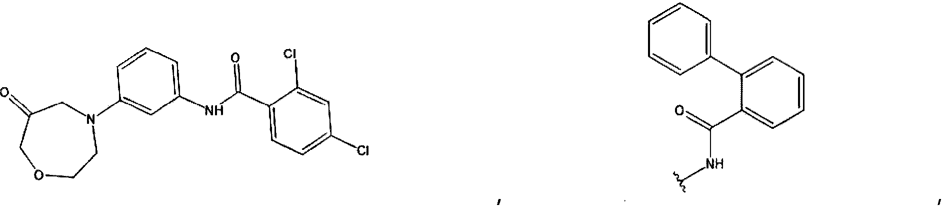

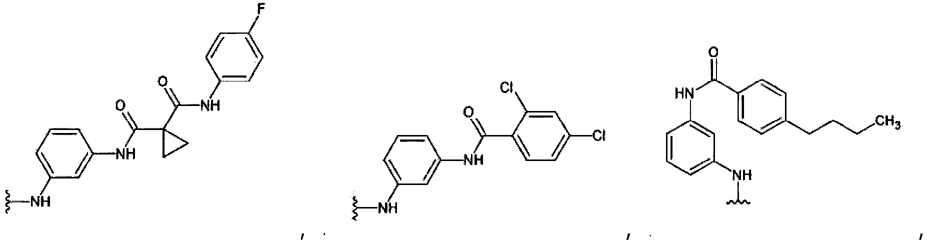

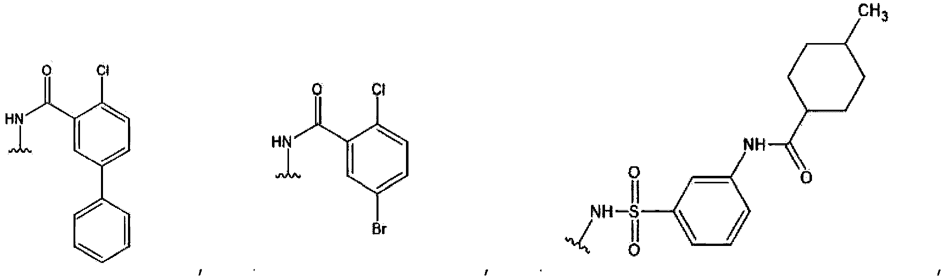

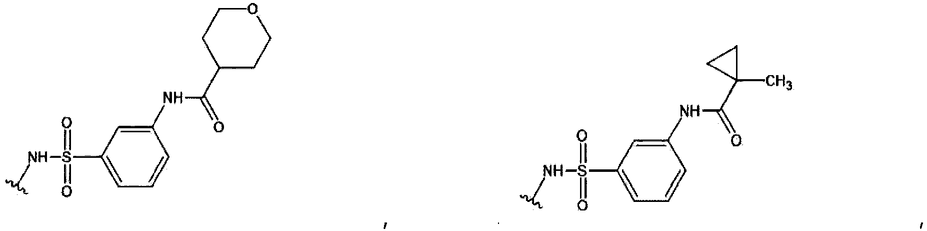

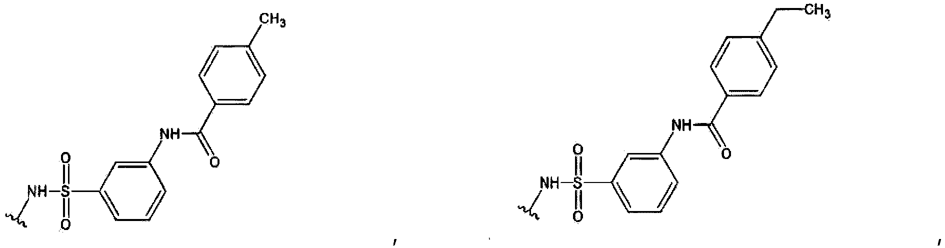

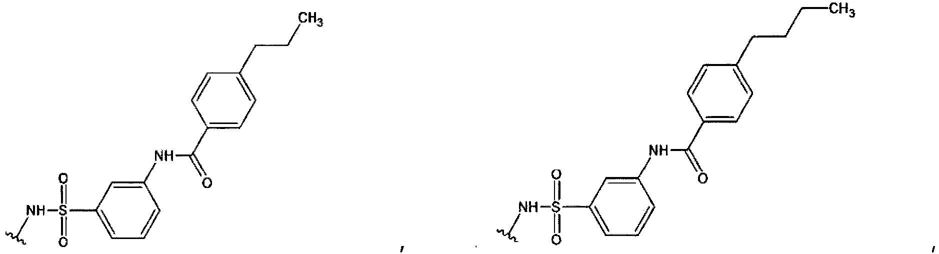

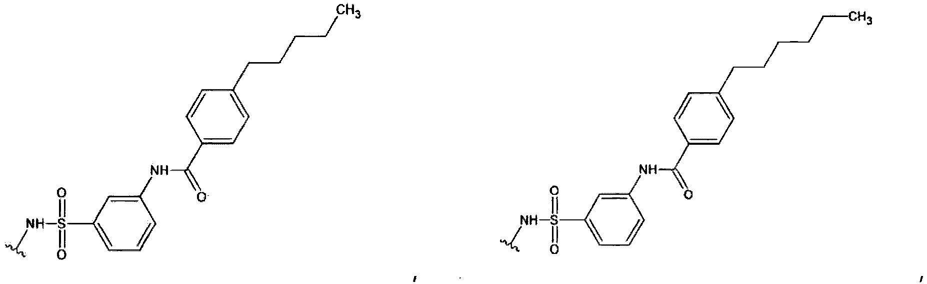

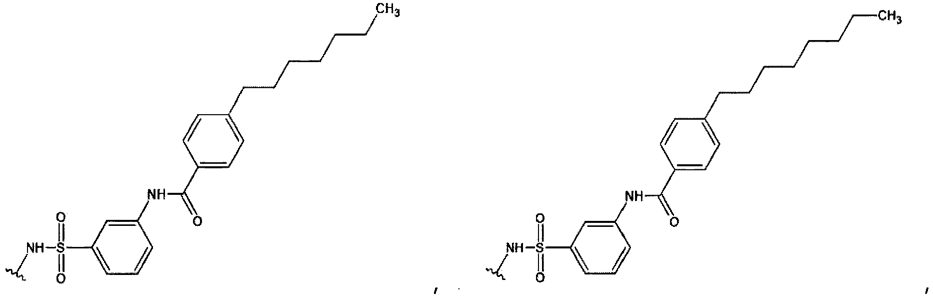

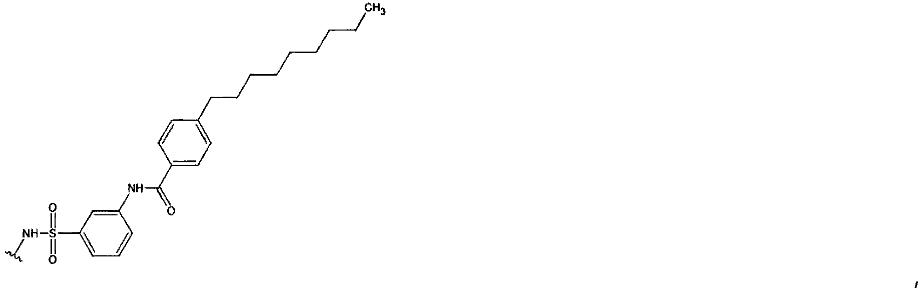

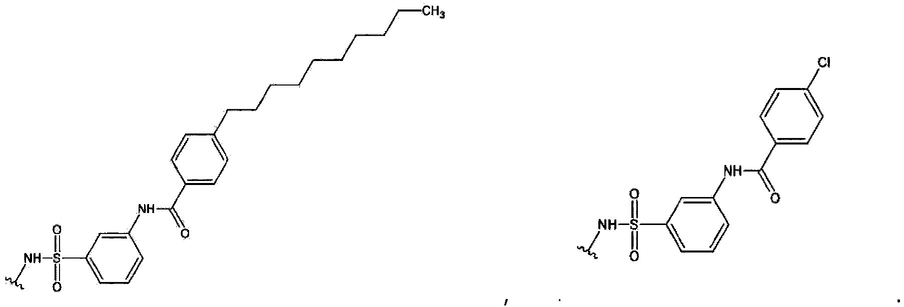

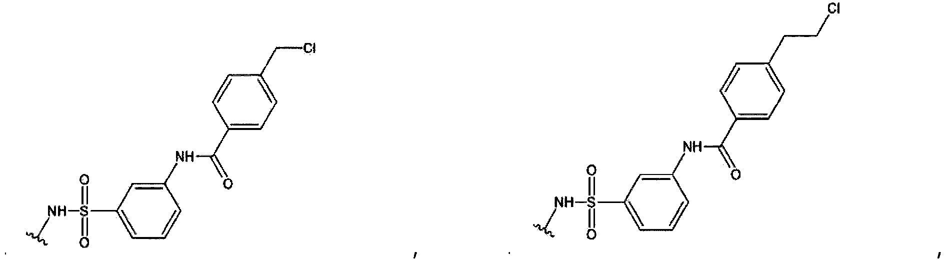

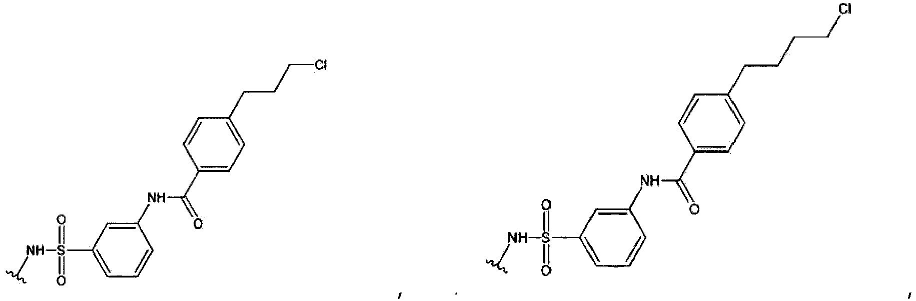

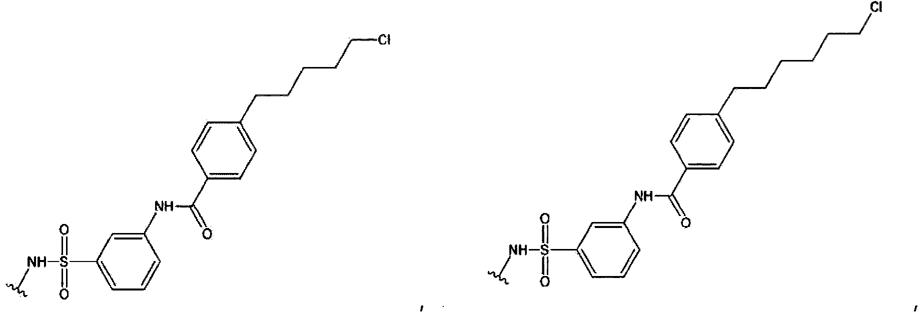

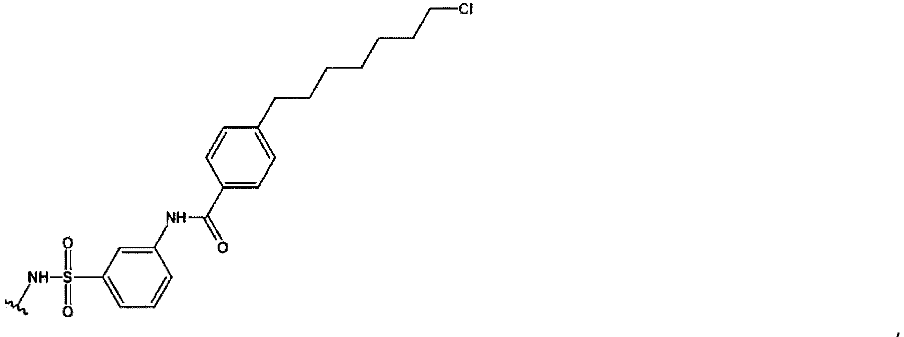

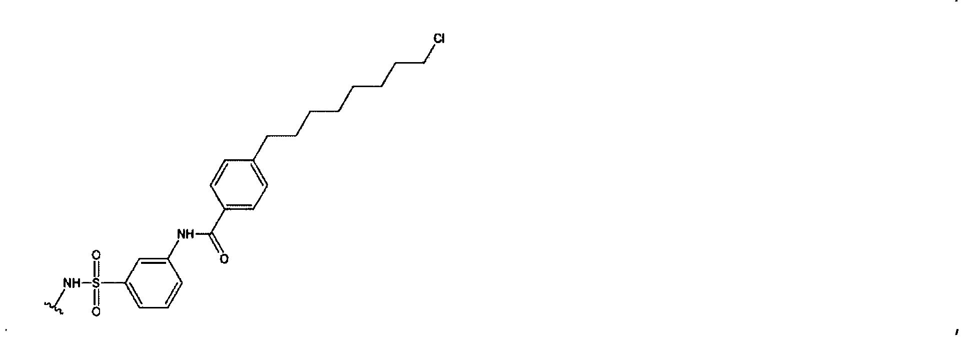

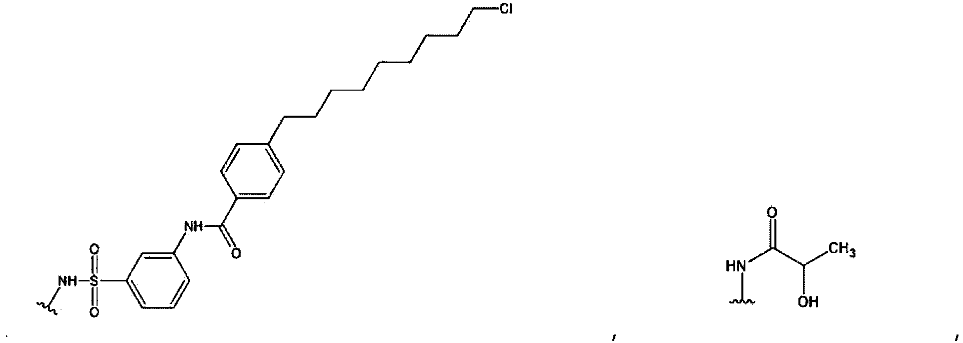

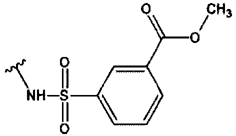

In the present invention, Chemical Formula (I) may be characterized by being represented by the following Chemical Formula (II), but is not limited thereto:

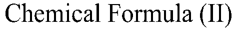

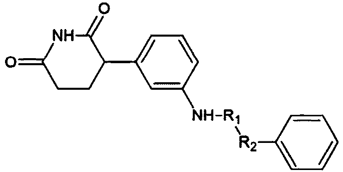

In Chemical Formula (II),
R₁ is absent, CH₂, CO, or SO₂;
R₂ is absent or C₆H₄NHCO; and
any benzene ring present in Chemical Formula (II) is an unsubstituted benzene ring or a benzene ring having one or more hydrogen atoms substituted.

In the present invention, the compound represented by Chemical Formula (II) may be characterized by being represented by any one of the Chemical formulae selected from the group consisting of Chemical Formulae (II-1) to (II-5), but is not limited thereto:

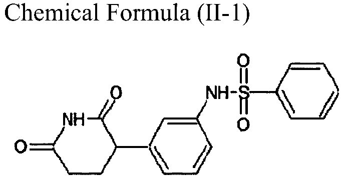

Any benzene ring present in Chemical Formula (II-1) may be an unsubstituted benzene ring or a benzene ring in which one or more hydrogens are substituted.

In some embodiments, any benzene ring present in Chemical Formula (II-1) may have one hydrogen atom substituted with a halogen (e.g., F, Cl, Br, or I), an alkyl group (branched or unbranched) such as methyl, ethyl, propyl, butyl, or pentyl, CF₃, NO₂, NH₂, NHCO(cycloalkyl)alkyl (e.g., NHCOC₆H₁₀CH₃, NHCOC₃H₄CH₃, or NHCOC5H9O), or a carboxyl group (e.g., COOCH₃). In another embodiment, one benzene ring or both benzene rings present in Chemical Formula (II-1) may have a total of two or more hydrogens independently substituted with halogen, alkyl, CF₃, NO₂, or NH₂. For example, in one benzene ring or in both benzene rings present in Chemical Formula (II-1), two hydrogens may be substituted with halogen and halogen, halogen and alkyl, CF₃ and NO₂, alkyl and NO₂, CF₃ and NH₂, or alkyl and NH₂.

In the present invention, the compound of Chemical Formula (II-1) may be any one of AEV40025, AEV40037, AEV40038, AEV40043, AEV40044, AEV40045, AEV40046, AEV40047, AEV40052, AEV40053, AEV40054, AEV40055, AEV40056, AEV40057, AEV40058, AEV40060, AEV40061, AEV40063, AEV40069, AEV40070, AEV40073, AEV40074, AEV40075, AEV40099, AEV40100, AEV40101, AEV40224, AEV40225, AEV40226, or AEV40227 shown in Table 1.

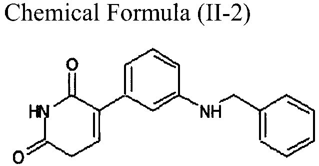

Any benzene ring present in Chemical Formula (II-2) may be an unsubstituted benzene ring or a benzene ring in which one or more hydrogens are substituted.

In some embodiments, any benzene ring present in Chemical Formula (II-2) may have one hydrogen atom substituted with a halogen (e.g., F, Cl, Br, or I), an alkyl group (branched or unbranched) such as methyl, ethyl, propyl, butyl, or pentyl, a hydroxyl group (OH), an alkoxy group (e.g., methoxy, ethoxy, or phenoxy), CX₃, CX₂H, CXH₂, or an aryl group. In another embodiment, one benzene ring or both benzene rings present in Chemical Formula (II-2) may have a total of two or more hydrogens independently substituted with halogen, alkyl, hydroxyl, alkoxy, CX₃, CX₂H, CXH₂, or aryl. For example, in one benzene ring or in both benzene rings present in Chemical Formula (II-2), two hydrogens may be substituted with halogen and halogen, halogen and alkyl, alkyl and alkyl, halogen and alkoxy, halogen and hydroxyl, or alkyl and alkoxy, and in one benzene ring or in both benzene rings present in Chemical Formula (II-2), three hydrogens may be substituted with three halogens.

In the present invention, the compound of Chemical Formula (II-2) may be any one of AEV40087, AEV40090, AEV40094, AEV40095, AEV40096, AEV40097, AEV40098, AEV40102, AEV40103, AEV40104, AEV40105, AEV40106, AEV40107, AEV40108, AEV40109, AEV40110, AEV40121, or AEV40122 shown in Table 1.

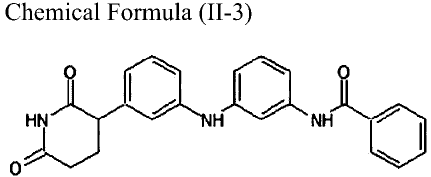

Any benzene ring present in Chemical Formula (II-3) may be an unsubstituted benzene ring or a benzene ring in which one or more hydrogens are substituted.

In some embodiments, any benzene ring present in Chemical Formula (II-3) may have one hydrogen atom substituted with a halogen (e.g., F, Cl, Br, or I) or an alkyl group (branched or unbranched) such as methyl, ethyl, propyl, butyl, or pentyl. In another embodiment, one benzene ring or both benzene rings present in Chemical Formula (II-3) may have a total of two or more hydrogens independently substituted with a halogen (e.g., F, Cl, Br, or I) or an alkyl group (branched or unbranched) such as methyl, ethyl, propyl, butyl, or pentyl. For example, in one benzene ring or in both benzene rings present in Chemical Formula (II-3), two hydrogens may be substituted with halogen and halogen.

In the present invention, the compound of Chemical Formula (II-3) may be AEV40220 or AEV40221 shown in Table 1.

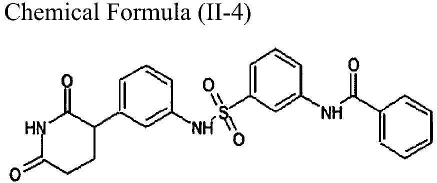

Any benzene ring present in Chemical Formula (II-4) may be an unsubstituted benzene ring or a benzene ring in which one or more hydrogens are substituted.

In some embodiments, any benzene ring present in Chemical Formula (II-4) may have one hydrogen atom substituted with a halogen (e.g., F, Cl, Br, or I), an alkyl group (branched or unbranched) such as methyl, ethyl, propyl, butyl, pentyl, hexyl, heptyl, octyl, nonyl, or decyl, an alkoxy group (e.g., methoxy, ethoxy, propoxy, butoxy, or pentyloxy), a haloalkyl group (CH₂X, C₂H₄X, C₃H₆X, C₄H₈X, C₅H₁₀X, C₆H₁₂X, C₇H₁₄X, C₈H1₆X, C₉H₁₈X, or C₁₀H₂₀X), CX₃, CX₂H, or CXH₂. In another embodiment, one benzene ring or both benzene rings present in Chemical Formula (II-4) may have a total of two or more hydrogens independently substituted with halogen, an alkyl group (branched or unbranched), or CX₃. For example, in one benzene ring or in both benzene rings present in Chemical Formula (II-4), two hydrogens may be substituted with halogen and halogen or halogen and CX₃, and in one benzene ring or in both benzene rings present in Chemical Formula (II-4), three hydrogens may be substituted with three halogens.

In the present invention, the compound of Chemical Formula (II-4) may be any one of AEV40135, AEV40136, AEV40142, AEV40143, AEV40149, AEV40150, AEV40151, AEV40152, AEV40153, AEV40154, AEV40155, AEV40156, AEV40157, AEV40158, AEV40159, AEV40193, AEV40204, AEV40264, AEV41001, AEV41002, AEV41003, AEV41004, AEV41005, AEV41009, AEV41010, AEV41011, AEV41013, AEV41083, AEV41047, or AEV41048 shown in Table 1.

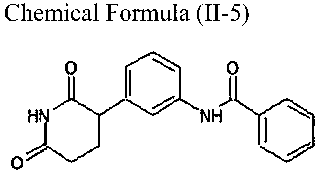

Any benzene ring present in Chemical Formula (II-5) may be an unsubstituted benzene ring or a benzene ring in which one or more hydrogens are substituted.

In some embodiments, any benzene ring present in Chemical Formula (II-5) may have one hydrogen atom substituted with a halogen (e.g., F, Cl, Br, or I), an alkyl group (branched or unbranched) such as methyl, ethyl, propyl, butyl, or pentyl, an alkoxy group (e.g., methoxy, ethoxy, propoxy, butoxy, or pentyloxy), CX₃, CX₂H, CXH₂, or an aryl group (e.g., phenyl). In another embodiment, one benzene ring or both benzene rings present in Chemical Formula (II-5) may have a total of two or more hydrogens independently substituted with a halogen (e.g., F, Cl, Br, or I), an alkyl group (branched or unbranched) such as methyl, ethyl, propyl, butyl, or pentyl, an alkoxy group (e.g., methoxy, ethoxy, propoxy, butoxy, or pentyloxy), CX₃, CX₂H, CXH₂, or an aryl group (e.g., phenyl). For example, in one benzene ring or in both benzene rings present in Chemical Formula (II-5), two hydrogens may be substituted with halogen and halogen, halogen and alkyl, halogen and alkoxy, alkyl and alkyl, or halogen and aryl, and in one benzene ring or in both benzene rings present in Chemical Formula (II-5), three hydrogens may be substituted with three halogens.

In the present invention, the compound of Chemical Formula (II-5) may be any one of AEV40076, AEV40088, AEV40134, AEV40137, AEV40138, AEV40139, AEV40140, AEV40141, AEV40144, AEV40145, AEV40146, AEV40147, AEV40148, AEV40162, AEV40164, AEV40165, AEV40166, AEV176, AEV40178, AEV40179, AEV40180, AEV40218, AEV40222, or AEV40223 shown in Table 1.

In the present invention, any benzene ring present in Chemical Formula (I) may be characterized by having one or more hydrogen atoms each independently substituted with alkyl, cycloalkyl, aryl, heterocyclyl, cycloalkylalkyl, aralkyl, heterocyclylalkyl, heterocycloalkylalkyl, halogen, hydroxyalkyl, alkoxy, alkoxyalkyl, amino, amido, urea, sulfonyl, hydroxy, aldehyde, carboxy, haloalkyl, alkoxy, cycloalkyloxy, aryloxy, heterocyclyloxy, heterocycloalkyloxy, cycloalkylalkyloxy, aralkyloxy, heterocyclylalkyloxy, heterocycloalkylalkyloxy; oxo (=O); amino, alkylamino, cycloalkylamino, arylamino, heterocyclylamino, heterocycloalkylamino, cycloalkylalkylamino, aralkylamino, heterocyclylalkylamino, heterocycloalkylalkylamino; imino; imido; amidino; guanidino; enamino; acylamino; sulfonylamino; urea, nitrourea; oxime; hydroxylamino; alkoxyamino; aralkoxyamino; hydrazino; hydrazido; hydrazono; azido; nitro; thio (-SH), alkylthio; =S; sulfinyl; sulfonyl; aminosulfonyl; phosphonate; phosphinyl; acyl; formyl; carboxy; ester; carbamate; amido; cyano; isocyanato; isothiocyanato; cyanato; thiocyanato; hydroxyl; alkoxy; alkoxyalkyl; amino; alkylamino; carboxy; nitro; cyano; thiol; thioether; imine; imide; amidine; guanidine; enamine; aminocarbonyl; acylamino; phosphonate; phosphine; thiocarbonyl; sulfinyl; sulfone; sulfonamide; ketone; aldehyde; ester; urea; urethane; oxime; hydroxylamine; alkoxyamine; aralkoxyamine; N-oxide; hydrazine; hydrazide; hydrazone; azide; isocyanate; isothiocyanate; cyanate; thiocyanate; B(OH)₂; or O(alkyl)aminocarbonyl, but is not limited thereto.

In the present invention, any benzene ring present in any one of Chemical Formulas (II-1) to (II-5) may be characterized by having one or more hydrogen atoms each independently substituted with any one selected from the group consisting of -F, -Cl, -Br, -I, (C₁-C₁₀) alkyl group, amino group (-NH₂), nitro group (-NO₂), -CF₃, -CF₂H, -CFH₂, -CCl₂, -CCl₂H, -CClH₂, - (CH₂)ₙCl, (C₁-C₁₀) alkoxy group, hydroxyl group (-OH), (C₁-C₁₀) alkoxyaryl group, and C₆-C₃₀ aryl group, but is not limited thereto.

In the present invention, the compound may be characterized by being any one of the compounds shown in Table 1, but is not limited thereto.

In the present invention, any benzene ring present in Chemical Formula (II-4) may be characterized by having one or more hydrogen atoms each independently substituted with halogen, C₁₋₁₀ alkyl, or C₁₋₁₀ haloalkyl, but is not limited thereto.

In the present invention, the compound may be characterized by being represented by the following Chemical Formula (III), but is not limited thereto:

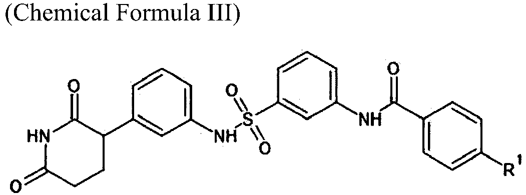

In Chemical Formula (III),
R¹ is halogen, C₁₋₁₀ alkyl, or C₁₋₁₀ haloalkyl.

In a specific embodiment, the compound may be characterized by being any one selected from the group consisting of the following chemical formulas:

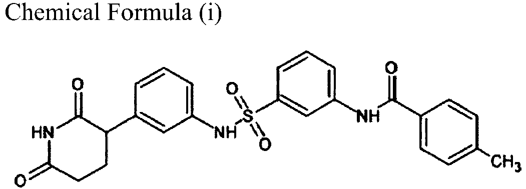

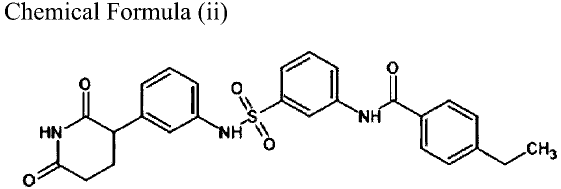

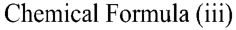

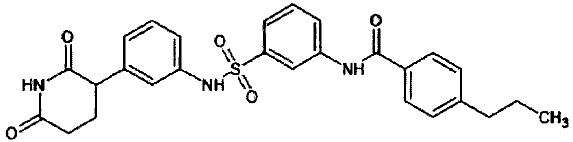

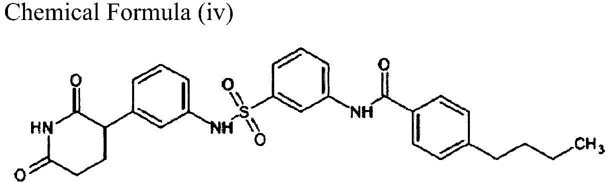

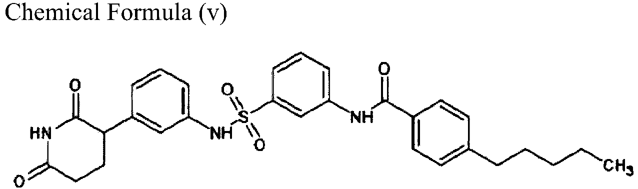

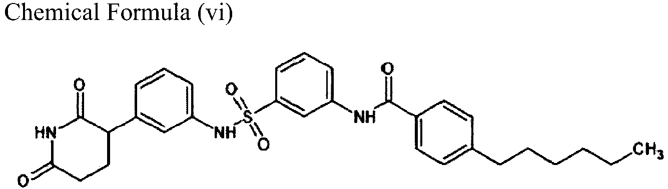

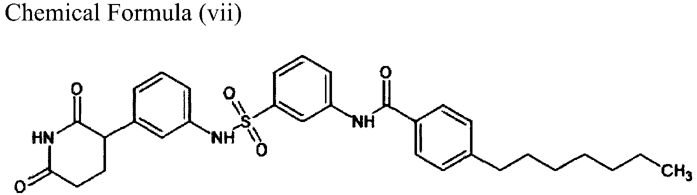

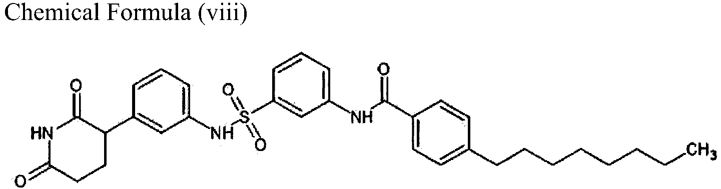

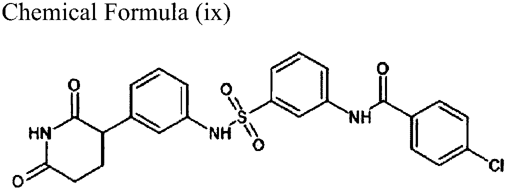

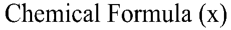

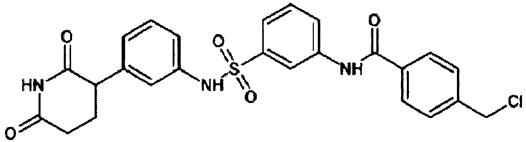

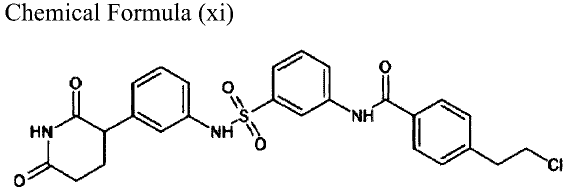

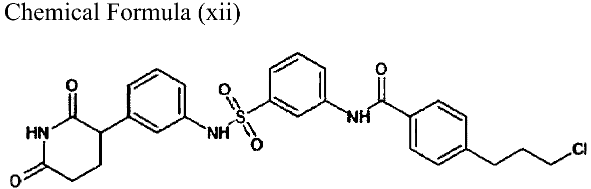

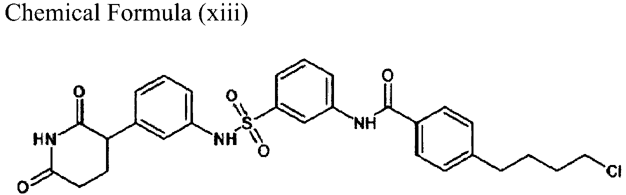

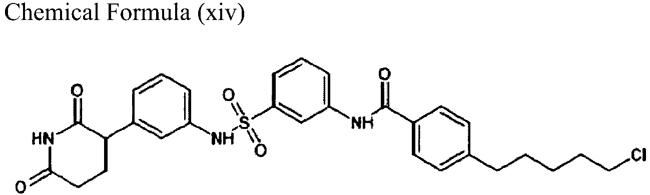

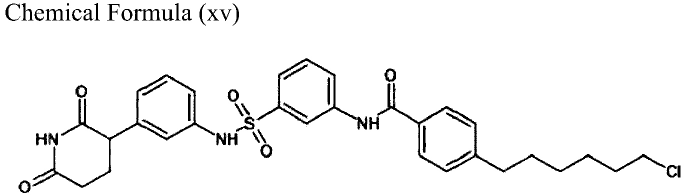

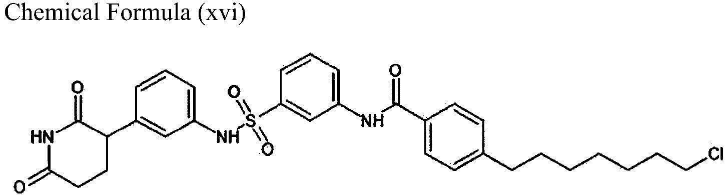

In the present invention, the compound, an optical isomer thereof, a racemic mixture thereof, a hydrate thereof, a solvate thereof, or a pharmaceutically acceptable salt thereof may be characterized by having binding affinity for cereblon.

In the present invention, the term "alkyl" group refers to a saturated, partially saturated, or unsaturated straight-chain or branched non-cyclic hydrocarbon having 1 to 10 carbon atoms, typically 1 to 8 carbon atoms, or, in some embodiments, 1 to 6, 1 to 4, or 2 to 6 carbon atoms. In some embodiments, the alkyl group is a saturated alkyl group. Representative saturated alkyl groups include -methyl, -ethyl, -n-propyl, -n-butyl, -n-pentyl, and -n-hexyl; while representative saturated branched alkyl groups include -isopropyl, -sec-butyl, -isobutyl, -tert-butyl, -isopentyl, -neopentyl, -tert-pentyl, -2-methylpentyl, -3-methylpentyl, -4-methylpentyl, and -2,3-dimethylbutyl. In some embodiments, the alkyl group is an unsaturated alkyl group, which is also referred to as an alkenyl or alkynyl group. The term "alkenyl" group refers to an alkyl group containing one or more carbon-carbon double bonds. The term "alkynyl" group refers to an alkyl group containing one or more carbon-carbon triple bonds. Examples of unsaturated alkyl groups include, but are not limited to, vinyl, allyl, -CH=CH(CH3), - CH=C(CH3)2, -C(CH3)=CH2, -C(CH3)=CH(CH3), -C(CH2CH3)=CH2, -C≡C-, and - CH2C≡. The alkyl group may be substituted or unsubstituted. When an alkyl group described in the present specification is referred to as being "substituted," it may be substituted with any substituent or substituents, including those found in the exemplary compounds and embodiments disclosed herein, as well as halogen; hydroxy; alkoxy; cycloalkyloxy, aryloxy, heterocyclyloxy, heterocycloalkyloxy, cycloalkylalkyloxy, aralkyloxy, heterocyclylalkyloxy, or heterocycloalkylalkyloxy; oxo ( = O); amino, alkylamino, cycloalkylamino, arylamino, heterocyclylamino, heterocycloalkylamino, cycloalkylalkylamino, aralkylamino, heterocyclylalkylamino, or heterocycloalkylalkylamino; imino; imido; amidino; guanidino; enamino; acylamino; sulfonylamino; urea or nitrourea; oxime; hydroxylamino; alkoxyamino; aralkoxyamino; hydrazino; hydrazido; hydrazono; azido; nitro; thio (-SH) or alkylthio; =S; sulfinyl; sulfonyl; aminosulfonyl; phosphonate; phosphinyl; acyl; formyl; carboxy; ester; carbamate; amido; cyano; isocyanato; isothiocyanato; cyanato; thiocyanato; or -B(OH)₂. In certain embodiments, when an alkyl group described in the present specification is referred to as being "substituted," it may be substituted with any substituent or substituents, including those found in the exemplary compounds and embodiments disclosed herein, as well as halogen (chloro, iodo, bromo, or fluoro); alkyl; hydroxyl; alkoxy; alkoxyalkyl; amino; alkylamino; carboxy; nitro; cyano; thiol; thioether; imine; imide; amidine; guanidine; enamine; aminocarbonyl; acylamino; phosphonate; phosphine; thiocarbonyl; sulfinyl; sulfone; sulfonamide; ketone; aldehyde; ester; urea; urethane; oxime; hydroxylamine; alkoxyamine; aralkoxyamine; N-oxide; hydrazine; hydrazide; hydrazone; azide; isocyanate; isothiocyanate; cyanate; thiocyanate; B(OH)2; or O(alkyl)aminocarbonyl.

The term "cycloalkyl" group refers to a saturated or partially saturated cyclic alkyl group having 3 to 10 carbon atoms, which may be a monocyclic ring or multiple fused or bridged rings that may be optionally substituted. In some embodiments, the cycloalkyl group has 3 to 8 ring members, whereas in other embodiments, the number of ring carbon atoms is in the range of 3 to 5, 3 to 6, or 3 to 7. In some embodiments, the cycloalkyl group is a saturated cycloalkyl group. Such saturated cycloalkyl groups include, for example, a monocyclic ring structure such as cyclopropyl, cyclobutyl, cyclopentyl, cyclohexyl, cycloheptyl, cyclooctyl, 1-methylcyclopropyl, 2-methylcyclopentyl, or 2-methylcyclooctyl, and polycyclic or bridged ring structures such as 1-bicyclo[1.1.1]pentyl, bicyclo[2.1.1]hexyl, bicyclo[2.2.1]heptyl, bicyclo[2.2.2]octyl, or adamantyl. In other embodiments, the cycloalkyl group is an unsaturated cycloalkyl group. Examples of unsaturated cycloalkyl groups include, in particular, cyclohexenyl, cyclopentenyl, cyclohexadienyl, butadienyl, pentadienyl, and hexadienyl. The cycloalkyl group may be substituted or unsubstituted. Such substituted cycloalkyl groups include, for example, cyclohexanol.

The term "aryl" group refers to an aromatic carbocyclic group having 6 to 14 carbon atoms, which may be a monocyclic ring (for example, phenyl) or multiple fused rings (for example, naphthyl or anthryl). In some embodiments, the aryl group contains 6 to 14 carbon atoms in the ring portion of the group, and in other embodiments, 6 to 12 or even 6 to 10 carbon atoms. Specific examples of aryl groups include phenyl, biphenyl, and naphthyl. Hydrogen atoms of the aryl group may be substituted or unsubstituted. The term "aryl group" also includes a group containing fused rings, such as fused aromatic-aliphatic ring systems (for example, indanyl or tetrahydronaphthyl). One or both termini of the aryl group may be bonded to another functional group.

The term "heterocyclyl" refers to a non-aromatic cycloalkyl in which 1 to 4 of the ring carbon atoms are independently replaced by heteroatoms selected from the group consisting of O, S, and N. In some embodiments, the heterocyclyl group includes 3 to 10 ring members, while in other such groups, the number of ring members is 3 to 5, 3 to 6, or 3 to 8. The heterocyclyl group may also be bonded to another group at any ring atom (that is, at any carbon atom or heteroatom of the heterocyclic ring). The heterocycloalkyl group may be substituted or unsubstituted. The heterocyclyl group includes unsaturated, partially saturated, and saturated ring systems such as imidazolyl, imidazolinyl, and imidazolidinyl (for example, imidazolidine-4-one or imidazolidine-2,4-dione) groups. The term "heterocyclyl" also includes fused ring species, including those having fused aromatic and non-aromatic groups, such as 1- and 2-aminotetralin, benzotriazolyl (for example, 1H-benzo[d][1,2,3]triazolyl), benzimidazolyl (for example, 1H-benzo[d]imidazolyl), 2,3-dihydrobenzo[1,4]dioxinyl, and benzo[1,3]dioxolyl. This term further includes bridged polycyclic ring systems containing heteroatoms, including, without limitation, quinuclidinyl. Representative examples of heterocyclyl groups include aziridinyl, azetidinyl, azepanyl, oxetanyl, pyrrolidinyl, imidazolidinyl (for example, imidazolidine-4-one or imidazolidine-2,4-dione), pyrazolidinyl, thiazolidinyl, tetrahydrothiophenyl, tetrahydrofuran-yl, dioxolyl, furanyl, thiophenyl, pyrrolyl, pyrrolinyl, imidazolyl, imidazolinyl, pyrazolyl, pyrazolinyl, triazolyl, tetrazolyl, oxazolyl, isoxazolyl, benzoisoxazolyl (for example, benzo[d]isoxazolyl), thiazolyl, thiazolinyl, isothiazolyl, thiadiazolyl, oxadiazolyl, piperidyl, piperazinyl (for example, piperazin-2-one), morpholinyl, thiomorpholinyl, tetrahydropyranyl (for example, tetrahydro-2H-pyranyl), tetrahydrothiopyranyl, oxathianyl, dioxyl, dithianyl, pyranyl, pyridyl, pyrimidyl, pyridazinyl, pyrazinyl, triazinyl, dihydropyridyl, dihydrodithiinyl, dihydrodithionyl, 1,4-dioxaspiro[4.5]decanyl, homopiperazinyl, quinuclidinyl, indolyl (for example, indolyl-2-one or isoindoline-1-one), indolinyl, isoindolyl, isoindolinyl, azaindolyl (pyrrolopyridyl or 1H-pyrrolo[2,3-b]pyridyl), indazolyl, indolizinyl, benzotriazolyl (for example, 1H-benzo[d][1,2,3]triazolyl), benzimidazolyl (for example, 1H-benzo[d]imidazolyl or 1H-benzo[d]imidazol-2(3H)-one), benzofuranyl, benzothiophenyl, benzothiazolyl, benzoxadiazolyl, benzoxazinyl, benzodithiinyl, benzoxathiinyl, benzothiazinyl, benzoxazolyl (that is, benzo[d]oxazolyl), benzothiazolyl, benzothiadiazolyl, benzo[1,3]dioxolyl, pyrazolopyridyl (for example, 1H-pyrazolo[3,4-b]pyridyl or 1H-pyrazolo[4,3-b]pyridyl), imidazopyridyl (for example, azabenzimidazolyl or 1H-imidazo[4,5-b]pyridyl), triazolopyridyl, isoxazolopyridyl, purinyl, xanthinyl, adeninyl, guaninyl, quinolinyl, isoquinolinyl (for example, 3,4-dihydroisoquinolin-1(2H)-one), quinolizinyl, quinoxalinyl, quinazolinyl, cinnolinyl, phthalazinyl, naphthyridinyl, pteridinyl, thianaphthylenyl, dihydrobenzothiazinyl, dihydrobenzofuranyl, dihydroindolyl, dihydrobenzodioxinyl, tetrahydroindolyl, tetrahydroindazolyl, tetrahydrobenzimidazolyl, tetrahydrobenzotriazolyl, tetrahydropyrrolopyridyl, tetrahydropyrazolopyridyl, tetrahydroimidazopyridyl, tetrahydrotriazolopyridyl, tetrahydropyrimidine-2(1H)-one, and tetrahydroquinolinyl, but are not limited thereto. Representative non-aromatic heterocyclyl groups do not include fused ring species containing fused aromatic groups. Examples of non-aromatic heterocyclyl groups include aziridinyl, azetidinyl, azepanyl, pyrrolidinyl, imidazolidinyl (for example, imidazolidine-4-one or imidazolidine-2,4-dione), pyrazolidinyl, thiazolidinyl, tetrahydrothiophenyl, tetrahydrofuranyl, piperidyl, piperazinyl (for example, piperazin-2-one), morpholinyl, thiomorpholinyl, tetrahydropyranyl (for example, tetrahydro-2H-pyranyl), tetrahydrothiopyranyl, oxathianyl, dithianyl, 1,4-dioxaspiro[4.5]decanyl, homopiperazinyl, quinuclidinyl, or tetrahydropyrimidine-2(1H)-one. Representative substituted heterocyclyl groups may be monosubstituted or polysubstituted, and include, without limitation, 2-, 3-, 4-, 5-, or 6-substituted, or disubstituted pyridyl or morpholinyl groups having various substituents such as those listed below.

The "cycloalkylalkyl" group has the formula -alkyl-cycloalkyl, in which the alkyl and cycloalkyl are as defined above. A substituted cycloalkylalkyl group may be substituted at the alkyl portion, the cycloalkyl portion, or both the alkyl and cycloalkyl portions. Representative examples of cycloalkylalkyl groups include, but are not limited to, cyclopropylmethyl, cyclobutylmethyl, cyclopentylmethyl, cyclohexylmethyl, cyclopropylethyl, cyclobutylethyl, cyclopentylethyl, cyclohexylethyl, cyclopentylpropyl, and cyclohexylpropyl.

The "aralkyl" group has the formula -alkyl-aryl, in which the alkyl and aryl are as defined above. A substituted aralkyl group may be substituted on the alkyl portion, the aryl portion, or both the alkyl and aryl portions. Representative aralkyl groups include benzyl and phenethyl groups, and aralkyl groups in which the aryl group is fused to a cycloalkyl group, such as indan-4-ylethyl, but are not limited thereto.

The "heterocyclylalkyl" group has the formula -alkyl-heterocyclyl, in which the alkyl and heterocyclyl are as defined above. The "heterocycloalkylalkyl" group has the formula - alkyl-heterocycloalkyl, in which the alkyl and heterocycloalkyl are as defined above. A substituted heterocyclylalkyl group may be substituted on the alkyl portion, the heterocyclyl portion, or both the alkyl and heterocyclyl portions. Representative heterocyclylalkyl groups include morpholine-4-ylethyl, morpholine-4-ylpropyl, furan-2-ylmethyl, furan-3-ylmethyl, pyridin-3-ylmethyl, tetrahydrofuran-2-ylethyl, and indol-2-ylpropyl, but are not limited thereto.

The term "halogen (X)" refers to fluorine (F), chlorine (Cl), bromine (Br), or iodine (I).

The term "haloalkyl" refers to an alkyl group in which one or more hydrogen atoms are substituted with halogen atoms, and may be, for example, fluoroalkyl, chloroalkyl, bromoalkyl, or iodoalkyl. In some embodiments, the haloalkyl may be a saturated haloalkyl (CₙH₂ₙX), and preferably a haloalkyl having 1 to 10 carbon atoms, such as CH₂X, C₂H₄X, C₃H₆X, C₄H₈X, C₅H₁₀X, C₆H₁₂X, C₇H₁₄X, C₈H₁₆X, C₉H₁₈X, C₁₀H₂₀X.

The "hydroxyalkyl" group refers to an alkyl group in which one or more hydrogen atoms are substituted with one or more hydroxyl groups. It may also be represented as an alcohol, and one or both termini may be bonded to another functional group.

The "alkoxy" group has the formula -O-(alkyl), in which the alkyl is as defined above. One or both termini of the alkoxy group may be bonded to another functional group.

The "alkoxyalkyl" group has the formula -(alkyl)-O-(alkyl), in which the alkyl is as defined above.

The "amino" or "amine" group has the formula -NH₂ or -NH-, and one or both termini may be bonded to another functional group.

In one embodiment, the "amino" group is represented by the formula -NH₂, -NH-, or - NH-alkyl, in which each alkyl is independently as defined above.

The "sulfonyl" group has the formula -SO₂-, and one or both termini may be bonded to another functional group.

The terms "cycloalkylamino," "arylamino," "heterocyclylamino," and "heterocycloalkylamino" reflect the foregoing description of "alkylamino," except that the term "alkyl" is respectively replaced with "cycloalkyl," "aryl," "heterocyclyl," and "heterocycloalkyl."

The "carboxy (carboxyl)" group or carboxylic acid has the formula -C(O)OH or - C(O)O-, and one or both termini may be bonded to another functional group.

The "hydroxy" group has the formula -OH.

The "aldehyde" group has the formula -CHO.

As used herein, unless otherwise specified, the "acyl" group has the formula -C(O)(R) or -C(O)H, wherein R is as defined above. The "formyl" group has the formula -C(O)H.

As used herein, unless otherwise specified, the "carbonyl" group has the formula -C(O)-, and one or both termini may be bonded to another functional group.

As used herein, unless otherwise specified, the "amido" group has the formula -C(O)-NH₂, -C(O)-NH(R), -C(O)-N(R)₂, -NH-C(O)H, -NH-C(O)-(R), -N(R)-C(O)H, or -N(R)-C(O)-(R), wherein each R is independently as defined above.

In one embodiment, the "amido" group is an "aminocarbonyl" group having the formula -C(O)-NH₂, -C(O)-NH(R), or -C(O)-N(R)₂, wherein each R is independently as defined above.

In one embodiment, the "amido" group is an "acylamino" group having the formula - NH-C(O)H, -NH-C(O)-(R), -N(R)-C(O)H, or -N(R)-C(O)-(R), wherein each R is independently as defined above.

The "sulfonylamino" group has the formula -NHSO2(R) or -N(alkyl)SO2(R), wherein each alkyl and R are as defined above.

The "urea" group has the formula -N(alkyl)C(O)N(R)2, -N(alkyl)C(O)NH(R), - N(alkyl)C(O)NH2, -NHC(O)N(R)2, -NHC(O)NH(R), or -NH(CO)NH2, wherein each alkyl and R are independently as defined above.

The "alkylamine" group refers to a group in which an alkyl group and an amine group are bonded together, and one or both termini may be bonded to another functional group.

The "aminocarbonylalkyl" group refers to a group in which an amino group, a carbonyl group, and an alkyl group are sequentially bonded, and one or both termini may be bonded to another functional group.

The "aminocarbonylaryl" group refers to a group in which an amino group, a carbonyl group, and an aryl group are sequentially bonded, and one or both termini may be bonded to another functional group.

The "aminosulfonylalkyl" group refers to a group in which an amino group, a sulfonyl group, and an alkyl group are sequentially bonded, and one or both termini may be bonded to another functional group.

The "aminocarbonylhydroxyalkyl" group refers to a group in which an amino group, a carbonyl group, and a hydroxyalkyl group are sequentially bonded, and one or both termini may be bonded to another functional group.

The "aminosulfonylaryl" group refers to a group in which an amino group, a sulfonyl group, and an aryl group are sequentially bonded, and one or both termini may be bonded to another functional group.

The "aminosulfonylalkyl" group refers to a group in which an amino group, a sulfonyl group, and an alkyl group are sequentially bonded, and one or both termini may be bonded to another functional group.

The "aminocarbonylaminoaryl" group refers to a group in which an amino group, a carbonyl group, an amino group, and an aryl group are sequentially bonded, and one or both termini may be bonded to another functional group.

The "alkylaminocarbonylalkoxy" group refers to a group in which an alkyl group, an amino group, a carbonyl group, and an alkoxy group are sequentially bonded, and one or both termini may be bonded to another functional group.

The "aminosulfonylaminocarbonylaminoaryl" group refers to a group in which an amino group, a sulfonyl group, an amino group, a carbonyl group, an amino group, and an aryl group are sequentially bonded, and one or both termini may be bonded to another functional group.

The "aminoarylalkylcarboxylalkyl" group refers to a group in which an amino group, an aryl group, an alkyl group, a carboxyl group, and an alkyl group are sequentially bonded, and one or both termini may be bonded to another functional group.

The "aminoarylalkylcarbonylaminoaryl" group refers to a group in which an amino group, an aryl group, an alkyl group, a carbonyl group, an amino group, and an aryl group are sequentially bonded, and one or both termini may be bonded to another functional group.

The "aminoarylalkylaminocarboxylalkyl" group refers to a group in which an amino group, an aryl group, an alkyl group, an amino group, a carboxyl group, and an alkyl group are sequentially bonded, and one or both termini may be bonded to another functional group.

The "aminoarylalkylaminocarbonylaminoaryl" group refers to a group in which an amino group, an aryl group, an alkyl group, an amino group, a carbonyl group, an amino group, and an aryl group are sequentially bonded, and one or both termini may be bonded to another functional group.

The "aminosulfonylarylaminocarbonylarylalkoxy" group refers to a group in which an amino group, a sulfonyl group, an aryl group, an amino group, a carbonyl group, an aryl group, and an alkoxy group are sequentially bonded, and one or both termini may be bonded to another functional group.

The "aminoarylalkylcarbonylaminoarylalkoxy" group refers to a group in which an amino group, an aryl group, an alkyl group, a carbonyl group, an amino group, an aryl group, and an alkoxy group are sequentially bonded, and one or both termini may be bonded to another functional group.

The "aminoarylalkylcarbonylaminoalkylcarboxylalkyl" group refers to a group in which an amino group, an aryl group, an alkyl group, a carbonyl group, an amino group, an alkyl group, a carboxyl group, and an alkyl group are sequentially bonded, and one or both termini may be bonded to another functional group.

The "aminoarylalkylcarbonylaminoalkylcarboxylic acid" group refers to a group in which an amino group, an aryl group, an alkyl group, a carbonyl group, an amino group, an alkyl group, and a carboxylic acid are sequentially bonded, and one or both termini may be bonded to another functional group.

The "aminoarylaminocarbonylalkylcarbonylaminoaryl" group refers to a group in which an amino group, an aryl group, an amino group, a carbonyl group, an alkyl group, a carbonyl group, an amino group, and an aryl group are sequentially bonded, and one or both termini may be bonded to another functional group.

The "aminoarylaminocarbonylaryl" group refers to a group in which an amino group, an aryl group, an amino group, a carbonyl group, and an aryl group are sequentially bonded, and one or both termini may be bonded to another functional group.

The "aminosulfonylarylaminocarbonylaryl" group refers to a group in which an amino group, a sulfonyl group, an aryl group, an amino group, a carbonyl group, and an aryl group are sequentially bonded, and one or both termini may be bonded to another functional group.

Except for alkyl groups, when a group described herein is referred to as being "substituted," it may be substituted with any suitable substituent or substituents. Exemplary substituents include, but are not limited to, those found in the exemplary compounds and embodiments disclosed herein, as well as halogen (chloro, iodo, bromo, or fluoro); alkyl; hydroxyl; alkoxy; alkoxyalkyl; amino; alkylamino; carboxy; nitro; cyano; thiol; thioether; imine; imide; amidine; guanidine; enamine; aminocarbonyl; acylamino; phosphonate; phosphine; thiocarbonyl; sulfinyl; sulfonyl; sulfonamide; ketone; aldehyde; ester; urea; urethane; oxime; hydroxylamine; alkoxyamine; aralkoxyamine; N-oxide; hydrazine; hydrazide; hydrazone; azide; isocyanate; isothiocyanate; cyanate; thiocyanate; oxygen (=O); B(OH)₂; O(alkyl)aminocarbonyl; cycloalkyl (which may be monocyclic or polycyclic, either fused or unfused, for example, cyclopropyl, cyclobutyl, cyclopentyl, or cyclohexyl); heterocyclyl (which may be monocyclic or polycyclic, either fused or unfused, for example, pyrrolidinyl, piperidyl, piperazinyl, morpholinyl, or thiazinyl); monocyclic or polycyclic, either fused or unfused, aryl (for example, phenyl or naphthyl); aryloxy; aralkyloxy; heterocyclyloxy; and heterocyclylalkoxy.

The compound according to the present invention may be prepared and used in the form of a prodrug, a hydrate, a solvate, or a pharmaceutically acceptable salt in order to enhance bioavailability or increase solubility. Accordingly, such prodrugs, hydrates, solvates, and pharmaceutically acceptable salts are also within the scope of the present invention. In addition, since the compound has a chiral carbon, stereoisomers thereof may exist, and such stereoisomers are also encompassed within the scope of the present invention.

The term "prodrug" refers to a substance that is converted into a parent drug in vivo. Prodrugs are often used because, in some cases, they are easier to administer than the parent drug. For example, while the parent drug may not be orally bioavailable, the prodrug may exhibit bioavailability through oral administration. A prodrug may also have improved solubility in a pharmaceutical composition compared to the parent drug. For instance, a prodrug may be a hydrolyzable ester of the compound according to the present invention, or a pharmaceutically acceptable salt thereof. Another example of a prodrug is a short peptide (polyamino acid) bound to an acidic group that is metabolically cleaved to expose the active site.

The term "hydrate" refers to a compound of the present invention or a salt thereof that includes a stoichiometric or non-stoichiometric amount of water bound through non-covalent intermolecular forces.

The term "solvate" refers to a compound of the present invention or a salt thereof that includes a stoichiometric or non-stoichiometric amount of a solvent bound through non-covalent intermolecular forces. Preferred solvents include volatile, non-toxic, and/or pharmaceutically acceptable solvents suitable for administration to humans.

The term "isomer" refers to a compound of the present invention or a salt thereof that has the same chemical or molecular formula but differs in structural or stereochemical configuration. Such isomers include structural isomers such as tautomers, and stereoisomers such as R- or S-isomers having an asymmetric carbon center and geometric isomers such as trans or cis forms. All such isomers and mixtures thereof are also encompassed within the scope of the present invention.

The term "pharmaceutically acceptable salt" refers to a salt form of the compound that does not cause significant irritation to an organism to which it is administered and does not impair the biological activity or physicochemical properties of the compound. The pharmaceutical salts include acid addition salts formed by inorganic acids containing pharmaceutically acceptable anions, such as hydrochloric acid, sulfuric acid, nitric acid, phosphoric acid, hydrobromic acid, and hydroiodic acid, as well as organic carboxylic acids such as tartaric acid, formic acid, citric acid, acetic acid, trichloroacetic acid, trifluoroacetic acid, gluconic acid, benzoic acid, lactic acid, fumaric acid, maleic acid, and salicylic acid, and sulfonic acids such as methanesulfonic acid, ethanesulfonic acid, benzenesulfonic acid, and p-toluenesulfonic acid. For example, pharmaceutically acceptable carboxylate salts include metal or alkaline earth metal salts such as lithium, sodium, potassium, calcium, and magnesium salts; amino acid salts such as lysine, arginine, and guanidine salts; and organic salts such as dicyclohexylamine, N-methyl-D-glucamine, tris(hydroxymethyl)methylamine, diethanolamine, choline, and triethylamine salts. The compound according to the present invention can be converted into its salt form by conventional methods.

In another aspect, the present invention relates to a pharmaceutical composition for preventing or treating benign tumors or cancers, comprising the compound, an optical isomer thereof, a racemic mixture thereof, a hydrate thereof, a solvate thereof, or a pharmaceutically acceptable salt thereof as an active ingredient.

In the present invention, the cancer may be a hematologic cancer or a solid cancer.

In the present invention, the hematologic cancer may be selected from the group consisting of acute leukemia, chronic leukemia, multiple myeloma, Hodgkin's lymphoma, and non-Hodgkin's lymphoma, but is not limited thereto.

In the present invention, the solid cancer may be selected from the group consisting of melanoma, myosarcoma, multiple myeloma, head and neck cancer, nasopharyngeal cancer, esophageal cancer, gastroesophageal junction cancer, esophageal adenocarcinoma, gastric cancer, bladder cancer, colorectal cancer, colon cancer, rectal cancer, small intestinal cancer, anal cancer, liver cancer, gallbladder cancer, cholangiocarcinoma, biliary tract cancer, pancreatic cancer, thyroid cancer, parathyroid cancer, lung cancer, breast cancer, ovarian cancer, fallopian tube cancer, uterine cancer, vaginal cancer, vulvar cancer, penile cancer, renal cancer, adrenal cancer, urothelial carcinoma, prostate cancer, testicular tumor, bone and soft tissue sarcoma, skin cancer, glioma, brain tumor, spinal tumor, Kaposi's sarcoma, squamous cell carcinoma, pleural mesothelioma, and primary peritoneal cancer, but is not limited thereto.

In the present invention, the term "pharmaceutical composition" or "pharmaceutical formulation" refers to a mixture comprising a pharmaceutically acceptable excipient such as a diluent or carrier, which renders the novel compound of the present invention particularly suitable for in vivo or in vitro diagnostic or therapeutic use. In some embodiments, the pharmaceutical composition comprising the composition of the present invention may be administered to a subject in a therapeutically effective amount as needed. In some embodiments, the composition of the present invention can be administered to a human.

The term "effective amount" or "therapeutically effective amount," as used in the present invention, refers to an amount of a compound or composition (for example, the compound or composition of the present invention) sufficient to achieve a beneficial or desired result. The effective amount may be administered in one or more doses, applications, or dosages, and is not intended to be limited to any particular formulation or route of administration.

The pharmaceutical composition provided in the present invention is principally directed to a pharmaceutical composition for administration to humans; however, those skilled in the art will understand that such compositions are generally suitable for administration to all types of animals. That is, the pharmaceutical composition according to the present invention may also be administered to other mammals requiring veterinary treatment, such as pets (e.g., dogs, cats, and the like), livestock (e.g., cattle, sheep, pigs, horses, and the like), and laboratory animals (e.g., rats, mice, guinea pigs, and the like). A skilled veterinary pharmacologist, well aware of the variations of pharmaceutical compositions required for administration to different animals, can readily design and/or perform such modifications through routine experimentation if necessary.

The pharmaceutical composition described in the present invention may be prepared by any method known in the field of pharmacology or by any method developed hereafter. In general, such methods for formulation comprise the step of associating the active ingredient with an excipient and/or one or more other auxiliary ingredients, followed, if necessary or desired, by shaping and/or packaging the resulting product into desired single- or multiple-dose units.

The pharmaceutical composition of the present invention may be manufactured, packaged, and/or sold, either in single unit doses or in multiple single unit doses, or in an unpackaged form. As used herein, the term "unit dose" refers to an individual quantity of the pharmaceutical composition containing a predetermined amount of the active ingredient. The amount of the active ingredient generally corresponds to the dose of the active ingredient to be administered to a subject and/or a convenient fraction thereof, for example, one-half or one-third of the dose.

The relative amounts of the active ingredient, pharmaceutically acceptable excipient, and/or any additional components in the pharmaceutical composition of the present invention may vary depending on the identity, size, and/or disorder of the subject to be treated, as well as on the route of administration of the composition. For example, the composition may contain from 0.001% to 100% (w/w) of the active ingredient.

As used in the present invention, the pharmaceutically acceptable excipient includes any solvent, dispersing medium, diluent, or other liquid vehicle; dispersion or suspension aid; surfactant; isotonic agent; thickener or emulsifier; preservative; solid binder; lubricant; or the like, suitable for the intended dosage form. Remington's The Science and Practice of Pharmacy, 21st Edition, A. R. Gennaro (Lippincott Williams & Wilkins, Baltimore, MD, 2006) discloses various excipients used in the preparation of pharmaceutical compositions and known techniques for their manufacture. The use of any conventional carrier medium is considered to fall within the scope of the present invention, except for those that produce an undesired biological effect or otherwise interact adversely with any other component of the pharmaceutical composition so as to be incompatible with the compound or its derivative. The pharmaceutically acceptable excipient is at least 95%, 96%, 97%, 98%, 99%, or 100% pure.

The excipients are approved for human and veterinary use. In some embodiments, the excipients are approved by the U.S. Food and Drug Administration (FDA). In some embodiments, the excipients are of pharmaceutical grade. In some embodiments, the excipients meet the standards of the United States Pharmacopeia (USP), the European Pharmacopeia (EP), the British Pharmacopeia (BP), and/or the International Pharmacopeia (IP).

In some embodiments, the excipients are approved for human and veterinary use. In some embodiments, the excipients are approved by the U.S. Food and Drug Administration (FDA). In some embodiments, the excipients are of pharmaceutical grade. In some embodiments, the excipients meet the standards of the United States Pharmacopeia (USP), the European Pharmacopeia (EP), the British Pharmacopeia (BP), and/or the International Pharmacopeia (IP).

The pharmaceutically acceptable excipients used in the preparation of the pharmaceutical composition include, but are not limited to, inert diluents, dispersants and/or granulating agents, surfactants and/or emulsifiers, disintegrants, binders, preservatives, buffering agents, lubricants, and/or oils.

Such excipients may optionally be included in the formulations of the present invention. Excipients such as cocoa butter and suppository wax, coloring agents, coating agents, sweeteners, flavoring agents, and perfuming agents may be present in the composition at the discretion of the formulator.

Exemplary diluents include, but are not limited to, calcium carbonate, sodium carbonate, calcium phosphate, dicalcium phosphate, calcium sulfate, calcium hydrogen phosphate, sodium phosphate, lactose, sucrose, cellulose, microcrystalline cellulose, kaolin, mannitol, sorbitol, inositol, sodium chloride, dried starch, corn starch, powdered sugar, and combinations thereof.

Exemplary granulating agents and/or dispersants include, but are not limited to, potato starch, corn starch, tapioca starch, sodium starch glycolate, clay, alginic acid, guar gum, citrus pulp, agar, bentonite, cellulose and wood products, natural sponge, cation-exchange resins, calcium carbonate, silicates, sodium carbonate, cross-linked poly(vinylpyrrolidone) (crospovidone), sodium carboxymethyl starch (sodium starch glycolate), carboxymethyl cellulose, cross-linked sodium carboxymethyl cellulose (croscarmellose), methylcellulose, pregelatinized starch (starch 1500), microcrystalline starch, water-insoluble starch, calcium carboxymethyl cellulose, magnesium aluminum silicate (Veegum), sodium lauryl sulfate, quaternary ammonium compounds, and combinations thereof.

Exemplary surfactants and/or emulsifiers include, but are not limited to, natural emulsifiers (e.g., acacia, agar, alginic acid, sodium alginate, tragacanth, chondrux, cholesterol, xanthan, pectin, gelatin, egg yolk, casein, lanolin, cholesterol, wax, and lecithin); colloidal clays (e.g., bentonite [aluminum silicate] and Veegum [magnesium aluminum silicate]); long-chain amino acid derivatives; high molecular weight alcohols (e.g., stearyl alcohol, cetyl alcohol, oleyl alcohol, triacetin monostearate, ethylene glycol distearate, glyceryl monostearate, propylene glycol monostearate, and polyvinyl alcohol); carbomers (e.g., carboxypolymethylene, polyacrylic acid, acrylic acid polymer, and carboxyvinyl polymer); carrageenan; cellulose derivatives (e.g., sodium carboxymethyl cellulose, powdered cellulose, hydroxymethyl cellulose, hydroxypropyl cellulose, hydroxypropyl methylcellulose, and methylcellulose); sorbitan fatty acid esters (e.g., polyoxyethylene sorbitan monolaurate [Tween 20], polyoxyethylene sorbitan [Tween 60], polyoxyethylene sorbitan monooleate [Tween 80], sorbitan monopalmitate [Span 40], sorbitan monostearate [Span 60], sorbitan tristearate [Span 65], glyceryl monooleate, and sorbitan monooleate [Span 80]); polyoxyethylene esters (e.g., polyoxyethylene monostearate [Myrj 45], polyoxyethylene hydrogenated castor oil, polyethoxylated castor oil, polyoxymethylene stearate, and Solutol); sucrose fatty acid esters; polyethylene glycol fatty acid esters (e.g., Cremophor); polyoxyethylene ethers (e.g., polyoxyethylene lauryl ether [Brij 30]); poly(vinylpyrrolidone); diethylene glycol monolaurate; triethanolamine oleate; sodium oleate; potassium oleate; ethyl oleate; oleic acid; ethyl laurate; sodium lauryl sulfate; Pluronic F68; Poloxamer 188; cetrimonium bromide; cetylpyridinium chloride; benzalkonium chloride; docusate sodium; and/or combinations thereof.

Exemplary binders include, but are not limited to, starches (e.g., corn starch and starch paste); gelatin; sugars (e.g., sucrose, glucose, dextrose, dextrin, molasses, lactose, lactitol, and mannitol); natural and synthetic gums (e.g., acacia, sodium alginate, Irish moss extract, panwar gum, ghatti gum, mucilage of isapol husks, carboxymethyl cellulose, methylcellulose, ethylcellulose, hydroxyethyl cellulose, hydroxypropyl cellulose, hydroxypropyl methylcellulose, microcrystalline cellulose, cellulose acetate, poly(vinylpyrrolidone), magnesium aluminum silicate [Veegum], and larch arabogalactan); alginates; polyethylene oxide; polyethylene glycol; inorganic calcium salts; silica; polymethacrylates; waxes; water; alcohols; and combinations thereof.

Exemplary preservatives may include antioxidants, chelating agents, antimicrobial preservatives, antifungal preservatives, alcohol-based preservatives, acidic preservatives, and other types of preservatives. Exemplary antioxidants include, but are not limited to, α-tocopherol, ascorbic acid, ascorbyl palmitate, butylated hydroxyanisole (BHA), butylated hydroxytoluene (BHT), monothioglycerol, potassium metabisulfite, propionic acid, propyl gallate, sodium ascorbate, sodium bisulfite, sodium metabisulfite, and sodium sulfite. Exemplary chelating agents include ethylenediaminetetraacetic acid (EDTA), citric acid monohydrate, disodium edetate, dipotassium edetate, edetic acid, fumaric acid, malic acid, phosphoric acid, sodium edetate, tartaric acid, and trisodium edetate. Exemplary antimicrobial preservatives include, but are not limited to, benzalkonium chloride, benzethonium chloride, benzyl alcohol, bronopol, cetrimide, cetylpyridinium chloride, chlorhexidine, chlorobutanol, chlorocresol, chloroxylenol, cresol, ethyl alcohol, glycerin, hexetidine, imidurea, phenol, phenoxyethanol, phenylethyl alcohol, phenylmercuric nitrate, propylene glycol, and thimerosal. Exemplary antifungal preservatives include, but are not limited to, butyl paraben, methyl paraben, ethyl paraben, propyl paraben, benzoic acid, hydroxybenzoic acid, potassium benzoate, potassium sorbate, sodium benzoate, sodium propionate, and sorbic acid. Exemplary alcohol-based preservatives include, but are not limited to, ethanol, polyethylene glycol, phenol, phenolic compounds, bisphenols, chlorobutanol, hydroxybenzoates, and phenylethyl alcohol. Exemplary acidic preservatives include, but are not limited to, vitamins A, C, and E, β-carotene, citric acid, acetic acid, dehydroacetic acid, ascorbic acid, sorbic acid, and phytic acid. Other preservatives include, but are not limited to, tocopherol, tocopheryl acetate, deteroxime mesilate, cetrimide, butylated hydroxyanisole (BHA), butylated hydroxytoluene (BHT), ethylenediamine, sodium lauryl sulfate (SLS), sodium lauryl ether sulfate (SLES), sodium bisulfite, sodium metabisulfite, potassium sulfite, potassium metabisulfite, Glydant Plus, Phenonip, methylparaben, Germall 115, Germaben II, Neolone, Kathon, and Euxyl. In certain embodiments, the preservative is an antioxidant. In another embodiment, the preservative is a chelating agent.

Exemplary buffering agents may include, but are not limited to, citrate buffer solution, acetate buffer solution, phosphate buffer solution, ammonium chloride, calcium carbonate, calcium chloride, calcium citrate, calcium glubionate, calcium gluseptate, calcium gluconate, D-gluconic acid, calcium glycerophosphate, calcium lactate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, magnesium hydroxide, aluminum hydroxide, alginic acid, pyrogen-free water, isotonic saline, Ringer's solution, ethyl alcohol, and combinations thereof.

Exemplary lubricants may include magnesium stearate, calcium stearate, stearic acid, silica, talc, malt, glyceryl behenate, hydrogenated vegetable oil, polyethylene glycol, sodium benzoate, sodium acetate, sodium chloride, leucine, magnesium lauryl sulfate, sodium lauryl sulfate, and combinations thereof, but are not limited thereto.

Exemplary oils may include almond, apricot kernel, avocado, babassu, bergamot, black currant seed, borage, cade, chamomile, canola, caraway, carnauba, castor, cinnamon, cocoa butter, coconut, cod liver, coffee, corn, cottonseed, emu, eucalyptus, evening primrose, fish, flaxseed, geraniol, pumpkin, grape seed, hazelnut, hyssop, isopropyl myristate, jojoba, kukui nut, lavandin, lavender, lemon, litsea cubeba, macadamia nut, mallow, mango seed, meadowfoam seed, mink, nutmeg, olive, orange-colored rapeseed, palm, palm kernel, peach kernel, peanut, poppy seed, pumpkin seed, rapeseed, rice bran, rosemary, safflower, sandalwood, sasanqua, savory, sea buckthorn, sesame, shea butter, silicone, soybean, sunflower, tea tree, thistle, tsubaki, vetiver, walnut, and wheat germ oils, but are not limited thereto. Exemplary oils include butyl stearate, caprylic triglyceride, capric triglyceride, cyclomethicone, diethyl sebacate, dimethicone 360, isopropyl myristate, mineral oil, octyldodecanol, oleyl alcohol, silicone oil, and combinations thereof, but are not limited thereto.

Liquid dosage forms for oral and parenteral administration include, but are not limited to, pharmaceutically acceptable emulsions, microemulsions, solutions, suspensions, syrups, and elixirs. In addition to the active ingredient, the liquid dosage forms may contain inert diluents commonly used in the art, such as, for example, water or other solvents, solubilizing agents, and emulsifiers, including ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, peanut, corn, germ, olive, castor, and sesame oils), glycerol, tetrahydrofurfuryl alcohol, polyethylene glycol and fatty acid esters of sorbitan, and mixtures thereof. In addition to the inert diluents, oral compositions may include adjuvants such as wetting agents, emulsifying and suspending agents, sweeteners, flavoring agents, and perfuming agents. In certain embodiments for parenteral administration, the novel compound of the present invention is mixed with solubilizing agents such as Cremophor, alcohols, oils, modified oils, glycols, polysorbates, cyclodextrins, polymers, and combinations thereof.

Injectable formulations, for example, sterile injectable aqueous or oily suspensions, may be formulated according to techniques known in the art using dispersing or wetting agents and suspending agents. The sterile injectable formulations may also be sterile injectable solutions, suspensions, or emulsions in non-toxic, parenterally acceptable diluents or solvents, for example, in solutions of 1,3-butanediol. Among the acceptable vehicles and solvents that may be employed are water, Ringer's solution, U.S.P., and isotonic sodium chloride solution. In addition, sterile fixed oils are conventionally employed as solvents or suspending media. For this purpose, any nonirritating fixed oil, including synthetic mono- or diglycerides, may be used. Furthermore, fatty acids such as oleic acid are used in the preparation of injectables.

Injectable formulations may be sterilized, for example, by filtration through a bacteria-retaining filter, or by incorporating a sterilizing agent in the form of a sterile solid composition that can be dissolved or dispersed in sterile water or another sterile injectable medium prior to use.

To prolong the effect of the drug, it is often desirable to slow the absorption of the drug from subcutaneous or intramuscular injection. This can be accomplished by using a liquid suspension of crystalline or amorphous material having low solubility. The rate of absorption of the drug then depends ultimately on the rate of dissolution, which in turn may be controlled by the crystal size and crystalline form. Alternatively, delayed absorption of a parenterally administered drug can be achieved by dissolving or suspending the drug in an oily vehicle.

Solid dosage forms for oral administration include capsules, tablets, pills, powders, and granules. In such solid dosage forms, the active ingredient is mixed with one or more inert pharmaceutically acceptable excipients or carriers, such as sodium citrate or dicalcium phosphate, and/or with: (a) fillers or extenders, for example, starch, lactose, sucrose, glucose, mannitol, and silica; (b) binders, for example, carboxymethylcellulose, alginate, gelatin, polyvinylpyrrolidone, sucrose, and acacia; (c) humectants, for example, glycerol; (d) disintegrants, for example, agar, calcium carbonate, potato or tapioca starch, alginic acid, certain silicates, and sodium carbonate; (e) dissolution retarders, for example, paraffin; (f) absorption accelerators, for example, quaternary ammonium compounds; (g) wetting agents, for example, cetyl alcohol and glycerol monostearate; (h) absorbents, for example, kaolin and bentonite clay; and (i) lubricants, for example, talc, calcium stearate, magnesium stearate, solid polyethylene glycol, sodium lauryl sulfate, and mixtures thereof.

In the case of capsules, tablets, and pills, the dosage forms may include a buffering agent. Similar types of solid compositions may also be employed as filling materials in soft and hard-filled gelatin capsules, using excipients such as lactose or milk sugar, as well as high-molecular-weight polyethylene glycols. The solid dosage forms such as tablets, sugar-coated tablets, capsules, pills, and granules may be manufactured with coatings and shells, such as enteric coatings or other coatings well known in the field of pharmaceutical formulation. These dosage forms may optionally include an opacifying agent and may be compositions that release the active ingredient alone or preferentially in a particular part of the intestinal tract, optionally in a delayed manner. Examples of coating compositions that can be used include polymeric materials and waxes. Similar types of solid compositions may also be employed as filling materials in soft and hard-filled gelatin capsules, using excipients such as lactose or milk sugar, as well as high-molecular-weight polyethylene glycols.

The active ingredient may be in a microencapsulated form together with one or more of the excipients described above. Solid dosage forms such as tablets, sugar-coated tablets, capsules, pills, and granules may be manufactured with coatings and shells, such as enteric coatings, controlled-release coatings, and other coatings well known in the field of pharmaceutical formulation. In these solid dosage forms, the active ingredient may be mixed with one or more inert diluents, for example, sucrose, lactose, or starch. Such dosage forms may also include, in addition to the non-combustible diluents commonly used, other materials such as tablet lubricants and additional tableting aids, for example, magnesium stearate and microcrystalline cellulose. In the case of capsules, tablets, and pills, the dosage forms may include a buffering agent. These dosage forms may optionally include an opacifying agent and may be compositions that release the active ingredient alone or preferentially in a particular part of the intestinal tract, optionally in a delayed manner. Examples of coating compositions that can be used include polymeric materials and waxes.

The dosage forms for topical and/or transdermal administration of the novel compound of the present invention, or a pharmaceutical composition containing the same, may include ointments, pastes, creams, lotions, gels, powders, solutions, sprays, inhalants, and/or patches. In general, the active ingredient is mixed, under sterile conditions, with a pharmaceutically acceptable carrier and/or any required preservative and/or buffering agent that may be needed. Additionally, the present invention contemplates the use of a transdermal patch, which often offers the added advantage of providing controlled delivery of the active ingredient into the body. Such dosage forms may be prepared, for example, by dissolving and/or dispersing the active ingredient in an appropriate medium. Alternatively or additionally, the rate of release may be controlled by providing a rate-controlling membrane and/or by dispersing the active ingredient in a polymer matrix and/or gel.

Formulations for topical administration include liquid and/or semi-liquid formulations such as liniments, lotions, oil-in-water and/or water-in-oil emulsions (for example, creams, ointments, and/or pastes), and/or solutions and/or suspensions, but are not limited thereto. The concentration of the active ingredient may be as high as the solubility limit of the active ingredient in the solvent; however, topically administrable formulations may, for example, contain about 1% to about 10% (w/w) of the active ingredient. Formulations for topical administration may further include one or more additional components as described herein.

The novel compound of the present invention or a pharmaceutical composition containing the same, as described herein, is typically manufactured in a unit dosage form for ease and uniformity of administration. However, it will be understood that the total daily regimen of the composition of the present invention will be determined by the attending physician within the sound scope of medical judgment. The particular therapeutically effective dosage level for any given subject will depend on a variety of factors, including the disease, disorder, or condition being treated and the severity thereof; the activity of the particular active ingredient employed; the specific composition adopted; the age, body weight, general health, sex, and diet of the subject; the time, route, and rate of excretion of administration of the particular active ingredient; the duration of the treatment; the drug used in combination or concurrently with the particular active ingredient adopted; and other factors well known in the medical art.

The novel compound of the present invention, its salt, or a pharmaceutical composition thereof may be administered by any route. In some embodiments, the novel compound, its salt, or the pharmaceutical composition thereof is administered by various routes, including oral, intravenous, intramuscular, intra-arterial, intramedullary, intrathecal, subcutaneous, intraventricular, transdermal, intradermal, rectal, intravaginal, intraperitoneal, topical (in the form of powder, ointment, cream, and/or drop), mucosal, nasal, oral, enteral, sublingual, intratracheal instillation, intrabronchial instillation, and/or inhalation; and/or by oral spray, nasal spray, and/or aerosol. Routes specifically considered include penetrating intravenous injection, local administration through blood and/or lymphatic supply, and/or direct administration to the affected site. In general, the most appropriate route of administration will depend on various factors, including the properties of the agent (for example, its stability in the gastrointestinal environment) and the condition of the subject (for example, whether the subject can tolerate oral administration).

In certain embodiments, the novel compound of the present invention, its salt, or a pharmaceutical composition thereof may be administered at a dosage level sufficient to deliver about 0.001 mg/kg to about 100 mg/kg, about 0.01 mg/kg to about 50 mg/kg, about 0.1 mg/kg to about 40 mg/kg, about 0.5 mg/kg to about 30 mg/kg, about 0.01 mg/kg to about 10 mg/kg, about 0.1 mg/kg to about 10 mg/kg, or about 1 mg/kg to about 25 mg/kg of body weight of the subject, administered once or more per day, to achieve the desired therapeutic effect. The intended dosage may be delivered three times a day, twice a day, once daily, once every two days, once every three days, once a week, once every two weeks, once every three weeks, or once every four weeks. In certain embodiments, the intended dosage may be delivered through multiple administrations (for example, 2, 3, 4, 5, 6, 7, 8, 9, 10, 11, 12, 13, or 14 or more administrations).

It will be understood that the dosage ranges described in the present invention provide guidance for the administration of the pharmaceutical composition to adults. For example, the amount administered to children or adolescents may be determined by a medical specialist or a person skilled in the art, and may be less than or equal to that administered to adults. The exact amount of the peptide according to the present invention required to achieve an effective amount will vary from subject to subject depending, for example, on the species, age, and general condition of the subject, the side effects or severity of the disorder, the identity of the compound, and the mode of administration.

It will be understood that the novel compounds and pharmaceutical compositions of the present invention may be used in combination therapy. The specific combination of treatments (therapeutic agents and/or procedures) for use in the combination therapy will take into account the desired therapeutic effect to be achieved and the suitability of the intended therapeutic agents and/or procedures.

The pharmaceutical composition of the present invention may be administered alone or in combination with one or more therapeutically active agents. In the case of a "combination," while the following modes of administration fall within the scope of the present invention, it is not intended to imply that the agents must necessarily be administered at the same time and/or formulated for co-delivery. The composition may be administered concurrently with, prior to, or subsequent to one or more other intended therapeutic agents or medical procedures. In general, each agent will be administered in a dosage and/or according to a time schedule appropriate for that agent. In addition, the present invention encompasses administering the pharmaceutical composition of the present invention in combination with an agent that can improve its bioavailability in the body, reduce and/or modify its metabolism, inhibit its excretion, and/or modify its distribution. It will be further understood that the novel compound and therapeutically active agent of the present invention used in such combination may be administered together in a single composition or separately in different compositions.

The specific combination used in the combination therapy will take into account the desired therapeutic effect to be achieved and/or the suitability of the procedure and/or therapeutically active agent comprising the peptide of the present invention. It will be understood that the combination used may achieve the intended effect for the same disorder (for example, the novel compound of the present invention may be administered in combination with another therapeutically active agent, such as a second therapeutic agent, used to treat the same disorder) and/or may achieve different effects (for example, control of any side effects).

As used herein, the term "therapeutically active agent" refers to any substance used as a medicament for treating, preventing, delaying, reducing, or ameliorating a disorder, and includes substances used for both prophylactic and curative treatment.

In some embodiments, another therapeutically active agent for combination administration is for the treatment of multiple myeloma. In some embodiments, the other therapeutically active agent may be a proteasome inhibitor and/or an immune-modulating drug. In the present invention, the other therapeutically active agent may be selected from the group consisting of dexamethasone, bortezomib, carfilzomib, melphalan, doxorubicin, and cyclophosphamide, but is not limited thereto.

In some embodiments, the pharmaceutical composition of the present invention may be administered in combination with any therapeutically active agent or procedure (e.g., surgery or radiation therapy) that is useful for treating, alleviating, improving, or mitigating one or more symptoms or characteristics, delaying their onset, inhibiting their progression, reducing their severity, and/or decreasing their incidence.

In one aspect, the present invention may be provided as a kit for treating a subject suffering from a tumor or cancerous disease.

In some embodiments, the kit includes i) instructions for administering the novel compound or pharmaceutical composition according to the present invention to a subject suffering from the disease; and ii) the novel compound or pharmaceutical composition according to the present invention. In some embodiments, the kit may comprise one or more unit dosage forms containing a dose of the novel compound or pharmaceutical composition as described herein that is effective for treating the disease in the subject. In some embodiments, the subject is a human patient.

In some embodiments, the kit further includes one or more items selected from the group consisting of a sterile syringe, a sterile needle, a sterile IV bag, an infusion pump, and any combination thereof.

As used in the present invention, the term "about" may be interpreted to mean approximately, nearly, roughly, or within a reasonable degree of variation. When the term "about" is used in conjunction with a numerical range, it is construed to modify the stated range by extending its boundaries above and below the specified values. In general, the term "about" is used in the present invention to modify numerical values indicated by a 10% variance above and below the specified value.

The terms "individual," "patient," and "subject" are used interchangeably and refer to any animal, including mammals such as mice, rats, other rodents, rabbits, dogs, cats, pigs, cattle, sheep, horses, or primates including humans.

As used herein, the term "treat" or "treatment," unless otherwise specified, refers to reversing, alleviating, inhibiting the progression of, or preventing a disease or disorder to which the term applies, or one or more symptoms thereof. The term "treating," as used herein, refers to the act of carrying out such treatment as defined above. Accordingly, the "treatment" or "therapy" of a disease in a mammal may include one or more of the following:
(1) arresting the development of the disease;
(2) preventing the spread of the disease;
(3) alleviating the disease;
(4) preventing recurrence of the disease; and
(5) palliating symptoms of the disease.

As used herein, the term "prevent" or "prevention," unless otherwise specified, refers to any act of suppressing or delaying the onset of a disease through the administration of the pharmaceutical composition according to the present invention.

In another aspect, the present invention provides a method for preventing or treating a cereblon activity-related disease, such as a benign tumor or cancer, comprising administering to a patient in need thereof a compound, an optical isomer thereof, a racemic mixture thereof, a hydrate thereof, a solvate thereof, or a pharmaceutically acceptable salt thereof.

The present invention also provides the compound, an optical isomer thereof, a racemic mixture thereof, a hydrate thereof, a solvate thereof, or a pharmaceutically acceptable salt thereof for used in prevention or treatment of a cereblon activity-related disease, such as a benign tumor or cancer.

The present invention further provides the use of the compound, an optical isomer thereof, a racemic mixture thereof, a hydrate thereof, a solvate thereof, or a pharmaceutically acceptable salt thereof in the manufacture of a medicament for preventing or treating a cereblon activity-related disease, such as a benign tumor or cancer.

The present invention also provides a method for preparing the compounds shown in Table 1. Hereinafter, among the preparation methods for the compounds shown in Table 1 according to the present invention, synthetic methods for some of the compounds are exemplified; however, the synthetic methods described in Preparation Examples 1 to 15 are not intended to limit the methods for preparing the compounds of the present invention. It will be apparent that the synthetic methods are merely illustrative and may be readily modified by those skilled in the art depending on specific substituents.

### Preparation Method

### Preparation of Intermediate B (3-amino-N-(3-(2,6-dioxopiperidin-3-yl)phenyl)benzenesulfonamide)

Intermediate B, which is used for the preparation of the compound of the present invention, may be prepared by the following reactions:
(a') reacting 2,6-bis(benzyloxy)-3-bromopyridine and 3-aminophenylboronic acid to prepare 3-(2,6-bis(benzyloxy)pyridin-3-yl)aniline;
(b') reacting 3-(2,6-bis(benzyloxy)pyridin-3-yl)aniline with hydrogen to prepare 3-(3-aminophenyl)piperidine-2,6-dione;
(c') reacting 3-(3-aminophenyl)piperidine-2,6-dione with 3-nitrobenzenesulfonyl chloride to prepare N-(3-(2,6-dioxopiperidin-3-yl)phenyl)-3-nitrobenzenesulfonamide; and
(d') reacting N-(3-(2,6-dioxopiperidin-3-yl)phenyl)-3-nitrobenzenesulfonamide with hydrogen to prepare 3-amino-N-(3-(2,6-dioxopiperidin-3-yl)phenyl)benzenesulfonamide.

In the above method, step (a') may comprise adding Pd(dppf)Cl₂ and potassium carbonate to a mixed solution of 1,4-dioxane and water, followed by further addition and reaction of 2,6-bis(benzyloxy)-3-bromopyridine and 3-aminophenylboronic acid.

In the above method, step (a') may be carried out at about 80 to 100°C, for example, about 85 to 95°C, for a reaction time of about 8 to 16 hours, for example, about 9 to 15 hours, and preferably about 10 to 14 hours, but is not limited thereto.

In the above method, step (b') may comprise adding 3-(2,6-bis(benzyloxy)pyridin-3-yl)aniline and palladium on carbon to a mixed solution of tetrahydrofuran (THF) and methanol, followed by reacting with hydrogen.

In the above method, step (b') may be carried out at room temperature for about 2 to 6 hours, preferably about 3 to 5 hours, but is not limited thereto.

In the present invention, the term "room temperature" refers to a general indoor temperature, which may be about 15 to 30°C, and preferably about 20 to 28°C, but is not limited thereto.

In the above method, step (c') may comprise adding 3-(3-aminophenyl)piperidine-2,6-dione and 3-nitrobenzenesulfonyl chloride to a mixed solution of dichloromethane and pyridine, followed by reaction.

In the above method, step (c') may be carried out at room temperature for about 8 to 16 hours, for example, about 9 to 15 hours, and preferably about 10 to 14 hours, but is not limited thereto.

In the above method, step (d') may comprise adding N-(3-(2,6-dioxopiperidin-3-yl)phenyl)-3-nitrobenzenesulfonamide and palladium on carbon to methanol, followed by reacting with hydrogen.

In the above method, step (d') may be carried out at room temperature for about 2 to 6 hours, preferably about 3 to 5 hours, but is not limited thereto.

### Method for Preparing the Compounds represented by Chemical Formulae (i) to (vii)

The method for preparing the compounds of Chemical Formulae (2) to (8) may comprise (a) reacting C₆H₅-(CH₂)n-C(O)Cl (where n is 0 to 6) with 3-amino-N-(3-(2,6-dioxopiperidin-3-yl)phenyl)benzenesulfonamide.

In the above method, step (a) may comprise dissolving potassium carbonate in N,N-dimethylacetamide, followed by adding C₆H₅-(CH₂)n-C(O)Cl (where n is an integer from 0 to 6) and 3-amino-N-(3-(2,6-dioxopiperidin-3-yl)phenyl)benzenesulfonamide to react them.

In the above method, step (a) may be carried out at room temperature and may comprise stirring and reacting for less than 1 hour, for example, about 1 to 20 minutes, such as about 2 to 10 minutes, preferably about 4 to 8 minutes, but is not limited thereto.

As one embodiment, the method for preparing the compound represented by Chemical Formula (2), N-(3-(N-(3-(2,6-dioxopiperidin-3-yl)phenyl)sulfamoyl)phenyl)-4-methylbenzamide, may comprise (a) reacting 3-amino-N-(3-(2,6-dioxopiperidin-3-yl)phenyl)benzenesulfonamide with benzoyl chloride:

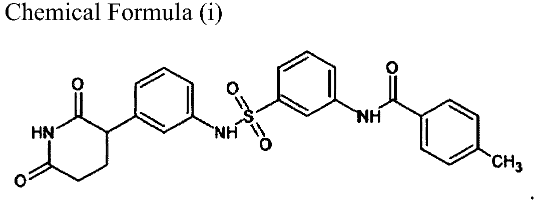

As one embodiment, the method for preparing the compound represented by Chemical Formula (3), N-(3-(N-(3-(2,6-dioxopiperidin-3-yl)phenyl)sulfamoyl)phenyl)-4-ethylbenzamide, may comprise (a) reacting 3-amino-N-(3-(2,6-dioxopiperidin-3-yl)phenyl)benzenesulfonamide with 2-phenylacetyl chloride:

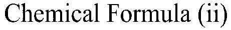

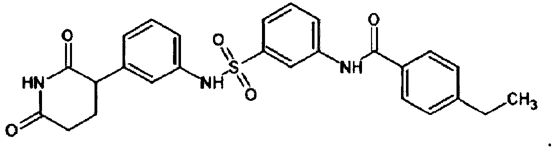

As one embodiment, the method for preparing the compound represented by Chemical Formula (4), N-(3-(N-(3-(2,6-dioxopiperidin-3-yl)phenyl)sulfamoyl)phenyl)-4-propylbenzamide, may comprise (a) reacting 3-amino-N-(3-(2,6-dioxopiperidin-3-yl)phenyl)benzenesulfonamide with 3-phenylpropanoyl chloride:

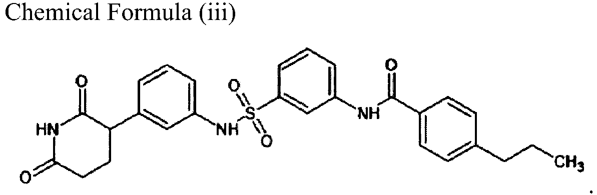

As one embodiment, the method for preparing the compound represented by Chemical Formula (iv), N-(3-(N-(3-(2,6-dioxopiperidin-3-yl)phenyl)sulfamoyl)phenyl)-4-butylbenzamide, may comprise (a) reacting 3-amino-N-(3-(2,6-dioxopiperidin-3-yl)phenyl)benzenesulfonamide with 4-phenylbutanoyl chloride:

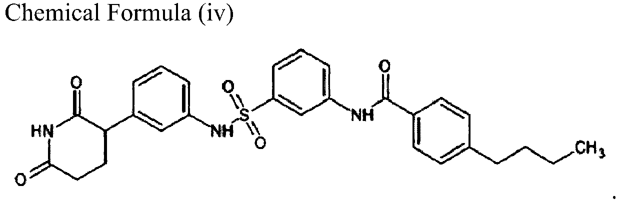

As one embodiment, the method for preparing the compound represented by Chemical Formula (v), N-(3-(N-(3-(2,6-dioxopiperidin-3-yl)phenyl)sulfamoyl)phenyl)-4-pentylbenzamide, may comprise (a) reacting 3-amino-N-(3-(2,6-dioxopiperidin-3-yl)phenyl)benzenesulfonamide with 5-phenylpentanoyl chloride:

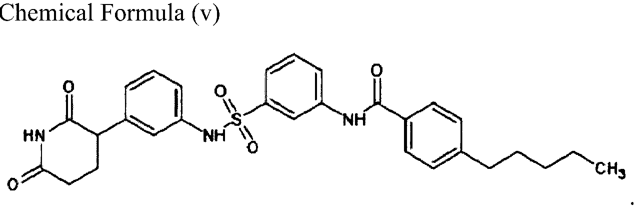

As one embodiment, the method for preparing the compound represented by Chemical Formula (vi), N-(3-(N-(3-(2,6-dioxopiperidin-3-yl)phenyl)sulfamoyl)phenyl)-4-hexylbenzamide, may comprise (a) reacting 3-amino-N-(3-(2,6-dioxopiperidin-3-yl)phenyl)benzenesulfonamide with 6-phenylhexanoyl chloride:

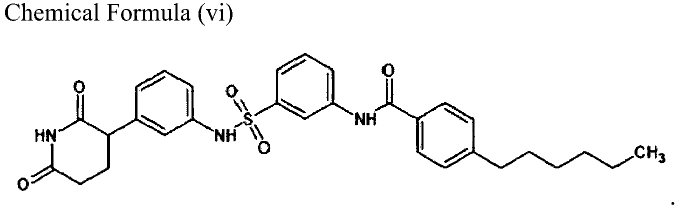

As one embodiment, the method for preparing the compound represented by Chemical Formula (vii), N-(3-(N-(3-(2,6-dioxopiperidin-3-yl)phenyl)sulfamoyl)phenyl)-4-heptylbenzamide, may comprise (a) reacting 7-phenylheptanoyl chloride with 3-amino-N-(3-(2,6-dioxopiperidin-3-yl)phenyl)benzenesulfonamide:

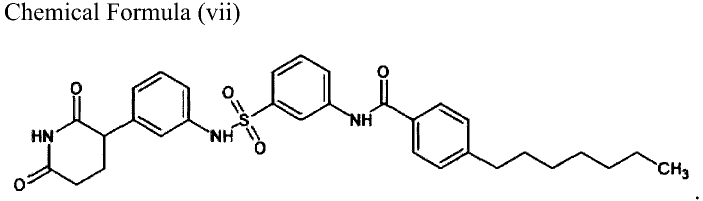

The 7-phenylheptanoyl chloride may be prepared by reacting 7-phenylheptanoic acid with thionyl chloride, and the reaction may be carried out at a temperature of about 60 to 80°C.

### Method for preparing the compound represented by Chemical Formula (ix) or Chemical Formula (x)

The method for preparing the compound of Chemical Formula (ix) or Chemical Formula (x) may comprise (a) reacting 4-chlorobenzoyl chloride or 4-(chloromethyl)benzoyl chloride with 3-amino-N-(3-(2,6-dioxopiperidin-3-yl)phenyl)benzenesulfonamide.

In the above method, step (a) may comprise dissolving potassium carbonate in N,N-dimethylacetamide, followed by adding 4-chlorobenzoyl chloride or 4-(chloromethyl)benzoyl chloride and 3-amino-N-(3-(2,6-dioxopiperidin-3-yl)phenyl)benzenesulfonamide to react them.

In the above method, step (a) may be carried out at room temperature and may comprise stirring and reacting for less than 1 hour, for example, about 1 to 20 minutes, such as about 2 to 10 minutes, preferably about 4 to 8 minutes, but is not limited thereto.

As one embodiment, the method for preparing the compound represented by Chemical Formula (ix), 4-chloro-N-(3-(N-(3-(2,6-dioxopiperidin-3-yl)phenyl)sulfamoyl)phenyl)benzamide, may comprise (a) reacting 3-amino-N-(3-(2,6-dioxopiperidin-3-yl)phenyl)benzenesulfonamide with 4-chlorobenzoyl chloride:

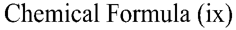

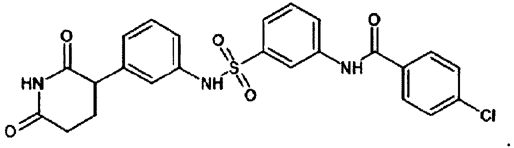

As one embodiment, the method for preparing the compound represented by Chemical Formula (x), 4-(chloromethyl)-N-(3-(N-(3-(2,6-dioxopiperidin-3-yl)phenyl)sulfamoyl)phenyl)benzamide, may comprise (a) reacting 3-amino-N-(3-(2,6-dioxopiperidin-3-yl)phenyl)benzenesulfonamide with 4-(chloromethyl)benzoyl chloride:

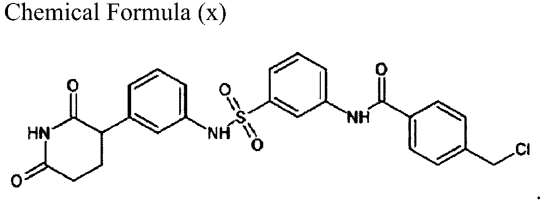

### Method for Preparing the Compounds represented by Chemical Formulae (xi) to (xv)

The method for preparing the compounds of Chemical Formulae (xi) to (xv) may comprise (a) reacting Cl-(CH₂)n-C₆H₄-COOH (where n is an integer from 2 to 6) with 3-amino-N-(3-(2,6-dioxopiperidin-3-yl)phenyl)benzenesulfonamide.

In the above method, step (a) may involve dissolving potassium carbonate in N,N-dimethylacetamide, followed by adding Cl-(CH₂)n-C₆H₄-COOH (where n is an integer from 2 to 6) and 3-amino-N-(3-(2,6-dioxopiperidin-3-yl)phenyl)benzenesulfonamide to react them.

In the above method, step (a) may be carried out at room temperature and may comprise stirring and reacting for less than 1 hour, for example, about 1 to 20 minutes, such as about 2 to 10 minutes, preferably about 4 to 8 minutes, but is not limited thereto.

In the above method, Cl-(CH₂)n-C₆H₄-COOH (where n is an integer from 2 to 6) may be prepared by demethylating Cl-(CH₂)n-C₆H₄-COOCH₃ (where n is an integer from 2 to 6) with lithium hydroxide, and the demethylation reaction may be carried out at room temperature, and the reaction may be performed by stirring and reacting for about 2 to 20 hours, for example, about 4 to 12 hours, preferably about 6 to 10 hours, although the present invention is not limited thereto. In this case, Cl-(CH₂)n-C₆H₄-COOCH₃ (where n is an integer from 2 to 6) may be dissolved in a mixed solution of methanol and water for the demethylation reaction.

The Cl-(CH₂)n-C₆H₄-COOCH₃ (where n is an integer from 2 to 6) may be prepared by halogenating OH-(CH₂)n-C₆H₄-COOCH₃ (where n is an integer from 2 to 6) with thionyl chloride. The halogenation reaction may be carried out at about 40 to 80°C, for example, about 50 to 70°C, preferably about 60°C, and the reaction may be performed by stirring and reacting for about 2 to 20 hours, for example, about 4 to 12 hours, preferably about 6 to 10 hours, although the present invention is not limited thereto. In this case, OH-(CH₂)n-C₆H₄-COOCH₃ (where n is an integer from 2 to 6) may be dissolved in toluene for the halogenation reaction.

In one embodiment, the method for preparing the compound represented by Chemical Formula (xi), 4-(2-chloroethyl)-N-(3-(N-(3-(2,6-dioxopiperidin-3-yl)phenyl)sulfamoyl)phenyl)benzamide, may comprise reacting 4-(2-chloroethyl)benzoic acid with 3-amino-N-(3-(2,6-dioxopiperidin-3-yl)phenyl)benzenesulfonamide:

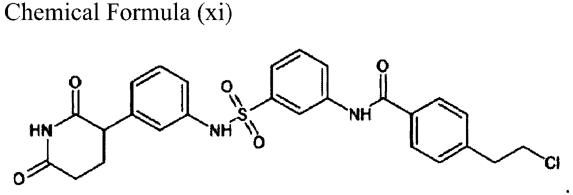

In a preferred embodiment, the compound represented by Chemical Formula (xi) may be prepared by a method comprising the following steps:

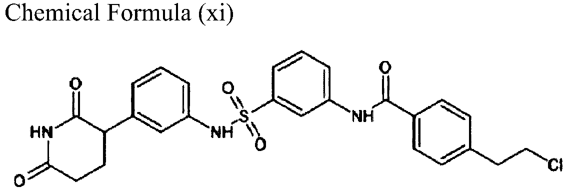

(a) reacting methyl 4-(2-oxopropyl)benzoate with thionyl chloride to prepare methyl 4-(2-chloroethyl)benzoate;
(b) demethylating methyl 4-(2-chloroethyl)benzoate by adding lithium hydroxide to prepare 4-(2-chloroethyl)benzoic acid; and
(c) reacting 4-(2-chloroethyl)benzoic acid with 3-amino-N-(3-(2,6-dioxopiperidin-3-yl)phenyl)benzenesulfonamide to prepare the compound represented by Chemical Formula (xi).

In one embodiment, the method for preparing the compound represented by Chemical Formula (xii), 4-(3-chloropropyl)-N-(3-(N-(3-(2,6-dioxopiperidin-3-yl)phenyl)sulfamoyl)phenyl)benzamide, may comprise reacting 4-(3-chloropropyl)benzoic acid with 3-amino-N-(3-(2,6-dioxopiperidin-3-yl)phenyl)benzenesulfonamide:

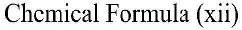

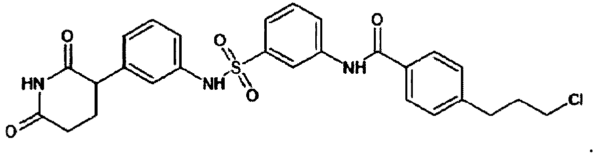

In a preferred embodiment, the compound represented by Chemical Formula (xii) may be prepared by a method comprising the following steps:

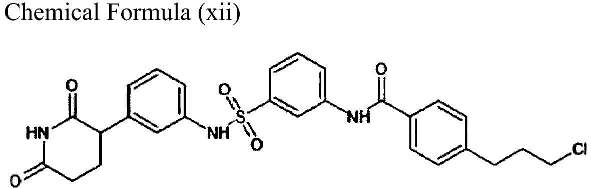

(a) reacting methyl 4-iodobenzoate with prop-2-yn-1-ol to prepare methyl 4-(3-hydroxyprop-1-yn-1-yl)benzoate;
(b) reacting methyl 4-(3-hydroxyprop-1-yn-1-yl)benzoate with hydrogen to prepare methyl 4-(3-hydroxypropyl)benzoate;
(c) reacting methyl 4-(3-hydroxypropyl)benzoate with thionyl chloride to halogenate and prepare methyl 4-(3-chloropropyl)benzoate;
(d) demethylating methyl 4-(3-chloropropyl)benzoate by adding lithium hydroxide to prepare 4-(3-chloropropyl)benzoic acid; and
(e) reacting 4-(3-chloropropyl)benzoic acid with 3-amino-N-(3-(2,6-dioxopiperidin-3-yl)phenyl)benzenesulfonamide to prepare the compound represented by Chemical Formula (xii).

In one embodiment, the method for preparing the compound represented by Chemical Formula (xiii), 4-(4-chlorobutyl)-N-(3-(N-(3-(2,6-dioxopiperidin-3-yl)phenyl)sulfamoyl)phenyl)benzamide, may comprise reacting 4-(4-chlorobutyl)benzoic acid with 3-amino-N-(3-(2,6-dioxopiperidin-3-yl)phenyl)benzenesulfonamide:

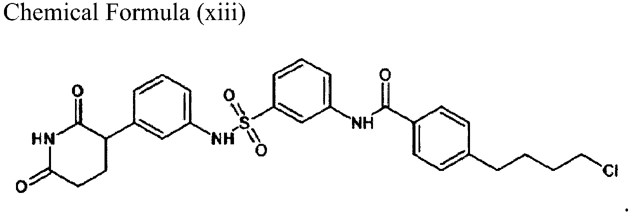

In a preferred embodiment, the compound represented by Chemical Formula (xiii) may be prepared by a method comprising the following steps: 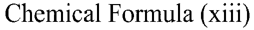 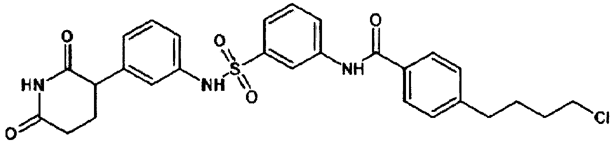
(a) reacting methyl 4-(4-hydroxybutyl)benzoate with thionyl chloride to halogenate and prepare methyl 4-(4-chlorobutyl)benzoate;
(b) demethylating methyl 4-(4-chlorobutyl)benzoate by adding lithium hydroxide to prepare 4-(4-chlorobutyl)benzoic acid; and
(c) reacting 4-(4-chlorobutyl)benzoic acid with 3-amino-N-(3-(2,6-dioxopiperidin-3-yl)phenyl)benzenesulfonamide to prepare the compound represented by Chemical Formula (xiii).

In one embodiment, the method for preparing the compound represented by Chemical Formula (xiv), 4-(5-chloropentyl)-N-(3-(N-(3-(2,6-dioxopiperidin-3-yl)phenyl)sulfamoyl)phenyl)benzamide, may comprise reacting 4-(5-chloropentyl)benzoic acid with 3-amino-N-(3-(2,6-dioxopiperidin-3-yl)phenyl)benzenesulfonamide:

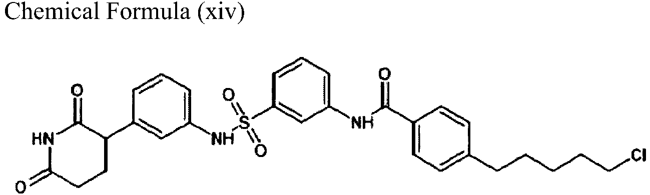

In a preferred embodiment, the compound represented by Chemical Formula (xiv) may be prepared by a method comprising the following steps:

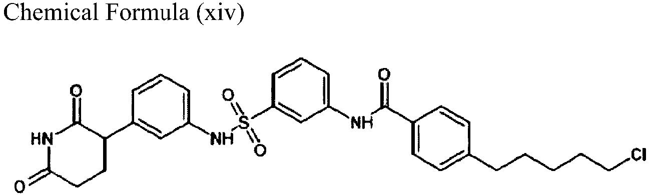

(a) reacting methyl 4-iodobenzoate with pent-4-yn-1-ol to prepare methyl 4-(5-hydroxypent-1-yn-1-yl)benzoate;
(b) reacting methyl 4-(5-hydroxypent-1-yn-1-yl)benzoate with hydrogen to prepare methyl 4-(5-hydroxypentyl)benzoate;
(c) reacting methyl 4-(5-hydroxypentyl)benzoate with thionyl chloride to halogenate and prepare methyl 4-(5-chloropentyl)benzoate;
(d) demethylating methyl 4-(5-chloropentyl)benzoate by adding lithium hydroxide to prepare 4-(5-chloropentyl)benzoic acid; and
(e) reacting 4-(5-chloropentyl)benzoic acid with 3-amino-N-(3-(2,6-dioxopiperidin-3-yl)phenyl)benzenesulfonamide to prepare the compound represented by Chemical Formula (xiv).

In one embodiment, the method for preparing the compound represented by Chemical Formula (xv), 4-(6-chlorohexyl)-N-(3-(N-(3-(2,6-dioxopiperidin-3-yl)phenyl)sulfamoyl)phenyl)benzamide, may comprise reacting 4-(6-chlorohexyl)benzoic acid with 3-amino-N-(3-(2,6-dioxopiperidin-3-yl)phenyl)benzenesulfonamide:

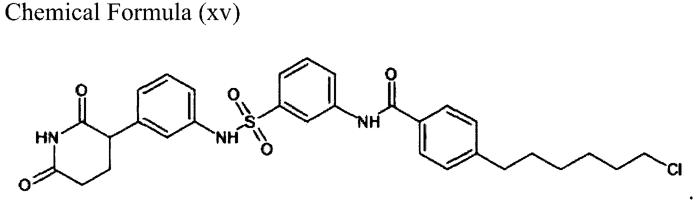

In a preferred embodiment, the compound represented by Chemical Formula (xv) may be prepared by a method comprising the following steps:

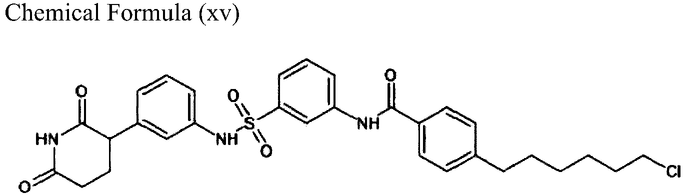

(a) reacting methyl 4-iodobenzoate with hex-5-yn-1-ol to prepare methyl 4-(6-hydroxyhex-1-yn-1-yl)benzoate;
(b) reacting methyl 4-(6-hydroxyhex-1-yn-1-yl)benzoate with hydrogen to prepare methyl 4-(6-hydroxyhexyl)benzoate;
(c) reacting methyl 4-(6-hydroxyhexyl)benzoate with thionyl chloride to halogenate and prepare methyl 4-(6-chlorohexyl)benzoate;
(d) demethylating methyl 4-(6-chlorohexyl)benzoate by adding lithium hydroxide to prepare 4-(6-chlorohexyl)benzoic acid; and
(e) reacting 4-(6-chlorohexyl)benzoic acid with 3-amino-N-(3-(2,6-dioxopiperidin-3-yl)phenyl)benzenesulfonamide to prepare the compound represented by Chemical Formula (xv).

In another aspect, the present invention relates to a cereblon activity inhibitor or a composition for inhibiting cereblon activity, comprising the compound, an optical isomer thereof, a racemic mixture thereof, a hydrate thereof, or a solvate thereof.

In one embodiment, the compound, an optical isomer thereof, a racemic mixture thereof, a hydrate thereof, a solvate thereof, or a salt thereof may be provided as an experimental reagent used to inhibit cereblon activity in vitro.

Hereinafter, the present invention will be described in more detail with reference to the Preparation Examples and Examples below. However, the following Preparation Examples and Examples are provided merely for illustration of the present invention, and the scope of the present invention is not limited thereto.

### Preparation Example

### Preparation of Intermediate B

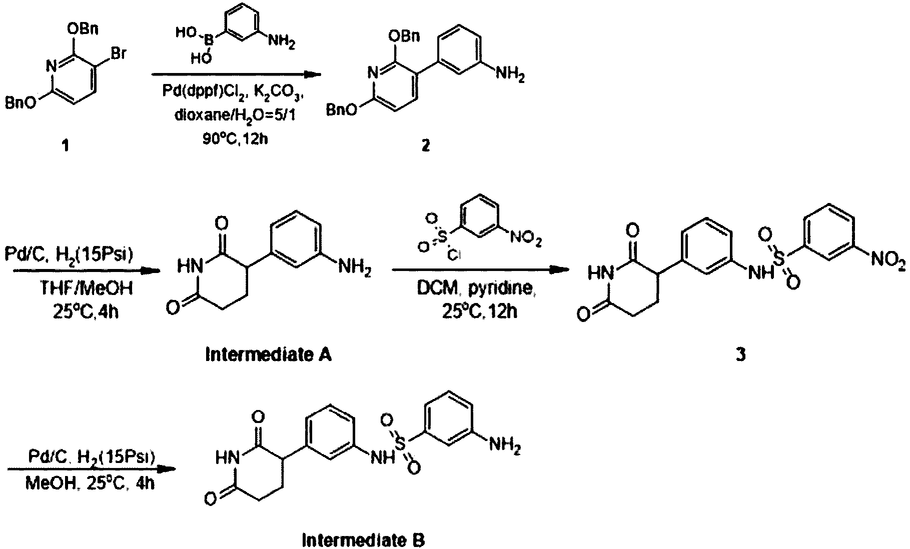

### Preparation of Compound 2

A mixture of 1,4**-dioxane** (CAS: 123-91-1) and water (H₂O) in a 5:1 ratio was prepared, and PD(dppf)Cl₂ (CAS: 72287-26-4) and potassium carbonate (CAS: 584-08-7) were added. **Compound 1** (2,6-bis(benzyloxy)-3-bromopyridine, CAS: 16727-47-2) and 3-aminophenylboronic acid (CAS: 206658-89-1) were then added, and the mixture was stirred at 90°C for 12 hours to obtain **Compound 2** (3-(2,6-bis(benzyloxy)pyridin-3-yl)aniline).

### Preparation of Intermediate A

A mixture of tetrahydrofuran (THF, CAS: 109-99-9) and methanol (CAS: 67-56-1) was prepared, to which **Compound 2** and palladium on carbon (CAS: 7440-05-3) were added. Hydrogen gas (H₂, 15 psi) was introduced, and the reaction mixture was stirred at room temperature for 4 hours to obtain **Intermediate A** (3-(3-aminophenyl)piperidine-2,6-dione).

### Preparation of Compound 3

A mixture of dichloromethane (CAS: 75-09-2) and pyridine (CAS: 110-86-1) was prepared, to which **Intermediate A** (3-(3-aminophenyl)piperidine-2,6-dione) and 3-nitrobenzenesulfonyl chloride (CAS: 121-51-7) were added. The reaction mixture was stirred at room temperature for 12 hours to obtain **Compound 3** (N-(3-(2,6-dioxopiperidin-3-yl)phenyl)-3-nitrobenzenesulfonamide).

### Preparation of Intermediate B

**Compound 3** (N-(3-(2,6-dioxopiperidin-3-yl)phenyl)-3-nitrobenzenesulfonamide) and palladium on carbon (CAS: 7440-05-3) were added to methanol (CAS: 67-56-1). Hydrogen gas (H₂, 15 psi) was introduced, and the reaction mixture was stirred at room temperature for 4 hours to obtain **Intermediate B** (3-amino-N-(3-(2,6-dioxopiperidin-3-yl)phenyl)benzenesulfonamide).

### Preparation Example 1. Preparation of Compound of Chemical Formula (i) (AEV41009)

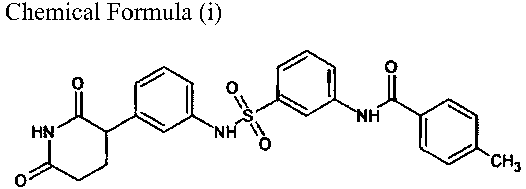

Potassium carbonate (CAS: 584-08-7) was dissolved in N,N-dimethylacetamide (CAS: 127-19-5), and **Intermediate B** and benzoyl chloride (CAS: 98-88-4) were added. The mixture was stirred at room temperature for 6 minutes to obtain Compound of Chemical Formula (i) **(AEV41009;** N-(3-(N-(3-(2,6-dioxopiperidin-3-yl)phenyl)sulfamoyl)phenyl)-4-methylbenzamide).

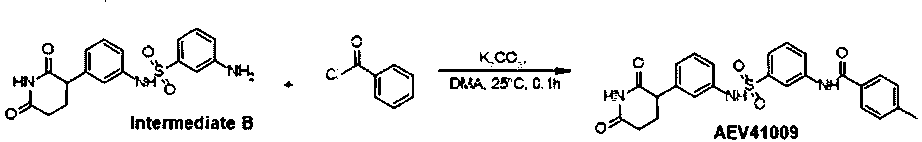

### Preparation Example 2. Preparation of Compound of Chemical Formula (ii) (AEV41010)

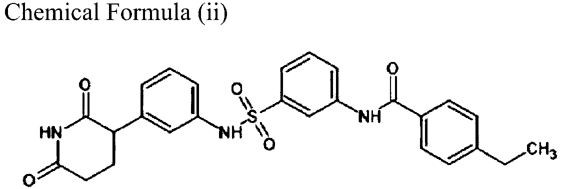

Potassium carbonate (CAS: 584-08-7) was dissolved in N,N-dimethylacetamide (CAS: 127-19-5), and **Intermediate B and** 2-phenylacetyl chloride (CAS: 103-80-0) were added. The mixture was stirred at room temperature for 6 minutes to obtain Compound of Chemical Formula (ii) **(AEV41010;** N-(3-(N-(3-(2,6-dioxopiperidin-3-yl)phenyl)sulfamoyl)phenyl)-4-ethylbenzamide).

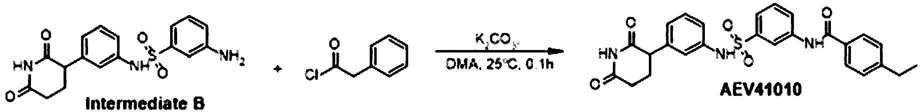

### Preparation Example 3. Preparation of Compound of Chemical Formula (iii) (AEV41011)

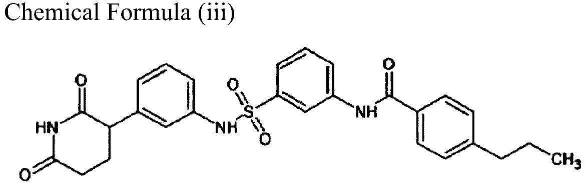

Potassium carbonate (CAS: 584-08-7) was dissolved in N,N-dimethylacetamide (CAS: 127-19-5), and **Intermediate B** and 3-phenylpropanoyl chloride (CAS: 645-45-4) were added. The mixture was stirred at room temperature for 6 minutes to obtain Compound of Chemical Formula (iii) **(AEV41011;** N-(3-(N-(3-(2,6-dioxopiperidin-3-yl)phenyl)sulfamoyl)phenyl)-4-propylbenzamide).

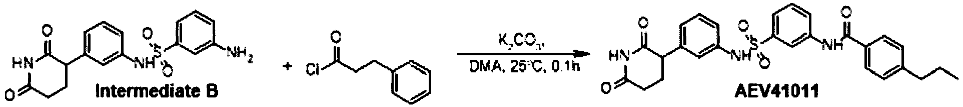

### Preparation Example 4. Preparation of Compound of Chemical Formula (iv) (AEV41012 or AEV40154)

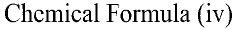

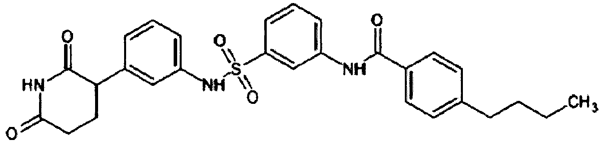

Potassium carbonate (CAS: 584-08-7) was dissolved in N,N-dimethylacetamide (CAS: 127-19-5), and **Intermediate B** and 4-phenylbutanoyl chloride (CAS: 18496-54-3) were added. The mixture was stirred at room temperature for 6 minutes to obtain Compound of Chemical Formula (iv) **(AEV41012;** 4-butyl-N-(3-(N-(3-(2,6-dioxopiperidin-3-yl)phenyl)sulfamoyl)phenyl)benzamide).

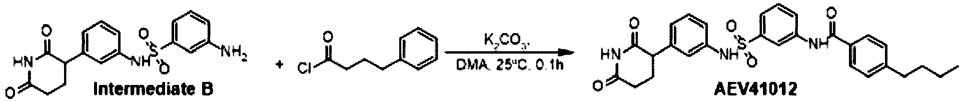

### Preparation Example 5. Preparation of Compound of Chemical Formula (v) (AEV41013)

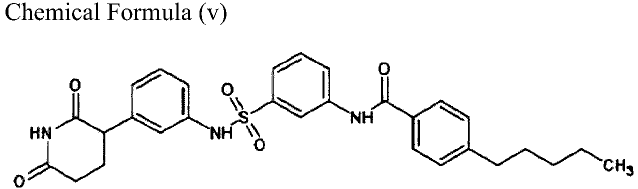

Potassium carbonate (CAS: 584-08-7) was dissolved in N,N-dimethylacetamide (CAS: 127-19-5), and **Intermediate B** and 5-phenylpentanoyl chloride (CAS: 20371-41-9) were added. The mixture was stirred at room temperature for 6 minutes to obtain Compound of Chemical Formula (v) **(AEV41013;** N-(3-(N-(3-(2,6-dioxopiperidin-3-yl)phenyl)sulfamoyl)phenyl)-4-pentylbenzamide).

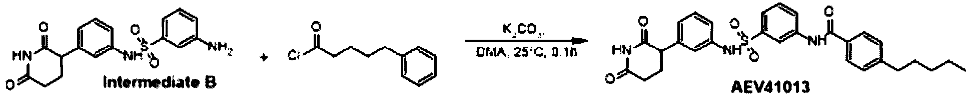

### Preparation Example 6. Preparation of Compound of Chemical Formula (vi) (AEV41083)

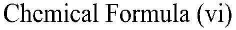

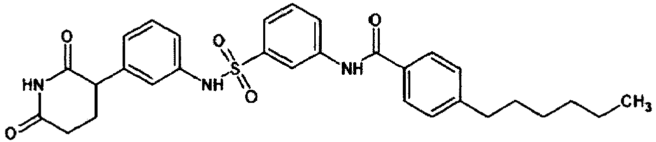

Potassium carbonate (CAS: 584-08-7) was dissolved in N,N-dimethylacetamide (CAS: 127-19-5), and **Intermediate B** and 6-phenylhexanoyl chloride (CAS: 21389-46-8) were added. The mixture was stirred at room temperature for 6 minutes to obtain Compound of Chemical Formula (vi) **(AEV41083;** N-(3-(N-(3-(2,6-dioxopiperidin-3-yl)phenyl)sulfamoyl)phenyl)-4-hexylbenzamide).

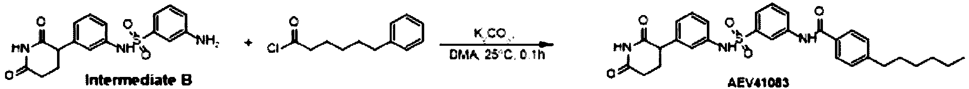

### Preparation Example 7. Preparation of Compound of Chemical Formula (vii) (AEV40204)

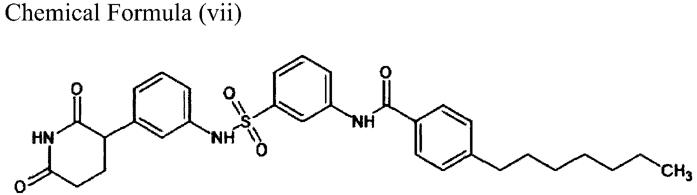

A mixture of Compound 204-1 (7-phenylheptanoic acid, CAS: 40228-90-8) and thionyl chloride (CAS: 7719-09-7) was stirred at 70°C to obtain **Compound 204-2** (7-phenylheptanoyl chloride).

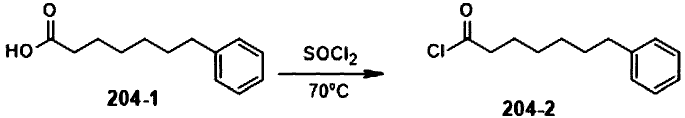

Potassium carbonate (CAS: 584-08-7) was dissolved in N,N-dimethylacetamide (CAS: 127-19-5), and **Intermediate B** and **Compound 204-2** (7-phenylheptanoyl chloride) were added. The mixture was stirred at room temperature for 6 minutes to obtain Compound of Chemical Formula (vii) **(AEV40204;** N-(3-(N-(3-(2,6-dioxopiperidin-3-yl)phenyl)sulfamoyl)phenyl)-4-heptylbenzamide).

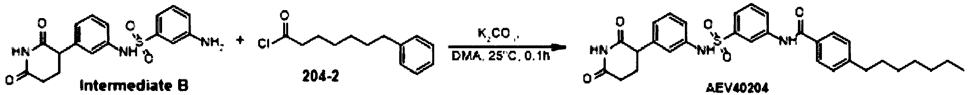

### Preparation Example 8. Preparation of Compound of Chemical Formula (viii) (AEV40264)

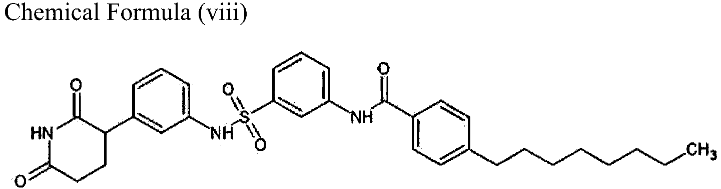

A mixture of **Intermediate B,** 4-octylbenzoic acid (CAS: 3575-31-3), HATU (1-[bis(dimethylamino)methylene]-1H-1,2,3-triazolo[4,5-b]pyridinium 3-oxide hexafluorophosphate, CAS: 148893-10-1), N,N-dimethylacetamide (CAS: 127-19-5), and triethylamine (CAS: 121-44-8) was stirred at 30°C for 12 hours to obtain Compound of Chemical Formula (viii) **(AEV40264;** N-(3-(N-(3-(2,6-dioxopiperidin-3-yl)phenyl)sulfamoyl)phenyl)-4-octylbenzamide).

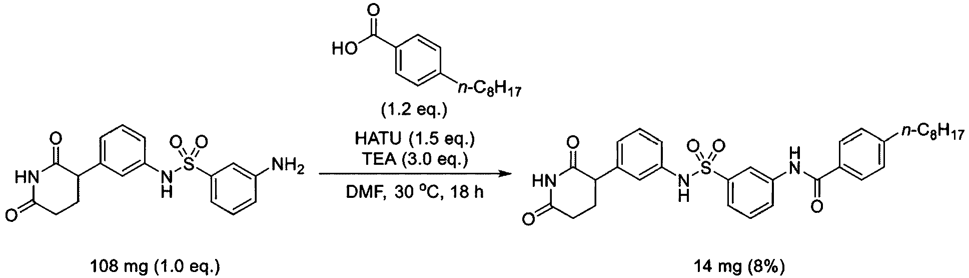

### Preparation Example 9. Preparation of Compound of Chemical Formula (ix) (AEV41001)

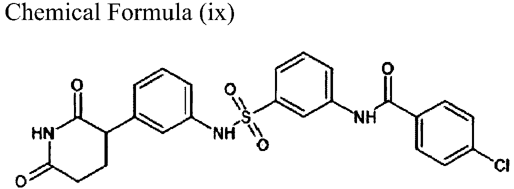

Potassium carbonate (CAS: 584-08-7) was dissolved in N,N-dimethylacetamide (CAS: 127-19-5), and **Intermediate B** and 4-chlorobenzoyl chloride (CAS: 104-83-6) were added. The mixture was stirred at room temperature for 6 minutes to obtain Compound of Chemical Formula (ix) **(AEV41001;** 4-chloro-N-(3-(N-(3-(2,6-dioxopiperidin-3-yl)phenyl)sulfamoyl)phenyl)benzamide).

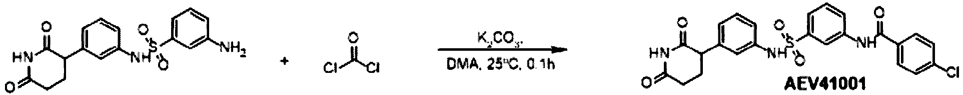

### Preparation Example 10. Preparation of Compound of Chemical Formula (x) (AEV41002)

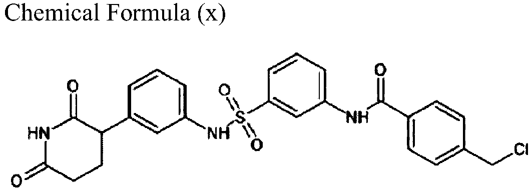

Potassium carbonate (CAS: 584-08-7) was dissolved in N,N-dimethylacetamide (CAS: 127-19-5), and **Intermediate B** and 4-(chloromethyl)benzoyl chloride (CAS: 63024-77-1) were added. The mixture was stirred at room temperature for 6 minutes to obtain Compound of Chemical Formula (x) **(AEV41002;** 4-(chloromethyl)-N-(3-(N-(3-(2,6-dioxopiperidin-3-yl)phenyl)sulfamoyl)phenyl)benzamide).

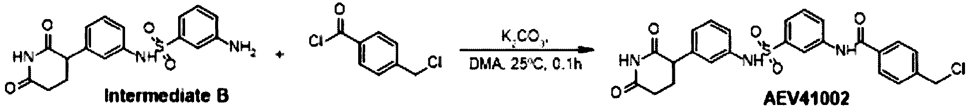

### Preparation Example 11. Preparation of Compound of Chemical Formula (xi) (AEV41003)

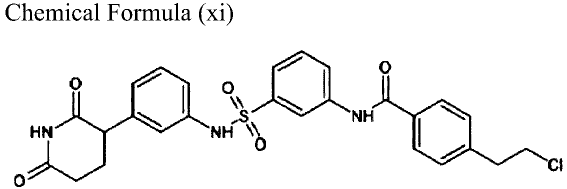

A mixture of methyl 4-(2-oxopropyl)benzoate (CAS: 46190-45-8), pyridine (CAS: 110-86-1), and thionyl chloride (CAS: 7719-09-7) in toluene (CAS: 108-88-3) was stirred at 60°C for 8 hours to obtain **Compound 3-2** (methyl 4-(2-chloroethyl)benzoate). A mixture of **Compound 3-2** (methyl 4-(2-chloroethyl)benzoate) and lithium hydroxide (CAS: 1310-65-2) in a mixed solution of methanol (CAS: 67-56-1) and water was stirred at room temperature for 8 hours to obtain **Compound 3-3** (4-(2-chloroethyl)benzoic acid).

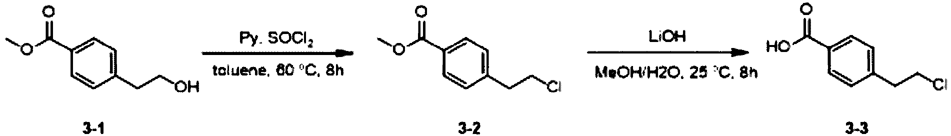

A mixture of potassium carbonate (CAS: 584-08-7) was dissolved in N,N-dimethylacetamide (CAS: 127-19-5), and **Intermediate B** and **Compound 3-3** (4-(2-chloroethyl)benzoic acid) were added. The mixture was stirred at room temperature for 6 minutes to obtain Compound of Chemical Formula (xi) **(AEV41003;** 4-(2-chloroethyl)-N-(3-(N-(3-(2,6-dioxopiperidin-3-yl)phenyl)sulfamoyl)phenyl)benzamide).

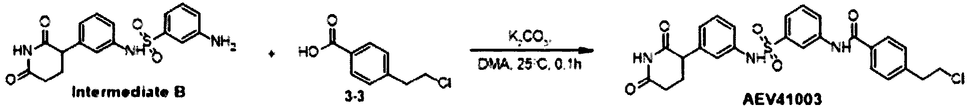

### Preparation Example 12. Preparation of Compound of Chemical Formula (xii) (AEV41004)

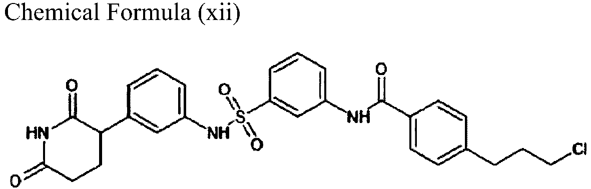

A mixture of PD(dppf)Cl₂ ([1,1'-(diphenylphosphino)ferrocene] dichloropalladium(II), CAS: 72287-26-4), copper(I) iodide (CAS: 7681-65-4), **Compound 4-1** (methyl 4-iodobenzoate, CAS: 619-44-3), and triethylamine (CAS: 121-44-8) was added to acetonitrile (CAS: 75-05-8), followed by the addition of prop-2-yn-1-ol (CAS: 107-19-7). The mixture was stirred at 60°C for 12 hours to obtain **Compound 4-2** (methyl 4-(3-hydroxyprop-1-yn-1-yl)benzoate, CAS: 61266-36-2).

A mixture of **Compound 4-2** (methyl 4-(3-hydroxyprop-1-yn-1-yl)benzoate, CAS: 61266-36-2) and palladium on carbon (CAS: 7440-05-3) in methanol (CAS: 67-56-1) was reacted under hydrogen gas (H₂, 15 psi) at room temperature for 12 hours to obtain **Compound 4-3** (methyl 4-(3-hydroxypropyl)benzoate, CAS: 15403-22-2).

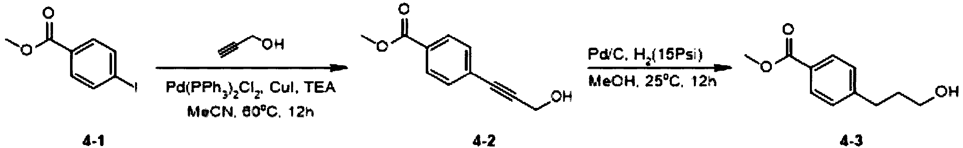

A mixture of **Compound** 4-3 (methyl 4-(3-hydroxypropyl)benzoate, CAS: 15403-22-2) and thionyl chloride (CAS: 7719-09-7) in toluene (CAS: 108-88-3) was stirred at 60°C for 8 hours to obtain **Compound** 4-4 (methyl 4-(3-chloropropyl)benzoate).

A mixture of **Compound 4-4** (methyl 4-(3-chloropropyl)benzoate) and lithium hydroxide (CAS: 1310-65-2) in a mixed solution of methanol (CAS: 67-56-1) and water was stirred at room temperature for 5 hours to obtain **Compound 4-5** (4-(3-chloropropyl)benzoic acid, CAS: 90919-20-3).

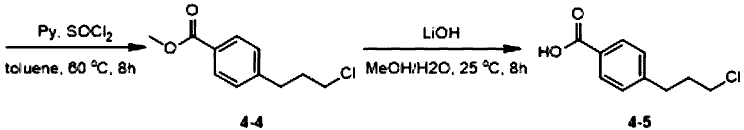

A mixture of potassium carbonate (CAS: 584-08-7) dissolved in N,N-dimethylacetamide (CAS: 127-19-5), **Intermediate B,** and **Compound 4-5** (4-(3-chloropropyl)benzoic acid, CAS: 90919-20-3) was stirred at room temperature for 6 minutes to obtain Compound of Chemical Formula (xii) **(AEV41004;** 4-(3-chloropropyl)-N-(3-(N-(3-(2,6-dioxopiperidin-3-yl)phenyl)sulfamoyl)phenyl)benzamide).

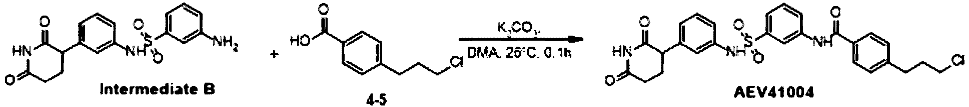

### Preparation Example 13. Preparation of Compound of Chemical Formula (xiii) (AEV41005)

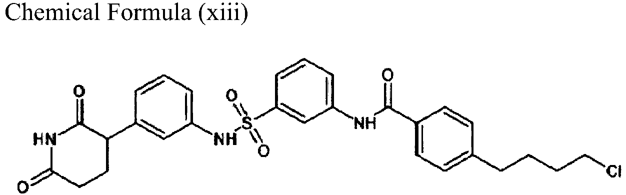

A mixture of **Compound 5-1** (methyl 4-(4-hydroxybutyl)benzoate, CAS: 123910-88-3), pyridine (CAS: 110-86-1), and thionyl chloride (CAS: 7719-09-7) in toluene (CAS: 108-88-3) was stirred at 60°C for 8 hours to obtain **Compound 5-2** (methyl 4-(4-chlorobutyl)benzoate). A mixture of **Compound 5-2** (methyl 4-(4-chlorobutyl)benzoate) and lithium hydroxide (CAS: 1310-65-2) in a mixed solution of methanol (CAS: 67-56-1) and water was stirred at room temperature for 8 hours to obtain **Compound 5-3** (4-(4-chlorobutyl)benzoic acid).

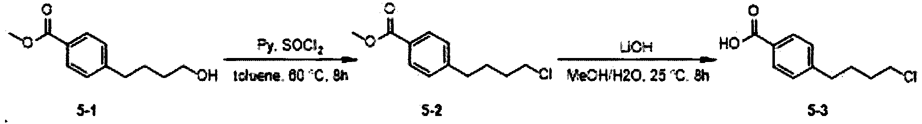

A mixture of potassium carbonate (CAS: 584-08-7) dissolved in N,N-dimethylacetamide (CAS: 127-19-5), **Intermediate B,** and **Compound 5-3** (4-(4-chlorobutyl)benzoic acid) was stirred at room temperature for 6 minutes to obtain Compound of Chemical Formula (xiii) (AEV41005; 4-(4-chlorobutyl)-N-(3-(N-(3-(2,6-dioxopiperidin-3-yl)phenyl)sulfamoyl)phenyl)benzamide).

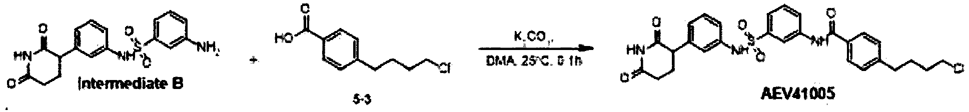

### Preparation Example 14. Preparation of Compound of Chemical Formula (xiv) (AEV41047)

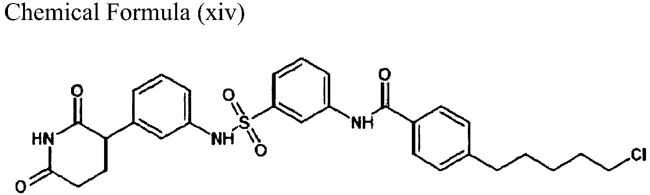

A mixture of PD(dppf)Cl₂ ([1,1'-(diphenylphosphino)ferrocene] dichloropalladium(II), CAS: 72287-26-4), copper(I) iodide (CAS: 7681-65-4), **Compound 47-1** (methyl 4-iodobenzoate, CAS: 619-44-3), and triethylamine (CAS: 121-44-8) in acetonitrile (CAS: 75-05-8) was prepared, followed by the addition of pent-4-yn-1-ol (CAS: 928-97-2). The mixture was stirred at 60°C for 12 hours to obtain **Compound 47-2** (methyl 4-(5-hydroxypent-1-yn-1-yl)benzoate).

A mixture of **Compound 47-2** (methyl 4-(5-hydroxypent-1-yn-1-yl)benzoate) and palladium on carbon (CAS: 7440-05-3) in methanol (CAS: 67-56-1) was stirred under hydrogen gas (H₂, 15 psi) at room temperature for 12 hours to obtain **Compound 47-3** (methyl 4-(5-hydroxypentyl)benzoate).

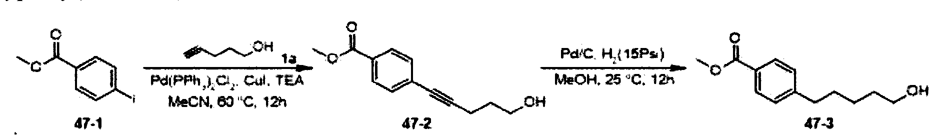

A mixture of pyridine (CAS: 110-86-1) and thionyl chloride (CAS: 7719-09-7) in toluene (CAS: 108-88-3) was prepared, and **Compound 47-3** (methyl 4-(5-hydroxypentyl)benzoate) was added. The mixture was stirred at 60°C for 8 hours to obtain **Compound 47-4** (methyl 4-(5-chloropentyl)benzoate).

A mixture of **Compound 47-4** (methyl 4-(5-chloropentyl)benzoate) and lithium hydroxide (CAS: 1310-65-2) in a mixed solution of methanol (CAS: 67-56-1) and water was stirred at room temperature for 5 hours to obtain **Compound 47-5** (4-(5-chloropentyl)benzoic acid).

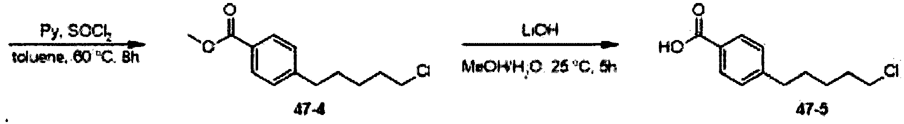

A mixture of potassium carbonate (CAS: 584-08-7) dissolved in N,N-dimethylacetamide (CAS: 127-19-5), **Intermediate B,** and **Compound 47-5** (4-(5-chloropentyl)benzoic acid) was stirred at room temperature for 6 minutes to obtain Compound of Chemical Formula (xiv) (AEV41047; 4-(5-chloropentyl)-N-(3-(N-(3-(2,6-dioxopiperidin-3-yl)phenyl)sulfamoyl)phenyl)benzamide).

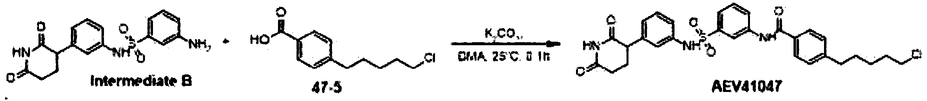

### Preparation Example 15. Preparation of Compound of Chemical Formula (xv) (AEV41048)

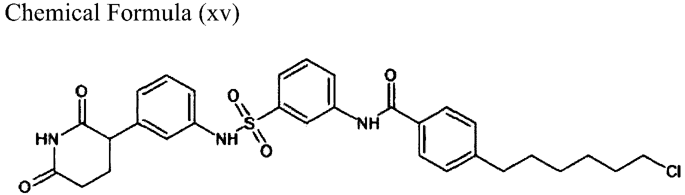

A mixture of PD(dppf)Cl₂ ([1,1'-(diphenylphosphino)ferrocene] dichloropalladium(II), CAS: 72287-26-4), copper(I) iodide (CAS: 7681-65-4), **Compound 48-1** (methyl 4-iodobenzoate, CAS: 619-44-3), and triethylamine (CAS: 121-44-8) in acetonitrile (CAS: 75-05-8) was prepared, followed by the addition of hex-5-yn-1-ol (CAS: 28916-38-3). The mixture was stirred at 60°C for 12 hours to obtain **Compound 48-2** (methyl 4-(6-hydroxyhex-1-yn-1-yl)benzoate).

A mixture of **Compound 48-2** (methyl 4-(6-hydroxyhex-1-yn-1-yl)benzoate) and palladium on carbon (CAS: 7440-05-3) in methanol (CAS: 67-56-1) was stirred under hydrogen gas (H₂, 15 psi) at room temperature for 12 hours to obtain **Compound 48-3** (methyl 4-(6-hydroxyhexyl)benzoate).

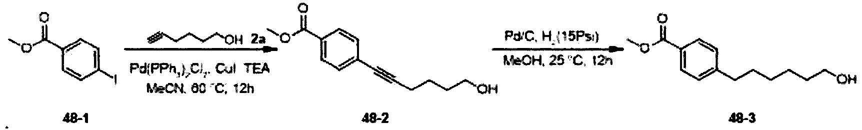

A mixture of thionyl chloride (CAS: 7719-09-7) and pyridine (CAS: 110-86-1) in toluene (CAS: 108-88-3) was prepared, and **Compound 48-3** (methyl 4-(6-hydroxyhexyl)benzoate) was added. The mixture was stirred at 60°C for 8 hours to obtain **Compound 48-4** (methyl 4-(6-chlorohexyl)benzoate).

A mixture of **Compound 48-4** (methyl 4-(6-chlorohexyl)benzoate) and lithium hydroxide (CAS: 1310-65-2) in a mixed solution of methanol (CAS: 67-56-1) and water was stirred at room temperature for 5 hours to obtain **Compound 48-5** (4-(6-chlorohexyl)benzoic acid).

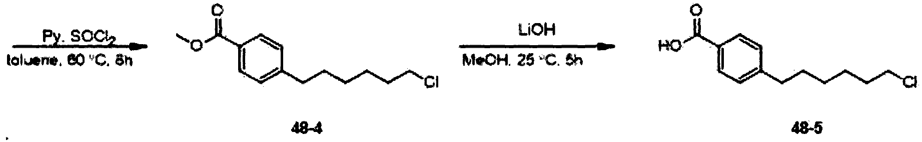

A mixture of potassium carbonate (CAS: 584-08-7) dissolved in N,N-dimethylacetamide (CAS: 127-19-5), **Intermediate B,** and **Compound 48-5** (4-(6-chlorohexyl)benzoic acid) was stirred at room temperature for 6 minutes to obtain the compound of Chemical Formula (xv) **(AEV41048;** 4-(6-chlorohexyl)-N-(3-(N-(3-(2,6-dioxopiperidin-3-yl)phenyl)sulfamoyl)phenyl)benzamide).

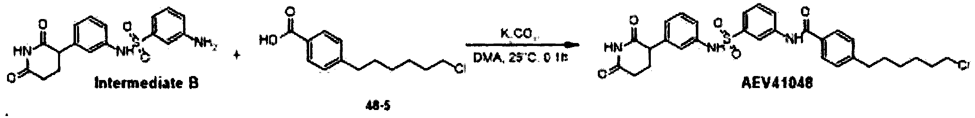

### Example 1. In Vitro CRBN Binding Assay

Thalidomide-based compounds are known to bind to cereblon (CRBN), an E3 ligase, and to regulate immune cell functions and exert various pharmacological effects by degrading transcription factors Aiolos and Ikaros as their substrates. Accordingly, the cereblon binding affinities of various compounds were compared in vitro.

The binding affinity to cereblon was measured using a fluorescence resonance energy transfer (FRET)-based AlphaScreen method, and the assay was performed according to the "Competitive Inhibition of the PROTAC Assay" described in the manual of the PROTAC Optimization Kit for BET Bromodomain-Cereblon Binding (#79770, BPS Bioscience, CA, USA). All reagents included in the kit were used, except for the candidate compounds, DMSO, and Flag/Glutathione beads (#6765300, PerkinElmer, USA).

As a result, compounds exhibiting cereblon-binding activity as shown in Table 1 were identified.

**[Table 1]**

| **Compound no.** | **MW** | **Structure** | **Alpha IC50 (µM)** |
|---|---|---|---|
| AEV40004 | 207.2 | 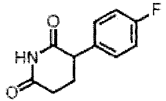 | 4.43 |
| AEV40016 | 246.27 | 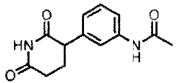 | 5.28 |
| AEV40024 | 274.32 | 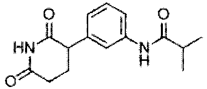 | 6.11 |
| AEV40025 | 344.39 | 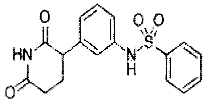 | 1.86 |
| AEV40029 | 204.23 | 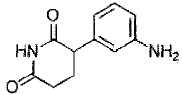 | 2.96 |
| AEV40030 | 282.31 | 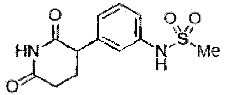 | 7.06 |
| AEV40031 | 276.29 | 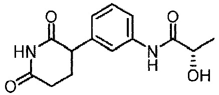 | 3.86 |
| AEV40032 | 244.25 | 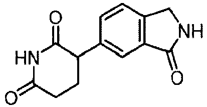 | 3.10 |
| AEV40034 | 264.26 | 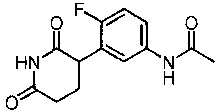 | 6.26 |
| AEV40035 | 292.31 | 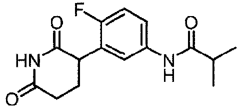 | 3.11 |
| AEV40036 | 394.43 | 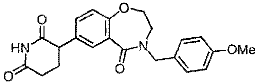 | 1.13 |
| AEV40037 | 378.83 | 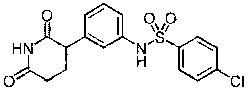 | 0.86 |
| AEV40038 | 396.82 | 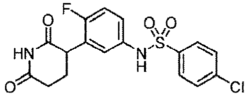 | 0.69 |
| AEV40039 | 203.24 | 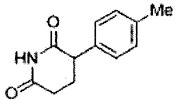 | 0.87 |
| AEV40040 | 244.25 | 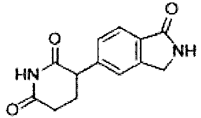 | 1.39 |
| AEV40041 | 258.28 | 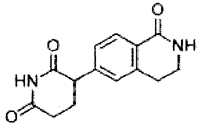 | 0.76 |
| AEV40042 | 302.33 | 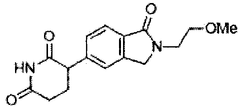 | 5.40 |
| AEV40043 | 392.85 | 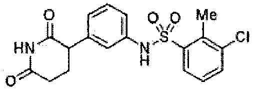 | 0.91 |
| AEV40044 | 396.82 | 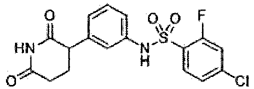 | 0.23 |
| AEV40045 | 362.38 | 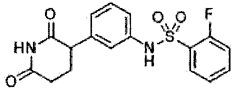 | 0.30 |
| AEV40046 | 372.44 | 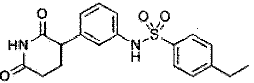 | 1.00 |
| AEV40047 | 386.47 | 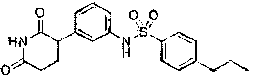 | 1.50 |
| AEV40048 | 394.45 | 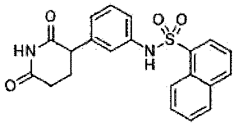 | 0.79 |
| AEV40049 | 348.4 | 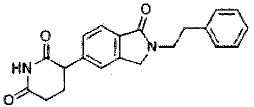 | 2.06 |
| AEV40050 | 258.28 | 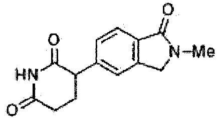 | 2.68 |
| AEV40051 | 348.38 | 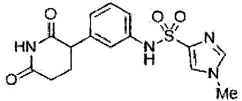 | 0.79 |
| AEV40052 | 376.4 | 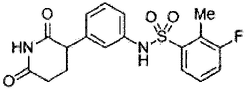 | 0.95 |
| AEV40053 | 457.38 | 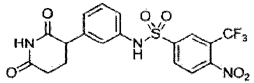 | 0.75 |
| AEV40054 | 413.27 | 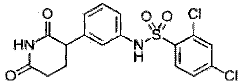 | 0.20 |
| AEV40055 | 389.38 | 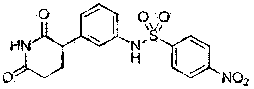 | 0.20 |
| AEV40056 | 389.38 | 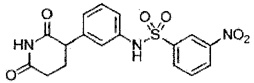 | 0.91 |
| AEV40057 | 378.83 | 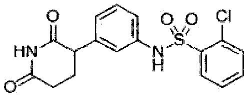 | 0.59 |
| AEV40058 | 413.27 | 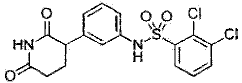 | 0.68 |
| AEV40059 | 322.34 | 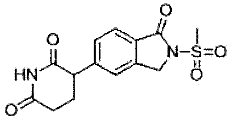 | 102.00 |
| AEV40060 | 403.41 | 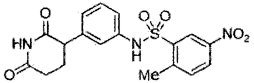 | 1.52 |
| AEV40061 | 389.38 | 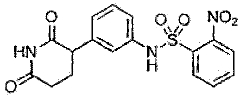 | 0.59 |
| AEV40062 | 244.25 | 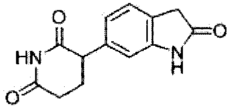 | 2.39 |
| AEV40063 | 378.83 | 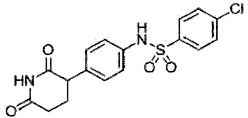 | 1.43 |
| AEV40069 | 358.41 | 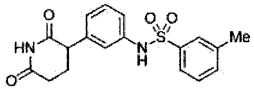 | 0.71 |
| AEV40070 | 412.38 | 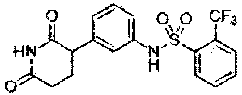 | 1.05 |
| AEV40071 | 412.42 | 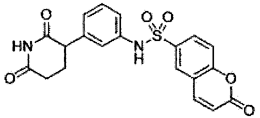 | 0.22 |
| AEV40072 | 350.41 | 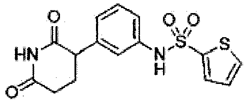 | 0.31 |
| AEV40073 | 359.40 | 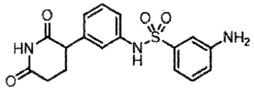 | 0.59 |
| AEV40074 | 359.40 | 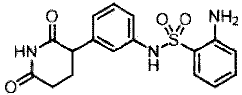 | 0.27 |
| AEV40075 | 427.40 | 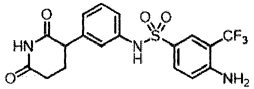 | 1.12 |
| AEV40076 | 377.22 | 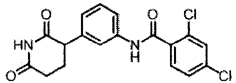 | 1.35 |
| AEV40077 | 418.85 | 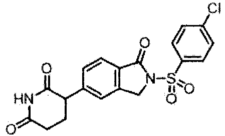 | 55.29 |
| AEV40078 | 258.28 | 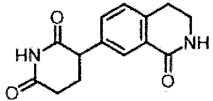 | 3.11 |
| AEV40079 | 274.28 | 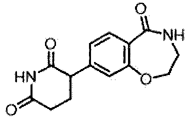 | 2.44 |
| AEV40085 | 350.37 | 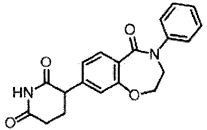 | 6.58 |
| AEV40086 | 364.40 | 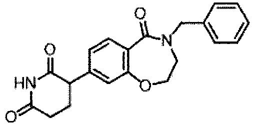 | 4.14 |
| AEV40087 | 294.35 | 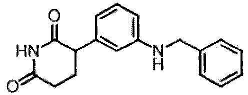 | 1.15 |
| AEV40088 | 308.34 | 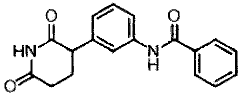 | 2.19 |
| AEV40089 | 205.21 | 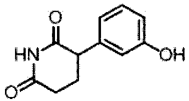 | 1.14 |
| AEV40090 | 328.80 | 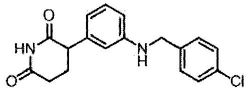 | 2.14 |
| AEV40092 | 314.40 | 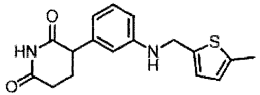 | 0.97 |
| AEV40094 | 322.41 | 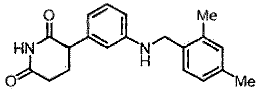 | 0.81 |
| AEV40095 | 326.37 | 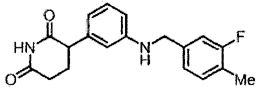 | 1.58 |
| AEV40096 | 358.82 | 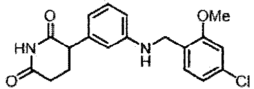 | 0.99 |
| AEV40097 | 328.34 | 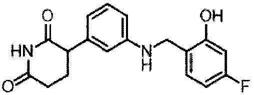 | 0.99 |
| AEV40098 | 350.46 | 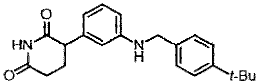 | 2.85 |
| AEV40099 | 358.41 | 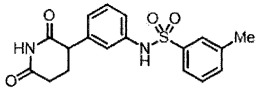 | 1.29 |
| AEV40100 | 412.38 | 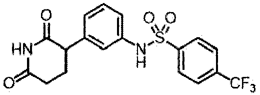 | 1.39 |
| AEV40101 | 362.38 | 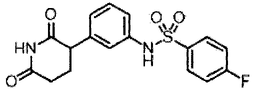 | 1.30 |
| AEV40102 | 308.38 | 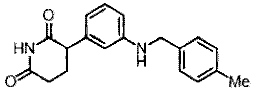 | 1.24 |
| AEV40103 | 386.45 | 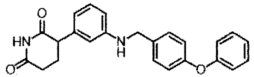 | 3.13 |
| AEV40104 | 362.35 | 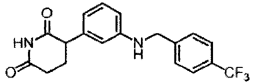 | 1.72 |
| AEV40105 | 344.36 | 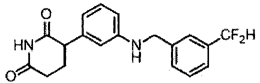 | 1.42 |
| AEV40106 | 308.38 | 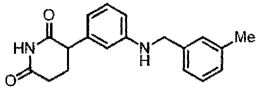 | 1.14 |
| AEV40107 | 312.34 | 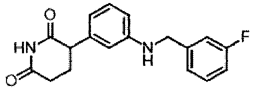 | 1.49 |
| AEV40108 | 338.41 | 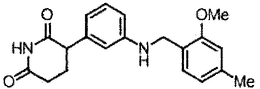 | 0.84 |
| AEV40109 | 407.69 | 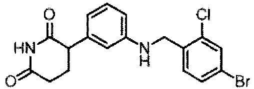 | 2.52 |
| AEV40110 | 348.33 | 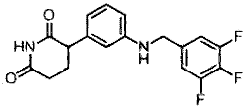 | 1.96 |
| AEV40113 | 312.37 | 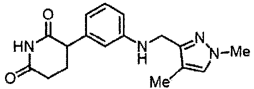 | 0.66 |
| AEV40120 | 344.41 | 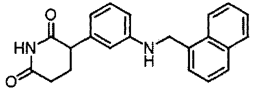 | 1.41 |
| AEV40121 | 386.45 | 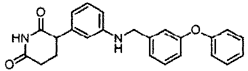 | 3.04 |
| AEV40122 | 370.45 | 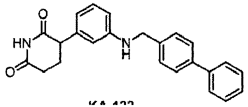 | 2.47 |
| AEV40129 | 323.352 | 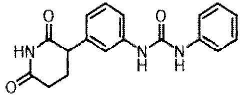 | 0.37 |
| AEV40130 | 318.373 | 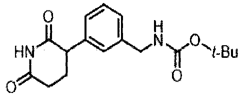 | 4.33 |
| AEV40131 | 357.794 | 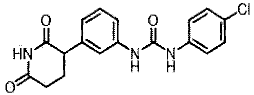 | 0.19 |
| AEV40132 | 218.256 | 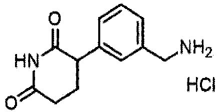 | >10 |
| AEV40133 | 391.3502 | 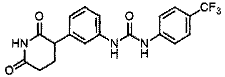 | 1.26 |
| AEV40134 | 377.221 | 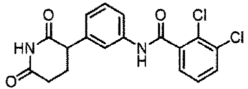 | 1.13 |
| AEV40135 | 497.75 | 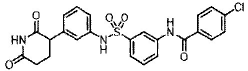 | 1.20 |
| AEV40136 | 497.95 | 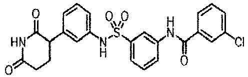 | 0.79 |
| AEV40137 | 377.221 | 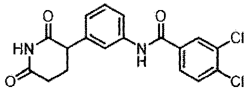 | 2.22 |
| AEV40138 | 411.663 | 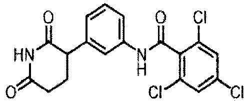 | 0.80 |
| AEV40139 | 342.779 | 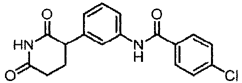 | 2.12 |
| AEV40140 | 326.327 | 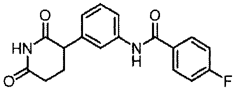 | 1.83 |
| AEV40141 | 322.364 | 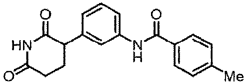 | 2.08 |
| AEV40142 | 532.392 | 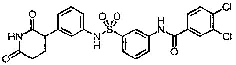 | 0.98 |
| AEV40143 | 463.508 | 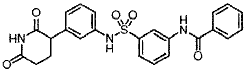 | 0.61 |
| AEV40144 | 342.779 | 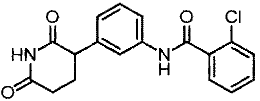 | 0.96 |
| AEV40145 | 326.327 | 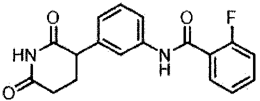 | 1.03 |
| AEV40146 | 322.364 | 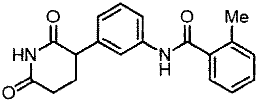 | 1.10 |
| AEV40147 | 356.806 | 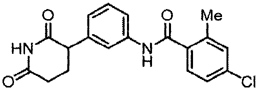 | 1.37 |
| AEV40148 | 372.805 | 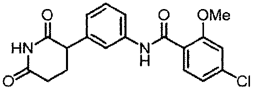 | 1.12 |
| AEV40149 | 549.497 | 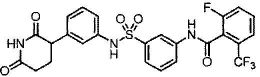 | 1.50 |
| AEV40150 | 532.392 | 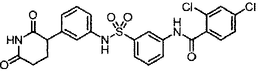 | 1.82 |
| AEV40151 | 493.534 | 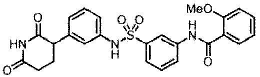 | 0.34 |
| AEV40152 | 531.506 | 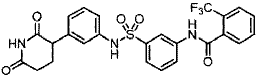 | 0.72 |
| AEV40153 | 517.479 | 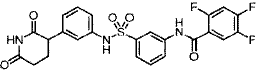 | 0.80 |
| AEV41012 | 519.616 | 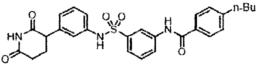 | 0.94 |
| AEV40155 | 542.404 | 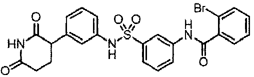 | 0.95 |
| AEV40156 | 499.489 | 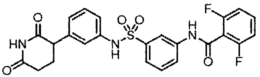 | 1.25 |
| AEV40157 | 542.404 | 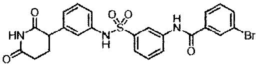 | 0.73 |
| AEV40158 | 517.479 | 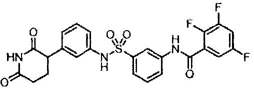 | 0.71 |
| AEV40159 | 550.382 | 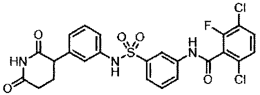 | 1.49 |
| AEV40160 | 301.342 | 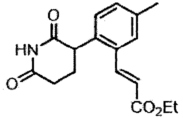 | 2.92 |
| AEV40161 | 384.435 | 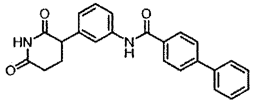 | >10 |
| AEV40162 | 384.435 | 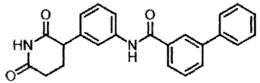 | 2.15 |
| AEV40163 | 488.562 | 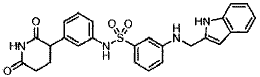 | 1.43 |
| AEV40164 | 344.318 | 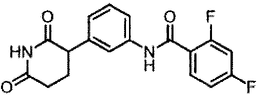 | 1.67 |
| AEV40165 | 336.391 | 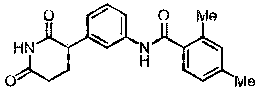 | 0.93 |
| AEV40166 | 344.318 | 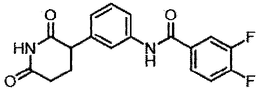 | 2.52 |
| AEV40171 | 488.562 | 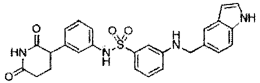 | 1.28 |
| AEV40172 | 490.534 | 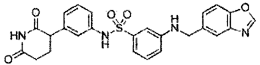 | 0.21 |
| AEV40173 | 523.004 | 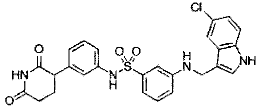 | 0.40 |
| AEV40176 | 372.805 | 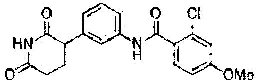 | 1.24 |
| AEV40178 | 377.221 | 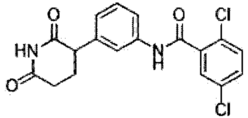 | 0.92 |
| AEV40179 | 389.233 | 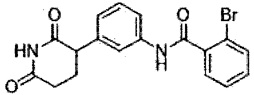 | 0.64 |
| AEV40180 | 376.335 | 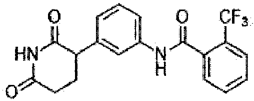 | 1.13 |
| AEV40181 | 526.992 | 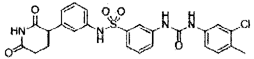 | 0.32 |
| AEV40182 | 348.801 | 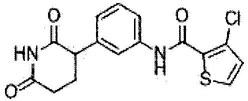 | 0.88 |
| AEV40183 | 332.740 | 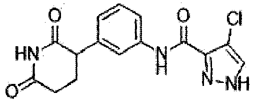 | 1.05 |
| AEV40184 | 366.42 | 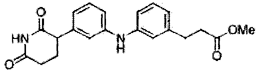 | 1.41 |
| AEV40185 | 475.97 | 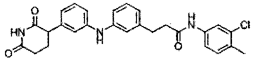 | 4.35 |
| AEV40186 | 409.49 | 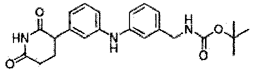 | 2.63 |
| AEV40187 | 359.39 | 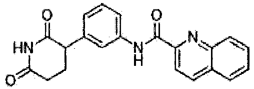 | 0.83 |
| AEV40188 | 346.77 | 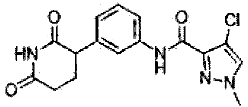 | 1.00 |
| AEV40189 | 348.8 | 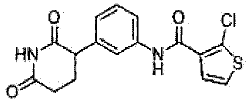 | 1.63 |
| AEV40190 | 347.37 | 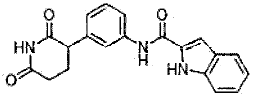 | 2.14 |
| AEV40191 | 347.37 | 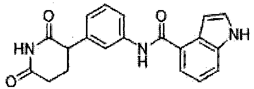 | 0.48 |
| AEV40192 | 476.96 | 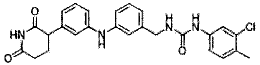 | 3.22 |
| AEV40193 | 521.59 | 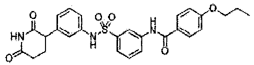 | 0.86 |
| AEV40194 | 347.37 | 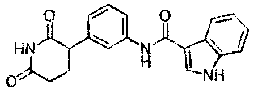 | 0.85 |
| AEV40196 | 525.65 | 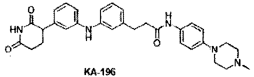 | 1.23 |
| AEV40199 | 527.66 | 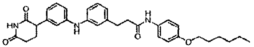 | 3.47 |
| AEV40200 | 476.58 | 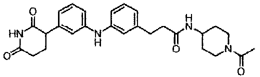 | 0.96 |
| AEV40201 | 493.57 | 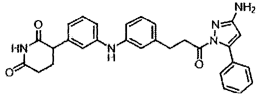 | 1.22 |
| AEV40202 | 467.53 | 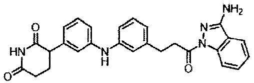 | 0.79 |
| AEV40203 | 230.22 | 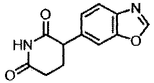 | 1.80 |
| AEV40204 | 561.7 | 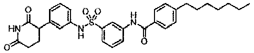 | 0.44 |
| AEV40205 | 469.56 | 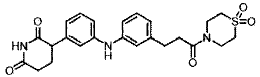 | 0.84 |
| AEV40206 | 365.39 | 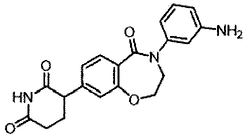 | 3.69 |
| AEV40207 | 315.33 | 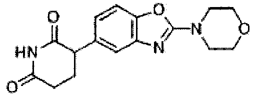 | 4.57 |
| AEV40208 | 313.36 | 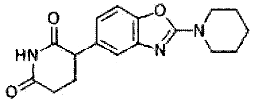 | 3.54 |
| AEV40209 | 493.57 | 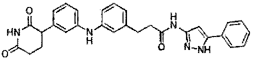 | 0.91 |
| AEV40210 | 503.6 | 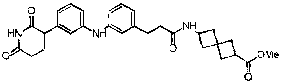 | 1.22 |
| AEV40211 | 489.57 | 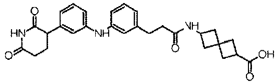 | >100 |
| AEV40212 | 447.49 | 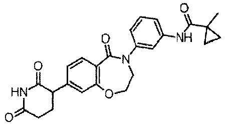 | 2.30 |
| AEV40213 | 489.57 | 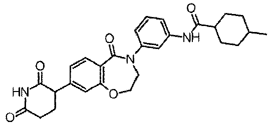 | 2.11 |
| AEV40214 | 525.61 | 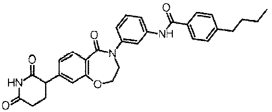 | 0.82 |
| AEV40215 | 570.58 | 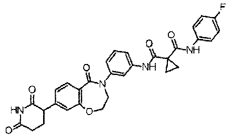 | 2.16 |
| AEV40216 | 477.52 | 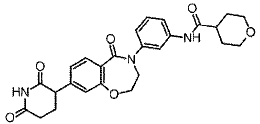 | 2.51 |
| AEV40217 | 538.38 | 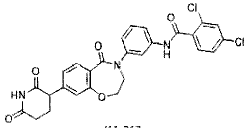 | 1.18 |
| AEV40218 | 384.44 | 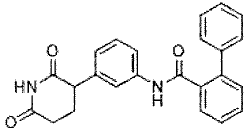 | 0.44 |
| AEV40219 | 500.53 | 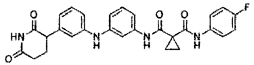 | 2.21 |
| AEV40220 | 468.33 | 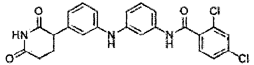 | 2.11 |
| AEV40221 | 455.56 | 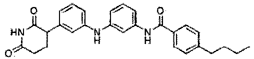 | 1.65 |
| AEV40222 | 418.88 | 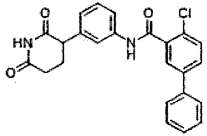 | 1.72 |
| AEV40223 | 421.68 | 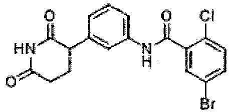 | 1.38 |
| AEV40224 | 483.58 | 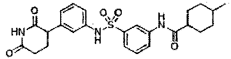 | 2.13 |
| AEV40225 | 471.53 | 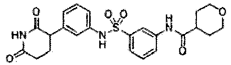 | 12.1 |
| AEV40226 | 402.42 | 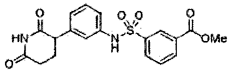 | 2.22 |
| AEV40227 | 441.5 | 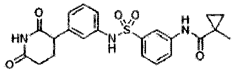 | 0.98 |
| AEV40204 | 561.7 | 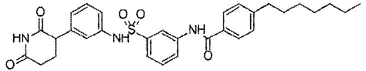 | 0.44 |
| AEV40264 | 575.72 | 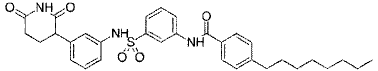 | 0.32 |
| AEV41 001 | 498 | 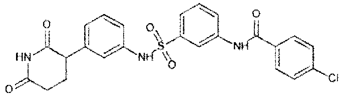 | 1.23 |
| AEV41002 | 512 | 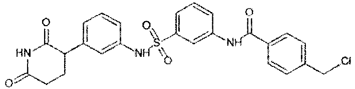 | 0.17 |
| AEV41003 | 526 | 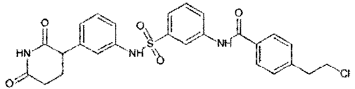 | 0.77 |
| AEV41004 | 540 | 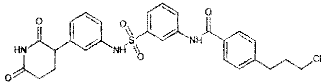 | 1.57 |
| AEV41005 | 554.1 | 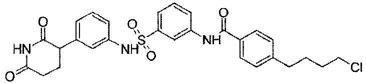 | 1.2 |
| AEV41009 | 477.5 | 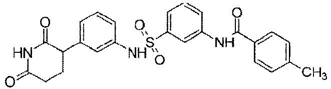 | 0.54 |
| AEV41010 | 491.6 | 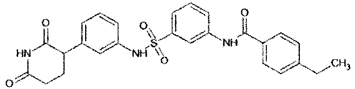 | 0.42 |
| AEV41011 | 505.6 | 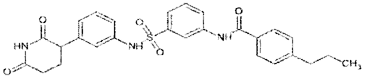 | 0.7 |
| AEV41013 | 533.6 | 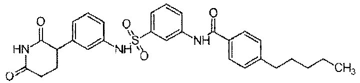 | 0.36 |
| AEV41047 | 568.1 | 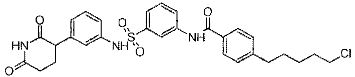 | 1.88 |
| AEV41048 | 582.1 | 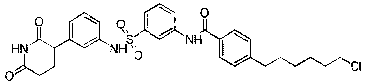 | 2.59 |
| AEV41083 | 477.7 | 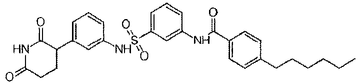 | 0.91 |

### Example 2. Tumor Cell Apoptosis Effect

To examine whether the compounds having CRBN-binding activity according to the present invention exert tumor cell apoptosis effects, the assay was conducted using various human cancer cell lines, including human multiple myeloma cell lines (MM.1S and MM.1R-LR), a human breast cancer cell line (MDA-MB-231), a human lung cancer cell line (A549), a human rhabdomyosarcoma cell line (RD), and a human lymphoma cell line (Jeko-1).

The MM.1S, MM.1R-LR, MDA-MB-231, A549, RD, and Jeko-1 cell lines were obtained from the American Type Culture Collection (ATCC, Manassas, VA, USA) and cultured in RPMI 1640 medium (Corning, MD, USA) or DMEM (Corning, MD, USA) supplemented with 10% fetal bovine serum (FBS) (Corning, MD, USA), 100 U/mL penicillin (Corning, MD, USA), and 100 µg/mL streptomycin (Corning, MD, USA) at 37°C in a 5% CO₂ incubator for 24 hours. The cells were seeded at a density of 1 × 10⁴ cells/well in 96-well plates and incubated for 24 hours. After incubation, the test compounds dissolved in dimethyl sulfoxide (DMSO, CAS No.: 67-68-5) were serially diluted to a final concentration of 10 µM and applied to the cells. Following an additional incubation for 2 to 5 days, the analysis was performed using a CCK-8 assay kit (Dojindo, Japan) according to the manufacturer's instructions.

**[Table 2]**

| Cell line | Source | Culture medium | Compound treatment period |
|---|---|---|---|
| MM.1S | ATCC | RPMI | 5 days |
| MM.1R-LR | in house (drug-resistant cell line established in-house after purchasing MM.1R from ATCC) | RPMI | 5 days |
| MDA-MB-231 | KCLB | DMEM | 2 days |
| A549 | ATCC | RPMI | 2 days |
| RD | ATCC | DMEM | 3 days |
| Jeko-1 | ATCC | RPMI | 3 days |

As a result, as shown in Table 3, the compounds exhibiting CRBN-binding activity were found to induce apoptosis in the tumor cell lines.

**[Table 3]**

| | CRBN binding affinity (Alpha assay) IC50(µM) | Cell viability (% of control) Compound treatment: 1uM | | | | | |
|---|---|---|---|---|---|---|---|
| | | MM.1S | MM.1R-LR | MDA-MB-231 | A549 | RD | Jeko-1 |
| Chemical Formula (i) | 0.54 | 92.9 | 81.3 | - | - | - | - |
| Chemical Formula (ii) | 0.42 | 88.4 | 66.1 | 95.7 | 96.7 | - | - |
| Chemical Formula(iii) | 0.70 | 56.9 | 66.1 | 96.7 | 98.9 | - | - |
| Chemical Formula (iv) | 0.94 | 7.4 | 56.7 | 78.7 | 94.0 | 79.7 | 37.7 |
| Chemical Formula (v) | 0.36 | 15.3 | 55.2 | 38.5 | 25.8 | 30.0 | 11.8 |
| Chemical Formula (vi) | 0.91 | 17.8 | 54.7 | 35.5 | 29.5 | 31.4 | 9.7 |
| Chemical Formula (vii) | 0.44 | 3.3 | 49.4 | 40.4 | 33.6 | 50.8 | 11.6 |
| Chemical Formula(viii) | 0.32 | 13.3 | 79.8 | 46.3 | 61.8 | 67.9 | 5.8 |
| Chemical Formula (ix) | 1.23 | 88.3 | 78.4 | - | - | - | - |
| Chemical Formula (x) | 0.17 | 97.8 | 90.2 | - | - | - | - |
| Chemical Formula (xi) | 0.77 | 92.1 | 79.9 | 97.0 | 104.0 | - | - |
| Chemical Formula (xii) | 1.57 | 10.5 | 63.8 | 83.5 | 101.9 | 78.6 | - |
| Chemical Formula(xiii) | 1.20 | 10.7 | 58.2 | 42.2 | 36.8 | 28.7 | 4.1 |
| Chemical Formula (xiv) | 1.88 | 14.9 | 56.9 | 28.2 | 32.2 | 20.5 | 15.7 |
| Chemical Formula (xv) | 2.59 | 10.8 | 51.5 | 45.6 | 47.0 | 54.0 | 13.0 |

## Claims

1. A compound represented by the following Chemical Formula (I), or an optical isomer thereof, a racemic mixture thereof, a hydrate thereof, a solvate thereof, or a pharmaceutically acceptable salt thereof: 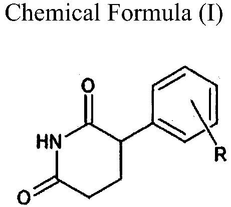 wherein, in Chemical Formula (I),
(i) R is independently hydrogen (H), halogen (X), hydroxyl (OH), amino (NH₂), alkyl, alkylamine, aminocarbonylalkyl, aminocarbonylaryl, aminosulfonylalkyl, aminocarbonylalcohol, aminosulfonylaryl, aminosulfonylalkyl, aminoalkylaryl, aminocarbonylaminoaryl, alkylaminocarbonylalkoxy, aminosulfonylaminocarbonylaminoaryl, aminoarylalkylcarboxylalkyl, aminoarylalkylcarbonylaminoaryl, aminoarylalkylaminocarboxylalkyl, aminoarylalkylaminocarbonylaminoaryl, aminosulfonylarylaminocarbonylarylalkoxy, aminoarylalkylcarbonylaminoarylalkoxy, aminoarylalkylcarbonylaminoalkylcarboxylalkyl, aminoarylalkylcarbonylaminoalkylcarboxylic acid, aminoarylaminocarbonylalkylcarbonylaminoaryl, aminoarylaminocarbonylaryl, or aminosulfonylarylaminocarbonylaryl; and
(ii) R is a heterocycloalkyl group including a CONH moiety, wherein the heterocycloalkyl forms a bicyclic fused ring with the benzene ring, the hydrogen (H) bound to the nitrogen (N) of the CONH group is unsubstituted or substituted, and the carbon (C) of the heterocycloalkyl is unsubstituted or substituted with oxygen (O),
and wherein any benzene ring present in Chemical Formula (I) is an unsubstituted benzene ring or a benzene ring having one or more hydrogen atoms substituted.

2. The compound, an optical isomer thereof, a racemic mixture thereof, a hydrate thereof, a solvate thereof, or a pharmaceutically acceptable salt thereof according to claim 1,
wherein the compound represented by Chemical Formula (I) is represented by the following Chemical Formula (II): 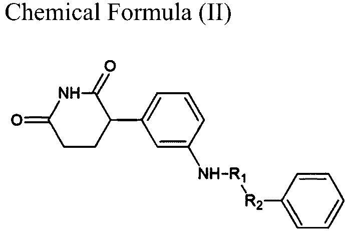 wherein, in Chemical Formula (II),
R₁ is absent, CH₂, CO, or SO₂;
R₂ is absent or C₆H₄NHCO; and
any benzene ring present in Chemical Formula (II) is an unsubstituted benzene ring or a benzene ring having one or more hydrogen atoms substituted.

3. The compound, an optical isomer thereof, a racemic mixture thereof, a hydrate thereof, a solvate thereof, or a pharmaceutically acceptable salt thereof according to claim 2,
wherein the compound represented by Chemical Formula (II) is represented by one selected from the group consisting of the following Chemical Formulae (II-1) to (II-5): 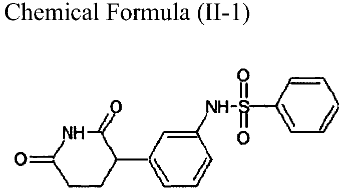
wherein any benzene ring present in Chemical Formula (II-1) is an unsubstituted benzene ring or a benzene ring having one or more hydrogen atoms substituted; 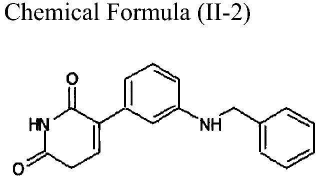
wherein any benzene ring present in Chemical Formula (11-2) is an unsubstituted benzene ring or a benzene ring having one or more hydrogen atoms substituted; 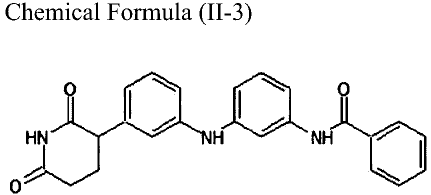
wherein any benzene ring present in Chemical Formula (II-3) is an unsubstituted benzene ring or a benzene ring having one or more hydrogen atoms substituted; 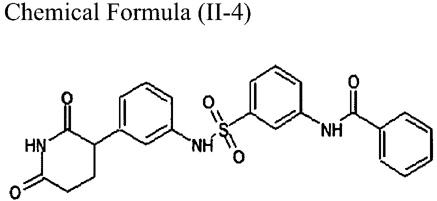
wherein any benzene ring present in Chemical Formula (II-4) is an unsubstituted benzene ring or a benzene ring having one or more hydrogen atoms substituted; 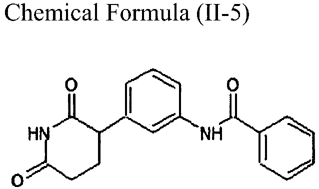
wherein any benzene ring present in Chemical Formula (11-5) is an unsubstituted benzene ring or a benzene ring having one or more hydrogen atoms substituted.

4. The compound, an optical isomer thereof, a racemic mixture thereof, a hydrate thereof, a solvate thereof, or a pharmaceutically acceptable salt thereof according to claim 1, wherein any benzene ring present in Chemical Formula (I) has one or more hydrogen atoms each independently substituted with alkyl, cycloalkyl, aryl, heterocyclyl, cycloalkylalkyl, aralkyl, heterocyclylalkyl, heterocycloalkylalkyl, halogen, hydroxyalkyl, alkoxy, alkoxyalkyl, amino, amido, urea, sulfonyl, hydroxy, aldehyde, carboxy, haloalkyl, alkoxy, cycloalkyloxy, aryloxy, heterocyclyloxy, heterocycloalkyloxy, cycloalkylalkyloxy, aralkyloxy, heterocyclylalkyloxy, heterocycloalkylalkyloxy; oxo (=O); amino, alkylamino, cycloalkylamino, arylamino, heterocyclylamino, heterocycloalkylamino, cycloalkylalkylamino, aralkylamino, heterocyclylalkylamino, heterocycloalkylalkylamino; imino; imido; amidino; guanidino; enamino; acylamino; sulfonylamino; urea, nitrourea; oxime; hydroxylamino; alkoxyamino; aralkoxyamino; hydrazino; hydrazido; hydrazono; azido; nitro; thio (-SH), alkylthio; =S; sulfinyl; sulfonyl; aminosulfonyl; phosphonate; phosphinyl; acyl; formyl; carboxy; ester; carbamate; amido; cyano; isocyanato; isothiocyanato; cyanato; thiocyanato; hydroxyl; alkoxy; alkoxyalkyl; amino; alkylamino; carboxy; nitro; cyano; thiol; thioether; imine; imide; amidine; guanidine; enamine; aminocarbonyl; acylamino; phosphonate; phosphine; thiocarbonyl; sulfinyl; sulfone; sulfonamide; ketone; aldehyde; ester; urea; urethane; oxime; hydroxylamine; alkoxyamine; aralkoxyamine; N-oxide; hydrazine; hydrazide; hydrazone; azide; isocyanate; isothiocyanate; cyanate; thiocyanate; B(OH)₂; or O(alkyl)aminocarbonyl.

5. The compound, an optical isomer thereof, a racemic mixture thereof, a hydrate thereof, a solvate thereof, or a pharmaceutically acceptable salt thereof according to claim 3, wherein any benzene ring present in any one of Chemical Formulas (II-1) to (II-5) has one or more hydrogen atoms each independently substituted with any one selected from the group consisting of -F, -Cl, -Br, -I, (C₁-C₁₀) alkyl group, amino group (-NH₂), nitro group (-NO₂), -CF₃, -CF₂H, -CFH₂, -CCl₂, - CCl₂H, -CClH₂, -(CH₂)ₙCl, (C₁-C₁₀) alkoxy group, hydroxyl group (-OH), (C₁-C₁₀) alkoxyaryl group, and C₆-C₃₀ aryl group.

6. The compound, an optical isomer thereof, a racemic mixture thereof, a hydrate thereof, a solvate thereof, or a pharmaceutically acceptable salt thereof according to claim 3, wherein any benzene ring present in Chemical Formula (II-4) has one or more hydrogen atoms each independently substituted with halogen, C₁₋₁₀ alkyl, or C₁₋₁₀ haloalkyl.

7. The compound, an optical isomer thereof, a racemic mixture thereof, a hydrate thereof, a solvate thereof, or a pharmaceutically acceptable salt thereof according to claim 1,
wherein the compound is represented by the following Chemical Formula (III): 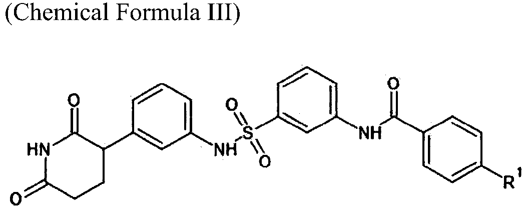 wherein, in Chemical Formula (III),
R¹ is a halogen (X), C₁₋₁₀ alkyl, or C₁₋₁₀ haloalkyl.

8. The compound, an optical isomer thereof, a racemic mixture thereof, a hydrate thereof, a solvate thereof, or a pharmaceutically acceptable salt thereof according to claim 1, wherein the compound, the optical isomer thereof, the racemic mixture thereof, the hydrate thereof, the solvate thereof, or the pharmaceutically acceptable salt thereof has cereblon-binding affinity.

9. The compound, an optical isomer thereof, a racemic mixture thereof, a hydrate thereof, a solvate thereof, or a pharmaceutically acceptable salt thereof according to claim 1, wherein the compound, the optical isomer thereof, the racemic mixture thereof, the hydrate thereof, the solvate thereof, or the pharmaceutically acceptable salt thereof is any one of the compounds shown in Table 1 below:
**[Table 1]**
| **Compound no.** | **MW** | **Structure** | **Alpha IC50 (µM)** |
|---|---|---|---|
| AEV40004 | 207.2 | 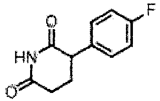 | 4.43 |
| AEV40016 | 246.27 | 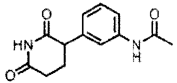 | 5.28 |
| AEV40024 | 274.32 | 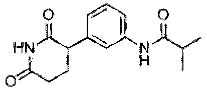 | 6.11 |
| AEV40025 | 344.39 | 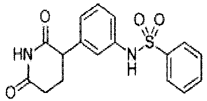 | 1.86 |
| AEV40029 | 204.23 | 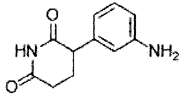 | 2.96 |
| AEV40030 | 282.31 | 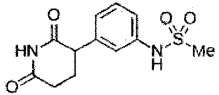 | 7.06 |
| AEV40031 | 276.29 | 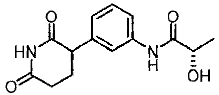 | 3.86 |
| AEV40032 | 244.25 | 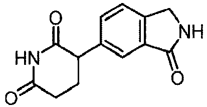 | 3.10 |
| AEV40034 | 264.26 | 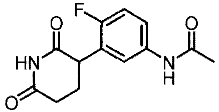 | 6.26 |
| AEV40035 | 292.31 | 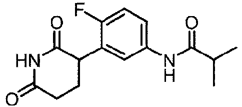 | 3.11 |
| AEV40036 | 394.43 | 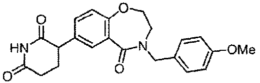 | 1.13 |
| AEV40037 | 378.83 | 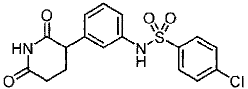 | 0.86 |
| AEV40038 | 396.82 | 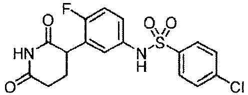 | 0.69 |
| AEV40039 | 203.24 | 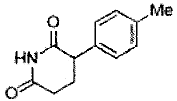 | 0.87 |
| AEV40040 | 244.25 | 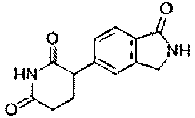 | 1.39 |
| AEV40041 | 258.28 | 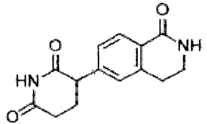 | 0.76 |
| AEV40042 | 302.33 | 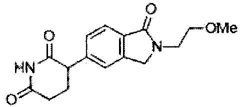 | 5.40 |
| AEV40043 | 392.85 | 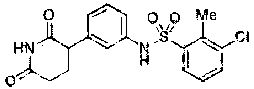 | 0.91 |
| AEV40044 | 396.82 | 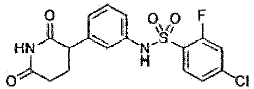 | 0.23 |
| AEV40045 | 362.38 | 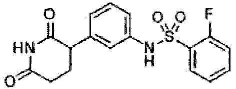 | 0.30 |
| AEV40046 | 372.44 | 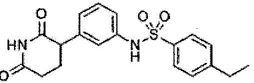 | 1.00 |
| AEV40047 | 386.47 | 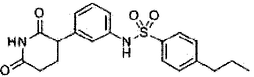 | 1.50 |
| AEV40048 | 394.45 | 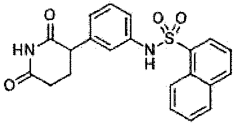 | 0.79 |
| AEV40049 | 348.4 | 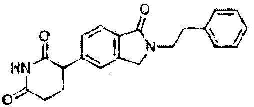 | 2.06 |
| AEV40050 | 258.28 | 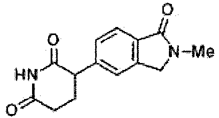 | 2.68 |
| AEV40051 | 348.38 | 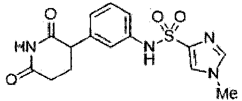 | 0.79 |
| AEV40052 | 376.4 | 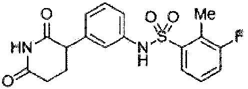 | 0.95 |
| AEV40053 | 457.38 | 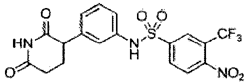 | 0.75 |
| AEV40054 | 413.27 | 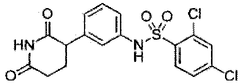 | 0.20 |
| AEV40055 | 389.38 | 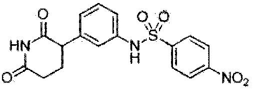 | 0.20 |
| AEV40056 | 389.38 | 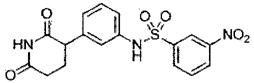 | 0.91 |
| AEV40057 | 378.83 | 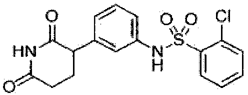 | 0.59 |
| AEV40058 | 413.27 | 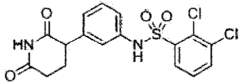 | 0.68 |
| AEV40059 | 322.34 | 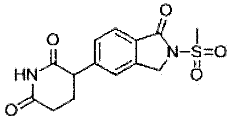 | 102.00 |
| AEV40060 | 403.41 | 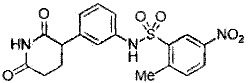 | 1.52 |
| AEV40061 | 389.38 | 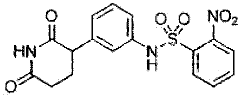 | 0.59 |
| AEV40062 | 244.25 | 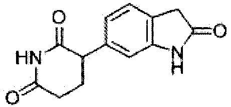 | 2.39 |
| AEV40063 | 378.83 | 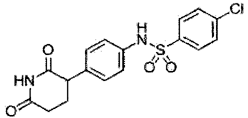 | 1.43 |
| AEV40069 | 358.41 | 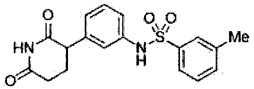 | 0.71 |
| AEV40070 | 412.38 | 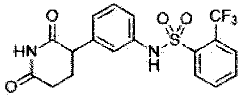 | 1.05 |
| AEV40071 | 412.42 | 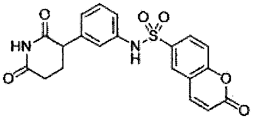 | 0.22 |
| AEV40072 | 350.41 | 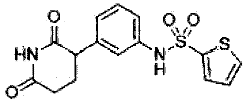 | 0.31 |
| AEV40073 | 359.40 | 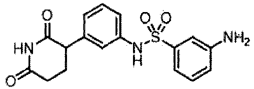 | 0.59 |
| AEV40074 | 359.40 | 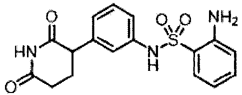 | 0.27 |
| AEV40075 | 427.40 | 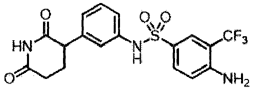 | 1.12 |
| AEV40076 | 377.22 | 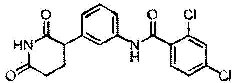 | 1.35 |
| AEV40077 | 418.85 | 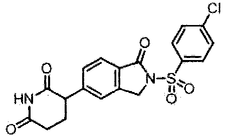 | 55.29 |
| AEV40078 | 258.28 | 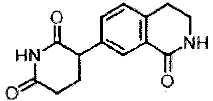 | 3.11 |
| AEV40079 | 274.28 | 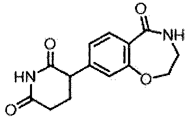 | 2.44 |
| AEV40085 | 350.37 | 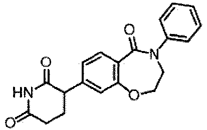 | 6.58 |
| AEV40086 | 364.40 | 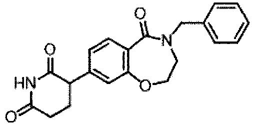 | 4.14 |
| AEV40087 | 294.35 | 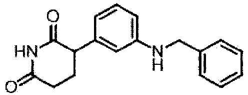 | 1.15 |
| AEV40088 | 308.34 | 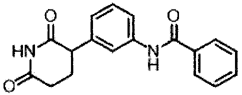 | 2.19 |
| AEV40089 | 205.21 | 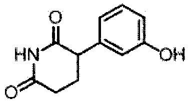 | 1.14 |
| AEV40090 | 328.80 | 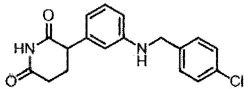 | 2.14 |
| AEV40092 | 314.40 | 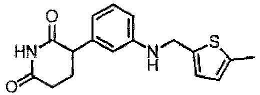 | 0.97 |
| AEV40094 | 322.41 | 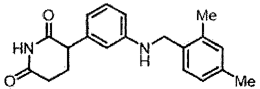 | 0.81 |
| AEV40095 | 326.37 | 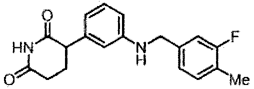 | 1.58 |
| AEV40096 | 358.82 | 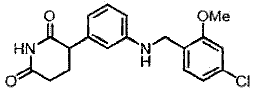 | 0.99 |
| AEV40097 | 328.34 | 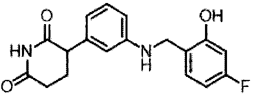 | 0.99 |
| AEV40098 | 350.46 | 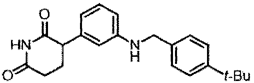 | 2.85 |
| AEV40099 | 358.41 | 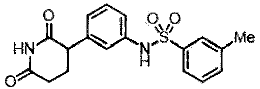 | 1.29 |
| AEV40100 | 412.38 | 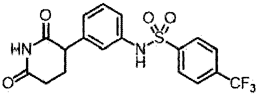 | 1.39 |
| AEV40101 | 362.38 | 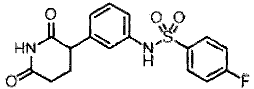 | 1.30 |
| AEV40102 | 308.38 | 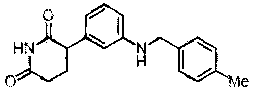 | 1.24 |
| AEV40103 | 386.45 | 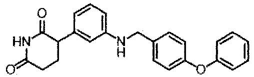 | 3.13 |
| AEV40104 | 362.35 | 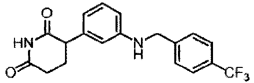 | 1.72 |
| AEV40105 | 344.36 | 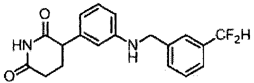 | 1.42 |
| AEV40106 | 308.38 | 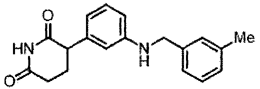 | 1.14 |
| AEV40107 | 312.34 | 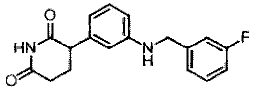 | 1.49 |
| AEV40108 | 338.41 | 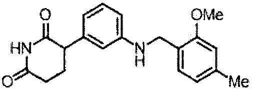 | 0.84 |
| AEV40109 | 407.69 | 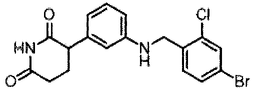 | 2.52 |
| AEV40110 | 348.33 | 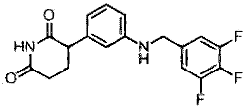 | 1.96 |
| AEV40113 | 312.37 | 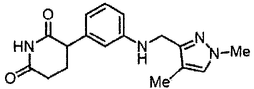 | 0.66 |
| AEV40120 | 344.41 | 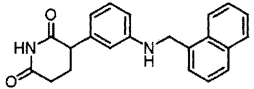 | 1.41 |
| AEV40121 | 386.45 | 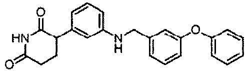 | 3.04 |
| AEV40122 | 370.45 | 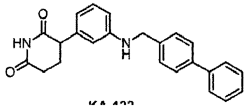 | 2.47 |
| AEV40129 | 323.352 | 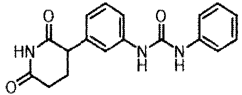 | 0.37 |
| AEV40130 | 318.373 | 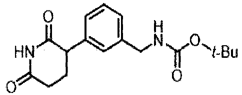 | 4.33 |
| AEV40131 | 357.794 | 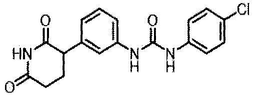 | 0.19 |
| AEV40132 | 218.256 | 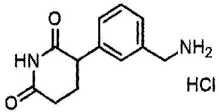 | >10 |
| AEV40133 | 391.3502 | 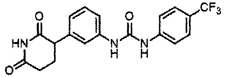 | 1.26 |
| AEV40134 | 377.221 | 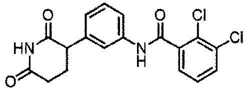 | 1.13 |
| AEV40135 | 497.75 | 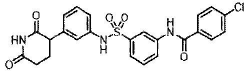 | 1.20 |
| AEV40136 | 497.95 | 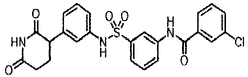 | 0.79 |
| AEV40137 | 377.221 | 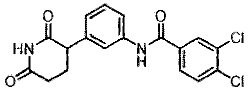 | 2.22 |
| AEV40138 | 411.663 | 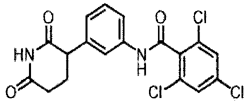 | 0.80 |
| AEV40139 | 342.779 | 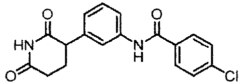 | 2.12 |
| AEV40140 | 326.327 | 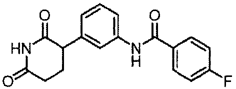 | 1.83 |
| AEV40141 | 322.364 | 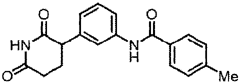 | 2.08 |
| AEV40142 | 532.392 | 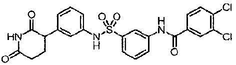 | 0.98 |
| AEV40143 | 463.508 | 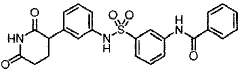 | 0.61 |
| AEV40144 | 342.779 | 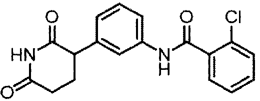 | 0.96 |
| AEV40145 | 326.327 | 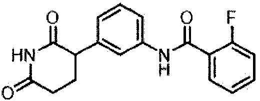 | 1.03 |
| AEV40146 | 322.364 | 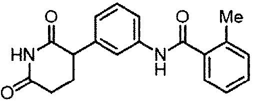 | 1.10 |
| AEV40147 | 356.806 | 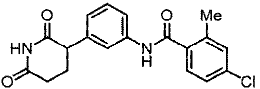 | 1.37 |
| AEV40148 | 372.805 | 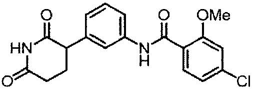 | 1.12 |
| AEV40149 | 549.497 | 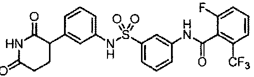 | 1.50 |
| AEV40150 | 532.392 | 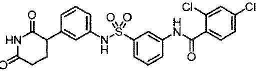 | 1.82 |
| AEV40151 | 493.534 | 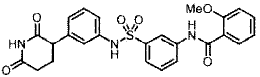 | 0.34 |
| AEV40152 | 531.506 | 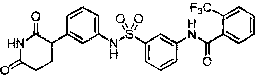 | 0.72 |
| AEV40153 | 517.479 | 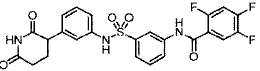 | 0.80 |
| AEV41012 | 519.616 | 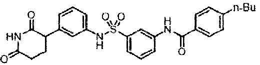 | 0.94 |
| AEV40155 | 542.404 | 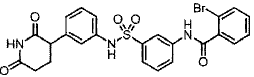 | 0.95 |
| AEV40156 | 499.489 | 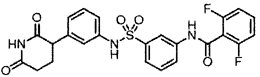 | 1.25 |
| AEV40157 | 542.404 | 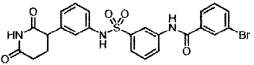 | 0.73 |
| AEV40158 | 517.479 | 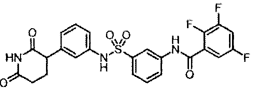 | 0.71 |
| AEV40159 | 550.382 | 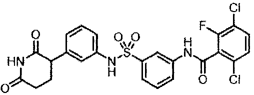 | 1.49 |
| AEV40160 | 301.342 | 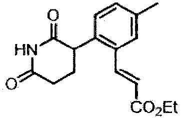 | 2.92 |
| AEV40161 | 384.435 | 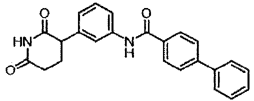 | >10 |
| AEV40162 | 384.435 | 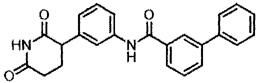 | 2.15 |
| AEV40163 | 488.562 | 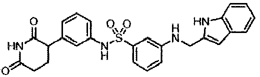 | 1.43 |
| AEV40164 | 344.318 | 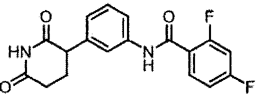 | 1.67 |
| AEV40165 | 336.391 | 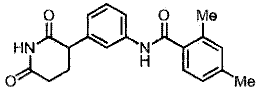 | 0.93 |
| AEV40166 | 344.318 | 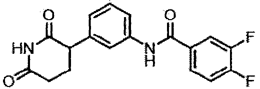 | 2.52 |
| AEV40171 | 488.562 | 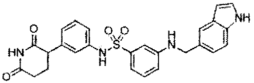 | 1.28 |
| AEV40172 | 490.534 | 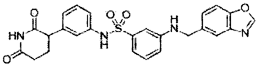 | 0.21 |
| AEV40173 | 523.004 | 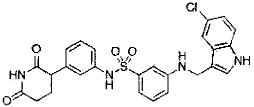 | 0.40 |
| AEV40176 | 372.805 | 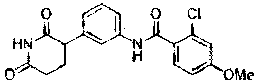 | 1.24 |
| AEV40178 | 377.221 | 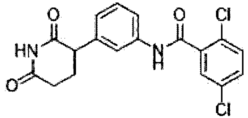 | 0.92 |
| AEV40179 | 389.233 | 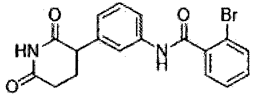 | 0.64 |
| AEV40180 | 376.335 | 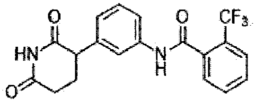 | 1.13 |
| AEV40181 | 526.992 | 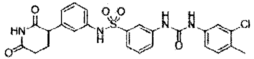 | 0.32 |
| AEV40182 | 348.801 | 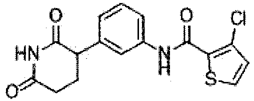 | 0.88 |
| AEV40183 | 332.740 | 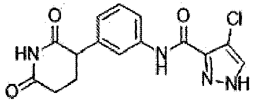 | 1.05 |
| AEV40184 | 366.42 | 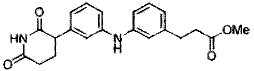 | 1.41 |
| AEV40185 | 475.97 | 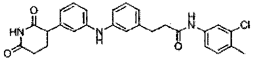 | 4.35 |
| AEV40186 | 409.49 | 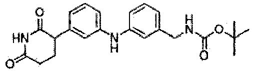 | 2.63 |
| AEV40187 | 359.39 | 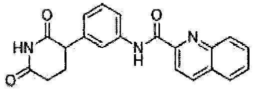 | 0.83 |
| AEV40188 | 346.77 | 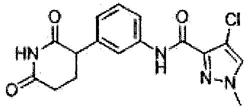 | 1.00 |
| AEV40189 | 348.8 | 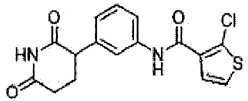 | 1.63 |
| AEV40190 | 347.37 | 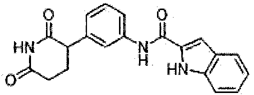 | 2.14 |
| AEV40191 | 347.37 | 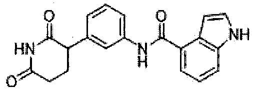 | 0.48 |
| AEV40192 | 476.96 | 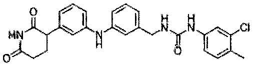 | 3.22 |
| AEV40193 | 521.59 | 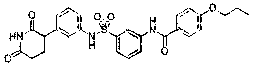 | 0.86 |
| AEV40194 | 347.37 | 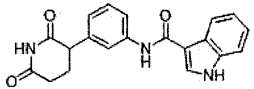 | 0.85 |
| AEV40196 | 525.65 | 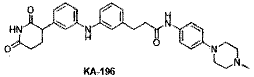 | 1.23 |
| AEV40199 | 527.66 | 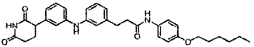 | 3.47 |
| AEV40200 | 476.58 | 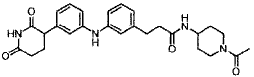 | 0.96 |
| AEV40201 | 493.57 | 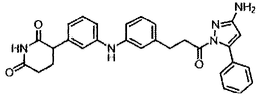 | 1.22 |
| AEV40202 | 467.53 | 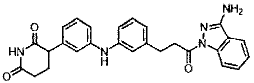 | 0.79 |
| AEV40203 | 230.22 | 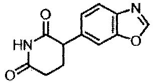 | 1.80 |
| AEV40204 | 561.7 | 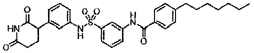 | 0.44 |
| AEV40205 | 469.56 | 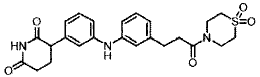 | 0.84 |
| AEV40206 | 365.39 | 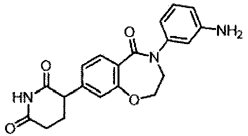 | 3.69 |
| AEV40207 | 315.33 | 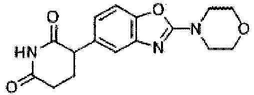 | 4.57 |
| AEV40208 | 313.36 | 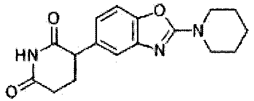 | 3.54 |
| AEV40209 | 493.57 | 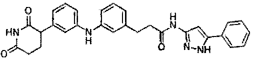 | 0.91 |
| AEV40210 | 503.6 | 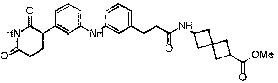 | 1.22 |
| AEV40211 | 489.57 | 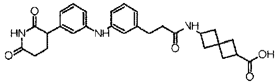 | >100 |
| AEV40212 | 447.49 | 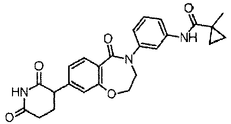 | 2.30 |
| AEV40213 | 489.57 | 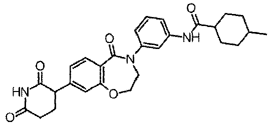 | 2.11 |
| AEV40214 | 525.61 | 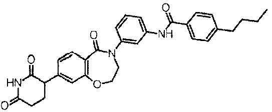 | 0.82 |
| AEV40215 | 570.58 | 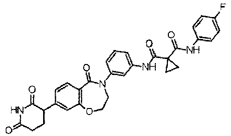 | 2.16 |
| AEV40216 | 477.52 | 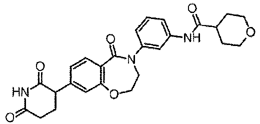 | 2.51 |
| AEV40217 | 538.38 | 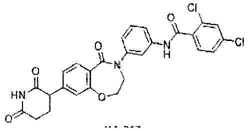 | 1.18 |
| AEV40218 | 384.44 | 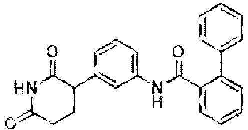 | 0.44 |
| AEV40219 | 500.53 | 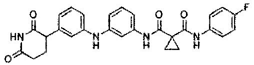 | 2.21 |
| AEV40220 | 468.33 | 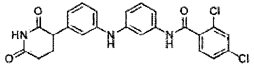 | 2.11 |
| AEV40221 | 455.56 | 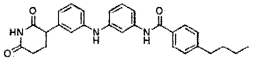 | 1.65 |
| AEV40222 | 418.88 | 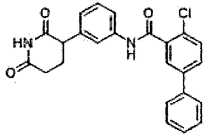 | 1.72 |
| AEV40223 | 421.68 | 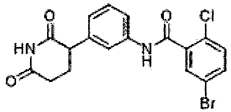 | 1.38 |
| AEV40224 | 483.58 | 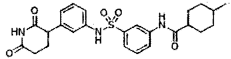 | 2.13 |
| AEV40225 | 471.53 | 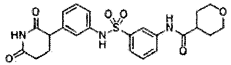 | 12.1 |
| AEV40226 | 402.42 | 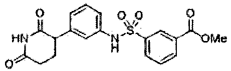 | 2.22 |
| AEV40227 | 441.5 | 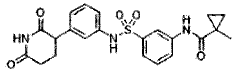 | 0.98 |
| AEV40204 | 561.7 | 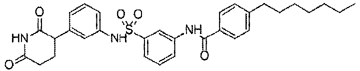 | 0.44 |
| AEV40264 | 575.72 | 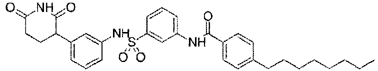 | 0.32 |
| AEV41001 | 498 | 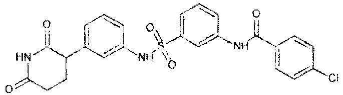 | 1.23 |
| AEV41002 | 512 | 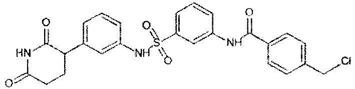 | 0.17 |
| AEV41003 | 526 | 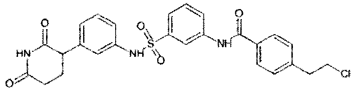 | 0.77 |
| AEV41004 | 540 | 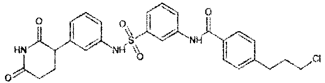 | 1.57 |
| AEV41005 | 554.1 | 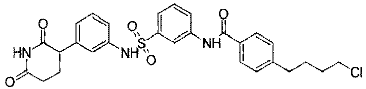 | 1.2 |
| AEV41009 | 477.5 | 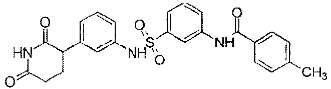 | 0.54 |
| AEV41010 | 491.6 | 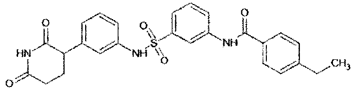 | 0.42 |
| AEV41011 | 505.6 | 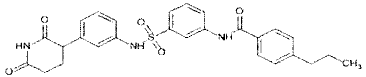 | 0.7 |
| AEV41013 | 533.6 | 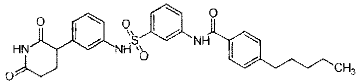 | 0.36 |
| AEV41047 | 568.1 | 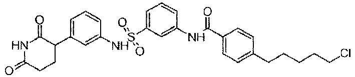 | 1.88 |
| AEV41048 | 582.1 | 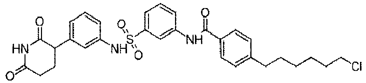 | 2.59 |
| AEV41083 | 477.7 | 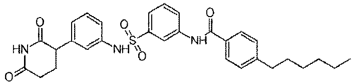 | 0.91 |

10. A pharmaceutical composition for preventing or treating a benign tumor or a cancer, comprising, as an active ingredient, a compound, an optical isomer thereof, a racemic mixture thereof, a hydrate thereof, a solvate thereof, or a pharmaceutically acceptable salt thereof according to any one of claims 1 to 9.

11. The pharmaceutical composition for preventing or treating a benign tumor or a cancer according to claim 10, wherein the cancer is a hematologic cancer or a solid cancer.

12. The pharmaceutical composition for preventing or treating a benign tumor or cancer according to claim 11, wherein the hematologic cancer is selected from the group consisting of acute leukemia, chronic leukemia, multiple myeloma, Hodgkin's lymphoma, and non-Hodgkin's lymphoma.

13. The pharmaceutical composition for preventing or treating a benign tumor or cancer according to claim 11, wherein the solid cancer is selected from the group consisting of melanoma, myosarcoma, multiple myeloma, head and neck cancer, nasopharyngeal cancer, esophageal cancer, gastroesophageal junction cancer, esophageal adenocarcinoma, gastric cancer, bladder cancer, colorectal cancer, colon cancer, rectal cancer, small intestinal cancer, anal cancer, liver cancer, gallbladder cancer, cholangiocarcinoma, biliary tract cancer, pancreatic cancer, thyroid cancer, parathyroid cancer, lung cancer, breast cancer, ovarian cancer, fallopian tube cancer, uterine cancer, vaginal cancer, vulvar cancer, penile cancer, renal cancer, adrenal cancer, urothelial carcinoma, prostate cancer, testicular tumor, bone and soft tissue sarcoma, skin cancer, glioma, brain tumor, spinal tumor, Kaposi's sarcoma, squamous cell carcinoma, pleural mesothelioma, and primary peritoneal cancer.

14. A composition for inhibiting cereblon activity, comprising a compound, an optical isomer thereof, a racemic mixture thereof, a hydrate thereof, or a solvate thereof according to any one of claims 1 to 9.
